# EUROPEAN PATENT SPECIFICATION

(11) **EP 4 482 841 B1**
(45) Date of publication and mention of the grant of the patent: **15.07.2026**
(21) Application number: 23705039.8
(22) Date of filing: 20.02.2023
(51) Int. Cl.: C07D 495/14, A61P 25/00, A61K 31/53

(54) **THIENOPYRROLOTRIAZINE COMPOUNDS, THEIR PREPARATION AND THEIR THERAPEUTIC USE**
THIENOPYRROLOTRIAZINVERBINDUNGEN, DEREN HERSTELLUNG UND DEREN THERAPEUTISCHE VERWENDUNG
COMPOSÉS DE THIÉNOPYRROLOTRIAZINE, LEUR PRÉPARATION ET LEUR APPLICATION THÉRAPEUTIQUE

(30) Priority: 21.02.2022 EP 22315036
(43) Date of publication of application: 01.01.2025
(73) Proprietor: Sanofi, 75017 Paris (FR)
(72) Inventor: ARNOUD, Olivier, 75017 PARIS (FR); CAUSSANEL, Franck, 75017 PARIS (FR); DADJI-FAIHUN, Rommel, 75017 PARIS (FR); LANGOT, Gwladys, 75017 PARIS (FR); MARGUET, Frank, 75017 PARIS (FR); VENIER, Olivier, 75017 PARIS (FR)
(74) Representative: Mathys & Squire
(86) International application number: PCT/EP2023/054138
(87) International publication number: WO 2023/156643

(56) References cited:
- WO-A1-2020/021447
- WO-A1-2021/214284

## Description

Disclosed herein are thienopyrrolotriazine compounds, their preparation, their pharmaceutical composition comprising said compounds, and their therapeutic use.

The compounds according to the present disclosure are useful as inhibitors of NOD-like receptor protein 3 (NLRP3) inflammasome pathway.

### The NOD-like receptor protein 3:

The NOD-like receptor (NLR) family, pyrin domain-containing protein 3 (NLRP3) or NACHT, LRR and PYD domains-containing protein 3 (NALP3), is a cytosolic sensor of diverse pathogen- and host-derived molecules. Upon activation, NLRP3 oligomerizes and recruits an adaptor protein called apoptosis-associated speck like protein (ASC). ASC then polymerizes to form a large aggregate known as ASC speck. In turn, polymerized ASC interacts with the cysteine protease caspase-1 to form a complex termed the inflammasome. This multicomplex protein forms a platform for the binding, dimerization, and activation of the caspase-1 protease. Caspase-1 then cleaves the precursor forms of the pro-inflammatory cytokines IL1β and IL18 (termed pro- IL1β and pro-IL18) and thereby activates adapted inflammatory responses. However, this pathway was shown to be associated with various inflammation associated processes and diseases, including:
- Neurodegenerative diseases such as Parkinson's Disease (PD), Multiple System Atrophy (MSA), Alzheimer's Disease (AD), Frontotemporal Dementia (FTD), Multiple Sclerosis (MS), Amyotrophic Lateral Sclerosis (ALS) and Brain injury (Guan Y & Han F. Front. Integr., Neurosci. 14 :37, 2020);
- Inflammatory diseases including Muckle-Wells Autoinflammatory Disorder (Agostini et al., 2004), cryopyrin-associated periodic syndrome (CAPS) (Mortimer et al., Nature Immunol. 2016, 17(10), 1176-1188); sickle cell disease; systemic lupus erythematosus (SLE); liver related diseases, viral hepatitis, non-alcoholic steatohepatitis (NASH), alcoholic steatohepatitis, and alcoholic liver disease (Petrasek et al., J. Clin. Invest. 2012, 122, 3476-89), and inflammatory arthritis related disorders, such as gout, pseudogout (chondrocalcinosis), osteoarthritis (Ridker et al., N. Engl. J. Med. 2017, 377, 1119-31), and rheumatoid arthritis (Mathews et al., Ann. Rheum. Dis. 2014, 73, 1202-10), acute or chronic arthropathy, and kidney related diseases such as hyperoxaluria (Knaufet et al., Kidney Int. 2013, 84, 895-901), lupus nephritis, hypertensive nephropathy (Krishnan et al., Br. J. Pharmacol. 2016, 173, 752-10 65), hemodialysis related inflammation and diabetic nephropathy (Shahzad et al., Kidney Int. 2015, 87, 74-84);
- Obesity & insulin resistance (Rheinheimer J. et al., Metabolism Clin & Experimental 74: 1-9, 2017); Pancreatitis (Fu Q. et al., BioMed Research International Volume 2018, Article ID 12949512018); Myocarditis (Toldo S et al, Int J Cardiol 2014);
- Eye diseases, where the NLRP3 inflammasome has been shown to contribute to diabetic retinopathy (Perrone L. et al., J. Cell. Physiol. 221: 262-272, 2009), acute glaucoma (Chi W. et al. National Academy Science 111: 11181-11186, 2014), age-related macular degeneration (Tseng W.A. et al., Investigative Ophthal & Visual Science 54: 11-120, 2013), Behcet's syndrome and dry eye disease (Zheng Q. et al., Experimental Eye Research 134: 133-140, 2015);
- Metabolic, cardiac, skin disorder & cancer, e.g., diabetic cardiomyopathy (Luo B. et al., PLoS ONE 9(8): e104771, 2014); Kawasaki disease (Jia et al. Cell Death and Disease 10:778; 2019; Anzai F. et al., J. Molecular & Cell Cardiology 138: 185-196, 2020); cardiovascular metabolic disorders, atherosclerosis, type I and type II diabetes and related complications, peripheral artery disease (PAD), acute heart failure and hypertension (Ridker et al., N. Engl. J. Med. 2017, 377, 1119-31; wound healing and scar formation; inflammatory skin diseases (Sweeney et al., Br. J. Dermatol. 2015, 173, 1361), asthma, sarcoidosis, age-related macular degeneration; cancer related diseases, e.g., myeloproliferative neoplasms, leukemias, myelodysplastic syndromes (MDS), myelofibrosis, lung cancer, colon cancer (Ridker et al., Lancet 2017, 390, 1833-42); and
- SARS-Cov-2: NLRP3 inflammasome is key player in antiviral responses (Zhao C. and Zhao W. 11, 211: 2020; Freeman & Swartz, Frontiers in Immunol. 11, 1518, 2020).

Inhibitors of NLRP3 are potential treatments for these conditions with unmet clinical needs.

Therefore, there is a need for inhibitors of the NLRP3 inflammasome pathway to provide new or alternative treatments.

Disclosed herein are compounds corresponding to formula (I) 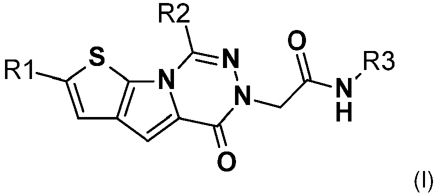 in which:
**R1** represents a hydrogen atom, a halogen atom, a -(C₁-C₂)-alkyl group,
**R2** represents a -(C₁-C₃)alkyl group being unsubstituted or substituted with 1 to 3 substituents independently selected from a hydroxy group , a -(C₁-C₃)-alkoxy group, or a -(C₃-C₄)cycloalkyl group,
**R3** is selected from
   ➢ a -(C₅-C₈)bicycloalkyl group, being unsubstituted or substituted with one -(C₁-C₃)alkyl group being unsubstituted or substituted with one or two -NH(CO)Me group,
   ➢ a -(C₄-C₇)heterocycloalkyl group with nitrogen as heteroatom, being unsubstituted or substituted with one or more substituents independently selected from
      - a halogen atom,
      - an oxo group,
      - a -(C₁-C₄)-alkyl group being unsubstituted or substituted with 1 to 3 substituents independently selected from a hydroxy group, a -(C₁-C₂)-alkyl group, a halogen atom, -(C₁-C₂)-alkoxy group, a nitrile group, a -C(O)O(C₁-C₃) group, or a -(C₃-C₈)-cycloalkyl group being unsubstituted or substituted by one or more halogen group,
      - a -(C₃-C₆)-cycloalkyl group, being unsubstituted or substituted by one or more (C₁-C₂) alkyl groups,
      - a -(CO)-(C₁-C₂)-alkyl group,
      - a heterocycloalkyl group, or
      - a heteroaryl group, being unsubstituted or substituted with one or more -(C₁-C₄)alkyl groups,
   ➢ a hetero(C₆-C₉)bicycloalkyl group with nitrogen as heteroatom, optionally substituted with one or more substituents independently selected from
      - a -(C₃-C₄) cycloalkyl group, or
      - a -(C₁-C₃)alkyl group,
      or
   ➢ a mono- or bicycloheteroaryl group, being unsubstituted or substituted with one or more substituents independently selected from
      ➢ a -(C₁-C₄)-alkyl group,
      ➢ a -(C₁-C₄)-alkoxy group, or
      ➢ a halogen atom,
or a pharmaceutically acceptable salt thereof.

The compounds of formula (I) may comprise one or more asymmetric carbon atoms. They may thus exist in the form of enantiomers, diastereoisomers, or mixtures thereof.

The compounds of formula (I) may exist in the form of bases or addition salts with acids or bases, in particular pharmaceutically acceptable salts.

Pharmaceutically acceptable salts of the compounds of formula (I) form part of the present disclosure.

As used herein, certain terms have the following definitions:
- a halogen atom: a fluorine, a chlorine, or a bromine atom;
- a -(C₁-C₄)-alkyl group: a linear or branched saturated aliphatic group containing between 1 and 4 carbon atoms. Examples include, but are not limited to, methyl (Me), ethyl, propyl, isopropyl (iPr), butyl, isobutyl, t-butyl, etc;
- a cycloalkyl group: a cyclic alkyl group. Examples include, but are not limited to, cyclopropyl, cyclobutyl, cyclopentyl, etc.;
- a bicycloalkyl group: a two cyclic alkyl group, where the cycloalkyl can have a bond or a carbon atom in common. Examples include, but are not limited to bicyclo[1.1.1]pentane, etc;
- a heterocycloalkyl group: a saturated cyclic group containing between 3 and 6 carbon atoms and containing one or two heteroatoms such as nitrogen. Examples include, but are not limited to, piperidine, azepane, and pyrrolidine;
- a heterobicycloalkyl group: a saturated bicyclic group containing between 3 and 4 carbon atoms and containing one or two heteroatoms such as nitrogen or oxygen. Examples include, but are not limited to, quinuclidine, *N*-2-azabicyclo[2.2.1]heptan-6-amine, azabicyclo[2.2.1]heptane, and azaspiro[3.3]heptane;
- an alkoxy group: a radical -O-alkyl in which the alkyl group is as defined above. Examples of alkoxy group include, but are not limited to, methoxy, ethoxy, etc.;
- a nitrile group: a group including -CN;
- a hydroxy group: a group including -OH;
- an aryl group: a cyclic aromatic group comprising between 5 and 10 carbon atoms. Example of an aryl group include phenyl group, etc;
- a mono or bicycloheteroaryl group: an unsaturated cyclic group containing between 4 and 9 carbon atoms and containing 1 or 2 heteroatoms such as sulfur or nitrogen. Examples include, but are not limited to, thiazole, pyrazole, pyrimidine, indazole, benzothiazole, etc.

In an embodiment the compounds of formula (I) comprise a first group composed of the compounds in which:
**R1** represents a hydrogen atom, a halogen atom chosen between bromine and chlorine atom, methyl group,
**R2** represents a -(C₁-C₃)alkyl group chosen between methyl, ethyl, propyl and isopropyl group being unsubstituted or substituted with 1 substituent independently selected from a hydroxy group, a methoxy group, or a cyclopropyl group,
**R3** is selected from
   ➢ a bicyclopentyl group, being substituted with one -(C₁-C₃)alkyl group being unsubstituted or substituted with one -NH(CO)Me group,
   ➢ a -(C₄-C₆)heterocycloalkyl group with nitrogen as heteroatom, being unsubstituted or substituted with one or more substituents independently selected from
      - a fluorine atom,
      - an oxo group,
      - a -(C₁-C₄)-alkyl group, being unsubstituted or substituted with 1 to 3 substituents independently selected from a hydroxy group, a methyl group, a fluorine atom, a methoxy group, a nitrile group, a -C(O)O(C₁-C₂) group, or a - (C₃-C₆)-cycloalkyl group being unsubstituted or substituted by one fluorine atom,
      - a -(C₃-C₅)-cycloalkyl group, being unsubstituted or substituted by one or more methyl groups,
      - -(CO)-methyl group,
      - a heterocyclopropyl group, or
      - an imidazole group, being unsubstituted or substituted with one methyl group,
   ➢ a hetero(C₆-C₉)bicycloalkyl group with nitrogen as heteroatom, optionally substituted with one or more substituents independently selected from
      - a -(C₃-C₄)cycloalkyl group, or
      - a methyl group,
      or
   ➢ a mono, or bicycloheteroaryl group, being unsubstituted or substituted with one or two substituents independently selected from
      ➢ a methyl group,
      ➢ a methoxy group, or
      ➢ a fluorine atom,
or a pharmaceutically acceptable salt thereof.

In an embodiment the compounds of formula (I) comprise a second group composed of the compounds in which
**R1** represents a hydrogen atom, a halogen atom chosen between a bromine and a chlorine atom,
**R2** represents -(C₁-C₃)alkyl group being unsubstituted or substituted with 1 to 3 hydroxy group, or a -(C₃-C₄)cycloalkyl group,
**R3** is selected from
   ➢ a (C₅-C₈)bicycloalkyl group, being unsubstituted or substituted with one -(C₁-C₃)alkyl group being unsubstituted or substituted with one or two -NH(CO)Me groups,
   ➢ a (C₄-C₇)heterocycloalkyl group with nitrogen as heteroatom, being unsubstituted or substituted with one or more substituents independently selected from
      - a -(C₁-C₄)-alkyl group, being unsubstituted or substituted with 1 to 3 substituents independently selected from a hydroxy group, a methyl group, a fluorine atom, a methoxy group, a nitrile group, a -C(O)O(C₁-C₃) group, or a -(C₃-C₅)cycloalkyl group being unsubstituted or substituted by one or more fluorine atom,
      - a (C₃-C₆)-cycloalkyl group, being unsubstituted or substituted by one or more -(C₁-C₂)alkyl groups, or
      - a heterocycloalkyl group,
   ➢ a hetero(C₆-C₈)bicycloalkyl group with nitrogen as heteroatom, optionally substituted with one or more -(C₃-C₄)cycloalkyl groups,
      or
   ➢ a mono- or bicycloheteroaryl group, being unsubstituted or substituted with one fluorine atom,
or a pharmaceutically acceptable salt thereof.

In an embodiment the compounds of formula (I) comprise a third group composed of the compounds in which **R1** represents a hydrogen atom or a pharmaceutically acceptable salt thereof.

In an embodiment the compounds of formula (I) comprise a fourth group composed of the compounds in which **R1** represents a bromine atom or a pharmaceutically acceptable salt thereof.

In an embodiment the compounds of formula (I) comprise a fifth group composed of the compounds in which **R1** represents a chlorine atom or a pharmaceutically acceptable salt thereof.

In an embodiment, the compounds of formula (I) comprise a sixth group composed of the compounds in which **R2** represents a -iPr group or a pharmaceutically acceptable salt thereof.

In an embodiment, the compounds of formula (I) comprise a seventh group composed of the compounds in which **R2** represents a -cyclopropyl group or a pharmaceutically acceptable salt thereof.

In an embodiment, the compounds of formula (I) comprise a eighth group composed of the compounds in which **R3** represents a -(C₄-C₇)heterocycloalkyl group with nitrogen as heteroatom, being unsubstituted or substituted with one or more substituents independently selected from
- a -(C₁-C₄)-alkyl group, being unsubstituted or substituted with 1 to 3 substituents independently selected from a hydroxy group, -(C₁-C₂)-alkyl group, a fluorine atom, -(C₁-C₂)-alkoxy group, a nitrile group, a -C(O)O(C₁-C₃) group, or a -(C₃-C₅)-cycloalkyl group being unsubstituted or substituted by one or more fluorine atom,
- a -(C₃-C₆)-cycloalkyl group, being unsubstituted or substituted by one or more -(C₁-C₂)alkyl group, or
- a heterocyclopropyl group.
or a pharmaceutically acceptable salt thereof.

All these sub-groups taken alone or in combination are part of the description.

Among the compounds of formula (I), mention may be made in particular of the following compounds:
1 (R)-2-(8-isopropyl-5-oxothieno[3',2':4,5]pyrrolo[1,2-d][1,2,4]triazin-6(5H)-yl)-N-(1-methylpiperidin-3-yl)acetamide
2 2-(8-isopropyl-5-oxothieno[3',2':4,5]pyrrolo[1,2-d][1,2,4]triazin-6(5H)-yl)-N-(thiazol-5-yl) acetamide
3 N-((R)-1-((S)-2-hydroxypropyl)piperidin-3-yl)-2-(8-isopropyl-5-oxothieno[3',2':4,5] pyrrolo[1,2-d][1,2,4]triazin-6(5H)-yl)acetamide
4 (R)-N-(1-cyclopropylpiperidin-3-yl)-2-(8-isopropyl-5-oxothieno[3',2':4,5]pyrrolo[1,2-d] [1,2,4]triazin-6(5H)-yl)acetamide
5 (R)-2-(2-chloro-8-isopropyl-5-oxothieno[3',2':4,5]pyrrolo[1,2-d][1,2,4] triazin-6(5H)-yl)-N-(1-methylpiperidin-3-yl)acetamide 2,2,2-trifluoroacetate
6 (R)-2-(2-chloro-8-isopropyl-5-oxothieno[3',2':4,5]pyrrolo[1,2-d][1,2,4]triazin-6(5H)-yl)-N-(1-cyclopropylpiperidin-3-yl)acetamide 2,2,2-trifluoroacetate
7 2-(2-chloro-8-isopropyl-5-oxothieno[3',2':4,5]pyrrolo[1,2-d][1,2,4]triazin-6(5H)-yl)-N-(thiazol-5-yl)acetamide
8 (R)-2-(2-bromo-8-isopropyl-5-oxothieno[3',2':4,5]pyrrolo[1,2-d][1,2,4]triazin-6(5H)-yl)-N-(1-methylpiperidin-3-yl)acetamide
9 (R)-2-(8-isopropyl-2-methyl-5-oxothieno[3',2':4,5]pyrrolo[1,2-d][1,2,4]triazin-6(5H)-yl)-N-(1-methylpiperidin-3-yl)acetamide 2,2,2-trifluoroacetate
10 (R)-2-(2-chloro-8-isopropyl-5-oxothieno[3',2':4,5]pyrrolo[1,2-d][1,2,4]triazin-6(5H)-yl)-N-(1-cyclobutylpiperidin-3-yl)acetamide formate
11 (R)-N-(1-cyclobutylpiperidin-3-yl)-2-(8-isopropyl-5-oxothieno[3',2':4,5]pyrrolo[1,2-d] [1,2,4]triazin-6(5H)-yl)acetamide formate
12 N-(benzo[d]thiazol-6-yl)-2-(8-isopropyl-5-oxothieno[3',2':4,5]pyrrolo[1,2-d][1,2,4]triazin-6(5H)-yl)acetamide
13 (R)-2-(2-chloro-8-cyclopropyl-5-oxothieno[3',2':4,5]pyrrolo[1,2-d][1,2,4]triazin-6(5H)-yl)-N-(1-cyclopropylpiperidin-3-yl)acetamide
14 (R)-N-(1-isobutylpiperidin-3-yl)-2-(8-isopropyl-5-oxothieno[3',2':4,5]pyrrolo[1,2-d] [1,2,4]triazin-6(5H)-yl)acetamide
15 N-(benzo[d]thiazol-5-yl)-2-(8-isopropyl-5-oxothieno[3',2':4,5]pyrrolo[1,2-d][1,2,4]triazin-6(5H)-yl)acetamide
16 2-(8-isopropyl-5-oxothieno[3',2':4,5]pyrrolo[1,2-d][1,2,4]triazin-6(5H)-yl)-N-(6-methoxypyridin-3-yl)acetamide
17 (R)-N-(1-(cyclopentylmethyl)piperidin-3-yl)-2-(8-isopropyl-5-oxothieno[3',2':4,5]pyrrolo [1,2-d][1,2,4]triazin-6(5H)-yl)acetamide
18 N-((3R)-1-((2,2-difluorocyclopropyl)methyl)piperidin-3-yl)-2-(8-isopropyl-5-oxothieno [3',2':4,5]pyrrolo[1,2-d][1,2,4]triazin-6(5H)-yl)acetamide compound with formaldehyde
19 (R)-N-(1-(2-hydroxyethyl)piperidin-3-yl)-2-(8-isopropyl-5-oxothieno [3',2':4,5]pyrrolo[1,2-d][1,2,4]triazin-6(5H)-yl)acetamide compound with formaldehyde
20 (R)-N-(1-cyclobutylpiperidin-3-yl)-2-(8-cyclopropyl-5-oxothieno[3',2':4,5]pyrrolo[1,2-d] [1,2,4]triazin-6(5H)-yl)acetamide
21 (R)-2-(8-cyclopropyl-5-oxothieno[3',2':4,5]pyrrolo[1,2-d][1,2,4]triazin-6(5H)-yl)-N-(1-cyclopropylpiperidin-3-yl)acetamide
22 2-(8-cyclopropyl-5-oxothieno[3',2':4,5]pyrrolo[1,2-d][1,2,4]triazin-6(5H)-yl)-N-((R)-1-((S)-2-hydroxypropyl)piperidin-3-yl)acetamide
23 (R)-N-(1-(2-hydroxy-2-methylpropyl)piperidin-3-yl)-2-(8-isopropyl-5-oxothieno[3',2':4,5] pyrrolo[1,2-d][1,2,4]triazin-6(5H)-yl)acetamide formate
24 (R)-2-(8-cyclopropyl-5-oxothieno[3',2':4,5]pyrrolo[1,2-d][1,2,4]triazin-6(5H)-yl)-N-(1-methylpiperidin-3-yl)acetamide formate
25 N-((R)-1-cyclobutylpiperidin-3-yl)-2-(8-isopropyl-5-oxothieno[3',2':4,5]pyrrolo[1,2-d] [1,2,4]triazin-6(5H)-yl)propanamide formate
26 N-((R)-1-cyclopropylpiperidin-3-yl)-2-(8-isopropyl-5-oxothieno[3',2':4,5]pyrrolo[1,2-d] [1,2,4]triazin-6(5H)-yl)propanamide formate
27 2-(8-isopropyl-5-oxothieno[3',2':4,5]pyrrolo[1,2-d][1,2,4]triazin-6(5H)-yl)-N-(1-methyl-1 H-indazol-6-yl)acetamide formate
28 (R)-N-(1-(cyclopropylmethyl)piperidin-3-yl)-2-(8-isopropyl-5-oxothieno[3',2':4,5]pyrrolo [1,2-d][1,2,4]triazin-6(5H)-yl)acetamide formate
29 (R)-2-(2-chloro-8-(methoxymethyl)-5-oxothieno[3',2':4,5]pyrrolo[1,2-d][1,2,4]triazin-6(5H)-yl)-N-(1-cyclobutylpiperidin-3-yl)acetamide formate
30 (R)-2-(2-chloro-8-(methoxymethyl)-5-oxothieno[3',2':4,5]pyrrolo[1,2-d][1,2,4]triazin-6(5H)-yl)-N-(1-cyclopropylpiperidin-3-yl)acetamide formate
31 (R)-2-(2-bromo-8-isopropyl-5-oxothieno[3',2':4,5]pyrrolo[1,2-d][1,2,4]triazin-6(5H)-yl)-N-(1-cyclobutylpiperidin-3-yl)acetamide 2,2,2-trifluoroacetate
32 (R)-N-(1-cyclobutylpiperidin-3-yl)-2-(8-(methoxymethyl)-5-oxothieno [3',2':4,5]pyrrolo[1,2-d][1,2,4]triazin-6(5H)-yl)acetamide
33 (R)-N-(1-cyclopropylpiperidin-3-yl)-2-(8-(methoxymethyl)-5-oxothieno[3',2':4,5]pyrrolo[1,2-d][1,2,4]triazin-6(5H)-yl)acetamide 2,2,2-trifluoroacetate
34 2-(2-chloro-8-isopropyl-5-oxothieno[3',2':4,5]pyrrolo[1,2-d][1,2,4]triazin-6(5H)-yl)-N-(5,5-difluoro-1-methylpiperidin-3-yl)acetamide 2,2,2-trifluoroacetate
35 N-(5,5-difluoro-1-methylpiperidin-3-yl)-2-(8-isopropyl-5-oxothieno[3',2':4,5]pyrrolo[1,2-d] [1,2,4]triazin-6(5H)-yl)acetamide
36 (R)-N-(1-(2-fluoroethyl)piperidin-3-yl)-2-(8-isopropyl-5-oxothieno[3',2':4,5]pyrrolo[1,2-d] [1,2,4]triazin-6(5H)-yl)acetamide formate
37 N-(1-(tert-butyl)piperidin-3-yl)-2-(8-isopropyl-5-oxothieno[3',2':4,5]pyrrolo[1,2-d][1,2,4] triazin-6(5H)-yl)acetamide formate
38 2-(8-isopropyl-5-oxothieno[3',2':4,5]pyrrolo[1,2-d][1,2,4]triazin-6(5H)-yl)-N-(1-(2,2,2-trifluoroethyl)piperidin-3-yl)acetamide 2,2,2-trifluoroacetate
39 2-(8-isopropyl-5-oxothieno[3',2':4,5]pyrrolo[1,2-d][1,2,4]triazin-6(5H)-yl)-N-(quinuclidin-3-yl)acetamide
40 (R)-2-(8-isopropyl-5-oxothieno[3',2':4,5]pyrrolo[1,2-d][1,2,4]triazin-6(5H)-yl)-N-(1-(oxetan-3-yl)piperidin-3-yl)acetamide
41 (R)-N-(1-(3-fluoropropyl)piperidin-3-yl)-2-(8-isopropyl-5-oxothieno[3',2':4,5]pyrrolo[1,2-d][1,2,4]triazin-6(5H)-yl)acetamide formate
42 (R)-N-(1-acetylpiperidin-3-yl)-2-(8-isopropyl-5-oxothieno[3',2':4,5]pyrrolo[1,2-d] [1,2,4]triazin-6(5H)-yl)acetamide
43 (R)-N-(1-((3,3-difluorocyclobutyl)methyl)piperidin-3-yl)-2-(8-isopropyl-5-oxothieno [3',2':4,5]pyrrolo[1,2-d][1,2,4]triazin-6(5H)-yl)acetamide formate
44 N-((R)-1-cyclopropylpiperidin-3-yl)-2-(8-(1-hydroxyethyl)-5-oxothieno[3',2':4,5]pyrrolo [1,2-d][1,2,4]triazin-6(5H)-yl)acetamide
45 (R)-2-(8-isopropyl-5-oxothieno[3',2':4,5]pyrrolo[1,2-d][1,2,4]triazin-6(5H)-yl)-N-(1-(1-methyl-1H-imidazol-2-yl)piperidin-3-yl)acetamide
46 2-(8-isopropyl-5-oxothieno[3',2':4,5]pyrrolo[1,2-d][1,2,4]triazin-6(5H)-yl)-N-(1-methyl-2-oxopiperidin-4-yl)acetamide
47 2-(8-isopropyl-5-oxothieno[3',2':4,5]pyrrolo[1,2-d][1,2,4]triazin-6(5H)-yl)-N-(1-methyl-6-oxopiperidin-3-yl)acetamide
48 2-(12-isopropyl-9-oxo-3-thia-1,10,11-triazatricyclo[6.4.0.02,6]dodeca-2(6),4,7,11-tetraen-10-yl)-N-[(3R)-1-isopropyl-3-piperidyl]acetamide
49 N-[(3R)-1-cyclopentyl-3-piperidyl]-2-(12-isopropyl-9-oxo-3-thia-1,10,11-triazatricyclo [6.4.0.02,6]dodeca-2(6),4,7,11-tetraen-10-yl)acetamide
50 N-((R)-1-cyclobutylpiperidin-3-yl)-2-(8-(1-hydroxyethyl)-5-oxothieno[3',2':4,5]pyrrolo [1,2-d][1,2,4]triazin-6(5H)-yl)acetamide
51 N-((R)-1-cyclopropylpiperidin-3-yl)-2-(8-(1-hydroxyethyl)-5-oxothieno[3',2':4,5]pyrrolo [1,2d][1,2,4]triazin-6(5H)-yl)acetamide
52 (R)-N-(1-cyclopropylpiperidin-3-yl)-2-(8-(2-hydroxypropan-2-yl)-5-oxothieno[3',2':4,5] pyrrolo[1,2-d][1,2,4]triazin-6(5H)-yl)acetamide
53 2-(12-isopropyl-9-oxo-3-thia-1,10,11-triazatricyclo[6.4.0.02,6]dodeca-2(6),4,7,11-tetraen-10-yl)-N-(2-methylpyrazol-3-yl)acetamide
54 2-(12-isopropyl-9-oxo-3-thia-1,10,11-triazatricyclo[6.4.0.02,6]dodeca-2(6),4,7,11-tetraen-10-yl)-N-(1-methylpyrazol-3-yl)acetamide
55 N-[(1-tert-butyl-5-oxo-pyrrolidin-3-yl)methyl]-2-(12-isopropyl-9-oxo-3-thia-1,10,11-triazatricyclo[6.4.0.02,6]dodeca-2(6),4,7,11-tetraen-10-yl)acetamide
56 2-(12-isopropyl-9-oxo-3-thia-1,10,11-triazatricyclo[6.4.0.02,6]dodeca-2(6),4,7,11-tetraen-10-yl)-N-(3-pyridyl)acetamide
57 2-(12-isopropyl-9-oxo-3-thia-1,10,11-triazatricyclo[6.4.0.02,6]dodeca-2(6),4,7,11-tetraen-10-yl)-N-[rac-(3R)-1-(2,2-dimethylcyclobutyl)-3-piperidyl]acetamide
58 2-(12-isopropyl-9-oxo-3-thia-1,10,11-triazatricyclo[6.4.0.02,6]dodeca-2(6),4,7,11-tetraen-10-yl)-N-(2-pyridyl)acetamide
59 2-(4-bromo-12-isopropyl-9-oxo-3-thia-1,10,11-triazatricyclo[6.4.0.02,6]dodeca-2(6),4,7,11-tetraen-10-yl)-N-(1-tert-butyl-3-piperidyl)acetamide
60 N-[(3R)-1-cyclopropyl-3-piperidyl]-2-(12-ethyl-9-oxo-3-thia-1,10,11-triazatricyclo [6.4.0.02,6]dodeca-2(6),4,7,11-tetraen-10-yl)acetamide
61 N-[(3R)-1-cyclopropylazepan-3-yl]-2-(12-isopropyl-9-oxo-3-thia-1,10,11-triazatricyclo [6.4.0.02,6]dodeca-2(6),4,7,11-tetraen-10-yl)acetamide
62 2-(12-isopropyl-9-oxo-3-thia-1,10,11-triazatricyclo[6.4.0.02,6]dodeca-2(6),4,7,11-tetraen-10-yl)-N-[(3R)-1-methylazepan-3-yl]acetamide
63 N-(1-cyclopropyl-5,5-difluoropiperidin-3-yl)-2-(8-isopropyl-5-oxothieno[3',2':4,5]pyrrolo [1,2-d][1,2,4]triazin-6(5H)-yl)acetamide 2,2,2-trifluoroacetate
64 2-(8-isopropyl-5-oxothieno[3',2':4,5]pyrrolo[1,2-d][1,2,4]triazin-6(5H)-yl)-N-((3R)-1-(1-methoxypropan-2-yl)piperidin-3-yl)acetamide
65 2-(8-isopropyl-5-oxothieno[3',2':4,5]pyrrolo[1,2-d][1,2,4]triazin-6(5H)-yl)-N-(2-methoxypyridin-4-yl)acetamide
66 2-(12-isopropyl-9-oxo-3-thia-1,10,11-triazatricyclo[6.4.0.02,6]dodeca-2(6),4,7,11-tetraen-10-yl)-N-pyrimidin-4-yl-acetamide
67 2-(12-isopropyl-9-oxo-3-thia-1,10,11-triazatricyclo[6.4.0.02,6]dodeca-2(6),4,7,11-tetraen-10-yl)-N-(4-pyridyl)acetamide
68 2-(12-isopropyl-9-oxo-3-thia-1,10,11-triazatricyclo[6.4.0.02,6]dodeca-2(6),4,7,11-tetraen-10-yl)-N-(6-methyl-3-pyridyl)acetamide
69 2-(12-ethyl-9-oxo-3-thia-1,10,11-triazatricyclo[6.4.0.02,6]dodeca-2(6),4,7,11-tetraen-10-yl)-N-[(3R)-1-[(2S)-2-hydroxypropyl]-3-piperidyl]acetamide
70 N-(5-fluoropyrimidin-4-yl)-2-(12-isopropyl-9-oxo-3-thia-1,10,11-triazatricyclo[6.4.0.02,6] dodeca-2(6),4,7,11-tetraen-10-yl)acetamide
71 2-(12-isopropyl-9-oxo-3-thia-1,10,11-triazatricyclo[6.4.0.02,6]dodeca-2(6),4,7,11-tetraen-10-yl)-N-[(3R)-1-(1-methylcyclopentyl)-3-piperidyl]acetamide
72 N-[(3R)-1-cyclopropylpiperidin-3-yl]-2-(9-oxo-12-propyl-3-thia-1,10,11-triazatricyclo [6.4.0.02,6]dodeca-2(6),4,7,11-tetraen-10-yl)acetamide
73 2-(12-ethyl-9-oxo-3-thia-1,10,11-triazatricyclo[6.4.0.02,6]dodeca-2(6),4,7,11-tetraen-10-yl)-N-[(3R)-1-methyl-3-piperidyl]acetamide
74 N-[3-(acetamidomethyl)bicyclo[1.1.1]pentan-1-yl]-2-(8-isopropyl-5-oxothieno[3',2':4,5] pyrrolo[1,2-d][1,2,4]triazin-6(5H)-yl)acetamide
75 N-[(3R)-1-(cyanomethyl)piperidin-3-yl]-2-(8-isopropyl-5-oxothieno[3',2':4,5]pyrrolo[1,2-d] [1,2,4]triazin-6(5H)-yl)acetamide
76 N-[(3R)-1-methylpyrrolidin-3-yl]-2-(8-isopropyl-5-oxothieno[3',2':4,5]pyrrolo[1,2-d][1,2,4] triazin-6(5H)-yl)acetamide
77 N-[(3R)-1-(2-cyanoethyl)piperidin-3-yl]-2-(8-isopropyl-5-oxothieno[3',2':4,5]pyrrolo[1,2-d] [1,2,4]triazin-6(5H)-yl)acetamide
78 N-[(3R)-1-[(2R)-2-hydroxypropyl]piperidin-3-yl]-2-(8-isopropyl-5-oxothieno[3',2':4,5] pyrrolo [1,2-d][1,2,4]triazin-6(5H)-yl)acetamide
79 N-[(3R)-1-(1-hydroxy-2-methylpropan-2-yl)piperidin-3-yl]-2-(8-isopropyl-5-oxothieno [3',2':4,5]pyrrolo[1,2-d][1,2,4]triazin-6(5H)-yl)acetamide
80 ethyl 2-methyl-2-[3-[[2-(8-isopropyl-5-oxothieno[3',2':4,5]pyrrolo[1,2-d][1,2,4]triazin-6(5H)-yl)acetyl]amino]piperidin-1-yl]propanoate
81 N-((R)-1-cyclobutylpiperidin-3-yl)-2-(8-(1-hydroxyethyl)-5-oxothieno[3',2':4,5]pyrrolo[1,2-d][1,2,4]triazin-6(5H)-yl)acetamide
82 N-[(1R,4R)-2-methyl-2-azabicyclo[2.2.1]heptan-6-yl]-2-(8-isopropyl-5-oxothieno [3',2':4,5] pyrrolo[1,2-d][1,2,4]triazin-6(5H)-yl)acetamide
83 N-(2-methyl-2-azaspiro[3.3]heptan-6-yl)-2-(8-isopropyl-5-oxothieno[3',2':4,5]pyrrolo[1,2-d][1,2,4]triazin-6(5H)-yl)acetamide
84 2-(8-isopropyl-5-oxothieno[3',2':4,5]pyrrolo[1,2-d][1,2,4]triazin-6(5H)-yl)-N-pyrimidin-5-ylacetamide
85 2-(12-isopropyl-9-oxo-3-thia-1,10,11-triazatricyclo[6.4.0.02,6]dodeca-2(6),4,7,11-tetraen-10-yl)-N-(2-oxo-4-piperidyl)acetamide
86 N-(1-cyclopropylpyrrolidin-3-yl)-2-(12-isopropyl-9-oxo-3-thia-1,10,11-triazatricyclo [6.4.0.02,6]dodeca-2(6),4,7,11-tetraen-10-yl)acetamide
87 2-(12-isopropyl-9-oxo-3-thia-1,10,11-triazatricyclo[6.4.0.02,6]dodeca-2(6),4,7,11-tetraen-10-yl)-N-[rac-(3R)-1-tert-butylpyrrolidin-3-yl]acetamide
88 N-[(1S,4R)-2-azabicyclo[2.2.1]heptan-6-yl]-2-(12-isopropyl-9-oxo-3-thia-1,10,11-triazatricyclo[6.4.0.02,6]dodeca-2(6),4,7,11-tetraen-10-yl)acetamidehydrochloride

In accordance with the present disclosure the compounds of general formula (I) can be prepared by the following process.

Unless otherwise mentioned, R is an alkyl group, X is a halogen atom and R₁ to R₃ are as defined previously.

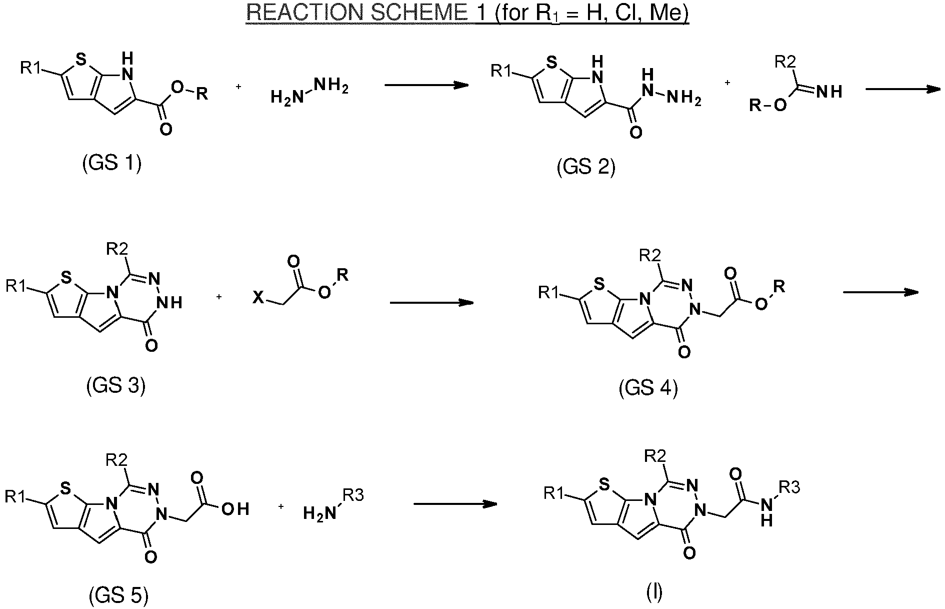

Compounds of general formula (I) can be prepared by the Reaction scheme 1 where a bicyclic thienopyrrolecarboxylic ester (GS 1) is transformed with hydrazine to provide the carboxy hydrazide analogue (GS 2). (GS 2) is then reacted with a hydrochloride salt of an imidate where R₂ is alkyl, cycloalkyl, alkoxyalkyl or hydroxyalkyl, following by addition of a base such as tBuOK to obtain after cyclization triazinone (GS 3). (GS 3) is then alkylated with alkyl haloacetate using a base like K₂CO₃ to give ester (GS 4), which is then saponified using an aqueous base to give acid (GS 5), followed by a coupling with an appropriate amine H₂N-R₃ using known standard methods like EDC/HOBT Et₃N, HATU DIPEA to provide a compound of general formula (I).

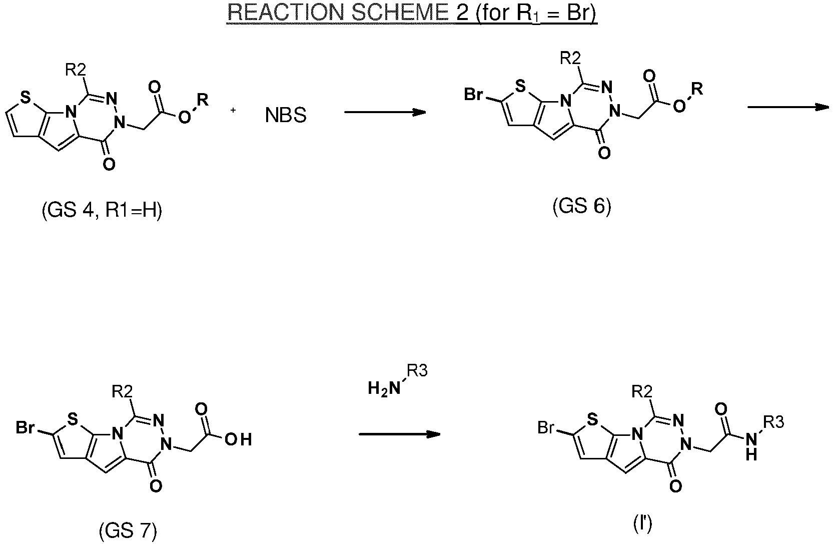

Compounds of the present disclosure with R₁=Br may be prepared by the reaction scheme 2 where the ester intermediate in which R₁=H (GS 4, R₁=H) is reacted with a brominated agent like NBS to obtain the corresponding brominated derivative (GS 6), which is then saponified using an aqueous base to obtain the carboxylic acid (GS 7), followed by a coupling with an appropriate amine H₂N-R₃ using known standard methods like EDC/HOBT Et₃N, HATU DIPEA to provide a compound of general formula (I').

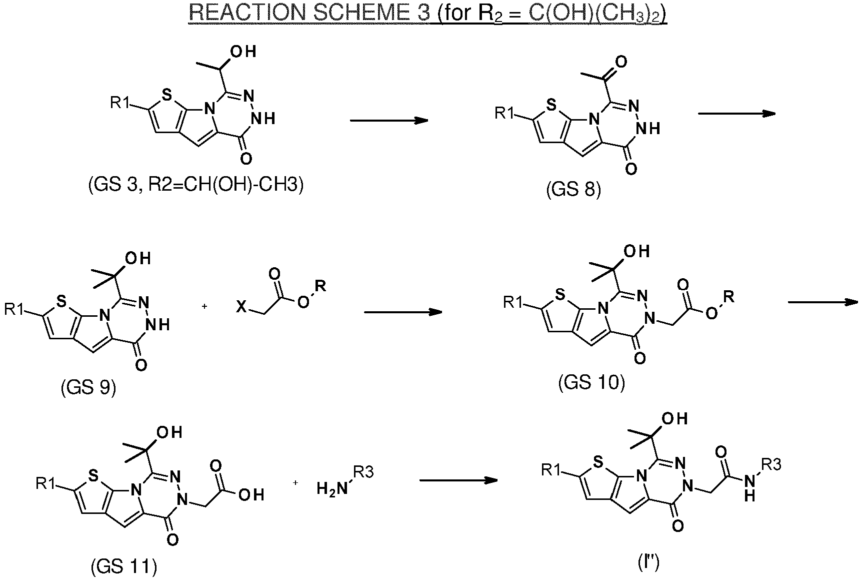

A modified reaction sequence of scheme 1 is applied for R₂ = C(OH)(CH₃)₂. Compounds of general formula (I), where R₂ is 2-hydroxypropan-2-yl, can be prepared by the reaction scheme 4 where general structure (GS 3 with R₂ = CH(OH)-CH₃) is oxidized with standard methods like Swern oxidation or Dess-Martin oxidation to provide a ketone (GS 8) which is engaged in a Grignard reaction using for example MeMgCl to obtain (GS 9). This intermediate is then alkylated with alkyl haloacetate using a base like K₂CO₃ to give ester (GS 10), which is then saponified to the corresponding carboxylic acid (GS 11), followed by a coupling with an appropriate amine H₂N-R₃ using known standard methods like EDC/HOBT Et₃N, HATU DIPEA to provide a compound of general formula (I").

In the following schemes, the starting compounds and the reagents, when their preparation method is not described, are commercially available or described in the literature, or else may be prepared according to methods that are described therein or that are known to those skilled in the art.

The following abbreviations and empirical formulae are used:
- ACN: acetonitrile
- DAST: diethylaminosulfur trifluoride
- DCM: dichloromethane
- Cs₂CO₃: cesium carbonate
- DIPEA: diisopropylethylamine
- DMF: N,N-dimethylformamide
- DMSO: dimethylsulfoxide
- EDC: 1-ethyl-3-(3-dimethylaminopropyl)carbodiimide
- Et₂O: diethylether
- EtOAc: ethylacetate
- Et₃N: triethylamine
- EtOH: ethanol
- FA: formic acid
- HATU: hexafluorophosphate Azabenzotriazole Tetramethyl Uronium
- HOBT: hydroxybenzotriazole
- HCl: hydrochloric acid
- IL 1b: Interleukin 1 beta
- K₂CO₃: potassium carbonate
- MeOH: methanol
- NaHCO₃: sodium bicarbonate
- Na₂CO₃: sodium carbonate
- Na₂S₂O₃: sodium thiosulfate
- Na₂SO₄: sodium sulfate
- NaOH: sodium hydroxyde
- NBS: N-bromosuccinimide
- NH₄Cl: ammonium chloride
- TMSO: trimethylsilyloxy
- P₂O₅: phosphorous pentoxyde
- tBuOK: potassium tertbutylate
- TFA: trifluoroacetic acid
- THF: tetrahydrofurane
- TNF-α: Tumor necrosis factor-*α*
- rt: room temperature
- Rt: Retention time
- °C: degree Celsius
- ml: milliliter(s)
- mmol: millimole(s)
- min: minute(s)
- µmol: micromole(s)
- µl: microliter(s)
- h: hour(s)

The examples which follow describe the preparation of certain compounds in accordance with the present disclosure. These examples are not limitative, and merely illustrate the present disclosure. The numbers of the compounds exemplified match those given in the above list of compounds and the table of IC₅₀ values, which illustrates the chemical structures and physical properties of some compounds according to the present disclosure.

In the tables:
**NMR:** the proton magnetic resonance spectra (¹H NMR), as described below, are recorded at 400 MHz or 500 MHz in DMSO-d₆, using the DMSO-d₆ peak as reference. The chemical shifts (δ) are expressed in parts per million (ppm). The signals observed are expressed as follows: s = singlet; d = doublet; t = triplet; m = multiplet or br s = broad singlet; br m = broad multiplet.
**LCMS:** the LCMS characteristics, as described below, indicates the different high-performance liquid chromatography analytical methods used.

### LCMS final compounds:

### Method A:

System Waters UPLC/SQD; ionization: electrospray in positive and/or negative mode (ES+/-) Column: ACQUITY CORTECS C18+ 1.7µm 2.1x50mm; Column temperature: 40°C; Flow: 1.0 ml/min; Solvents: A = H₂O (0.1% formic acid) ; B = ACN (0.1% formic acid)

### Gradient:

| (min) | %A | %B |
|---|---|---|
| 0 | 98 | 2 |
| 2.00 | 0 | 100 |
| 2.60 | 0 | 100 |
| 2.70 | 98 | 2 |
| 3.00 | 98 | 2 |

### Method B:

System Waters UPLC/SQD2; ionization: electrospray in positive &/or negative mode (ES+/-) Column : ACQUITY CSH C18+ 1.7µm 2.1x50mm; Column temperature: 60°C; Flow: 1.0 ml/min; Solvents: A = H₂O (0.1% formic acid); B = ACN (0.1% formic acid)

### Gradient:

| (min) | %A | %B |
|---|---|---|
| 0 | 97 | 3 |
| 2.10 | 0 | 100 |
| 2.45 | 0 | 100 |
| 2.50 | 97 | 3 |

### Method C:

System Waters UPLC/SQD; ionization: electrospray in positive &/or negative mode (ES+/-) Column: ACQUITY CORTECS C18+ 1.7µm 2.1x50mm; Column temperature: 40°C; Flow: 1.0 ml/min; Solvents: A = H2O (0.1% formic acid) ; B = ACN (0.1% formic acid)

### Gradient:

| (min) | %A | %B |
|---|---|---|
| 0 | 98 | 2 |
| 1.00 | 98 | 2 |
| 7.50 | 0 | 100 |
| 9.20 | 0 | 100 |
| 9.30 | 98 | 2 |

### Method D:

System Waters XeVo - Qtof; ionization: electrospray in positive and/or negative mode (ES+) Column: ACQUITY CSH C18+ 1.7µm 2.1x100mm; Column temperature: 45°C; Flow: 0.5 ml/min; Solvents: A = H₂O (0.1% formic acid); B = ACN (0.1% formic acid)

### Gradient:

| (min) | %A | %B |
|---|---|---|
| 0 | 95 | 5 |
| 0.3 | 95 | 5 |
| 4.00 | 0 | 100 |
| 4.60 | 0 | 100 |
| 5.30 | 95 | 5 |

### LCMS, intermediates

### Methods E and F:

LC-MS: system Waters UPLC Hclass / SQD2; ionization: electrospray in positive and/or negative mode (ES+/-); Column: Cortecs uplc C18 1.6µm 2.1x50mm; Column temperature: 55°C; Flow: 0.8 ml/min; Solvents: A = H₂O (0.1% formic acid); B = ACN (0.1% formic acid)

### Gradient:

| Method E (2 min): | | | | | |
|---|---|---|---|---|---|
| (min) | %A | %B | Metod F (3 min): | | |
| | | | (min) | %A | %B |
| 0 | 95 | 5 | | | |
| 1.00 | 50 | 50 | 0 | 98 | 2 |
| 1.30 | 0 | 100 | 2.50 | 0 | 100 |
| 1.45 | 0 | 10 | 2.90 | 0 | 100 |
| 1.75 | 98 | 2 | 2.95 | 98 | 2 |
| 2.00 | 98 | 2 | 3.00 | 98 | 2 |

Mass spectrometry results are reported as the ratio of mass over charge.

### Example 1 ; (R)-2-(8-isopropyl-5-oxothieno[3',2':4,5]pyrrolo[1,2-d][1,2,4]triazin-6(5H)-yl)-N-(1-methylpiperidin-3-yl)acetamide

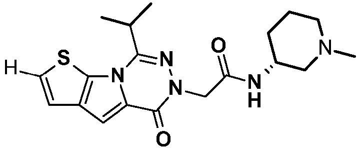

### Int 1: 6H-thieno[2,3-b]pyrrole-5-carbohydrazide

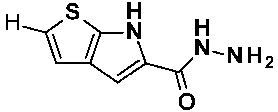

To a solution of methyl 6*H*-thieno[2,3-b]pyrrole-5-carboxylate (15.45 mmol, 2,8 g) in 15 ml of MeOH, hydrazine solution (50-60% in water) (61.13 mmol, 6ml) is added, and this mixture is heated at reflux overnight. After cooling down the solution, the precipitate formed is filtered off, washed with Et₂O and dried under vacuum. The title compound is obtained as a white solid (2.56 g, 91% yield).

LCMS (method E): Rt = 0.61 min; MS m/z [M+H]⁺ 182

### Int 2: 8-isopropylthieno[3',2':4,5]pyrrolo[1,2-d][1,2,4]triazin-5(6H)-one

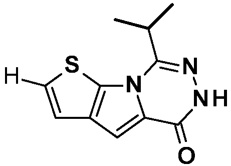

Under nitrogen, to a solution of 6H-thieno[2,3-b]pyrrole-5-carbohydrazide (1,55 g, 8,55 mmol) in 20 ml of DMF is added methyl 2-methylpropanimidoate hydrochloride (1,46 g, 10,26 mmol). This mixture is stirred at rt for 30 min. Then, solid tBuOK (2.15g, 18.82 mmol) is added and the resulting mixture is stirred in a preheated oil bath at 90°C for 1 h. After cooling down to 0°C, 70 ml of water is added, and the mixture is acidified with HCI (1M) to pH 5. The precipitate formed is filtered off, washed with water and EtOAc/CH₂Cl₂ (9/1). The solid obtained is washed with pentane and dried under vacuum to give the title compound as a beige solid (1.0 g, 50% yield).

LCMS (method E): Rt = 1.21 min; MS m/z [M+H]⁺ 234

### Int 3: Ethyl 2-(8-isopropyl-5-oxothieno[3',2':4,5]pyrrolo[1,2-d][1,2,4]triazin-6(5H)-yl)acetate

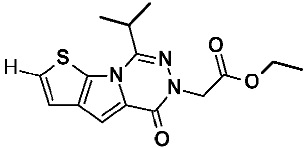

Under nitrogen, to a solution of 8-isopropylthieno[3',2':4,5]pyrrolo[1,2-d][1,2,4]triazin-5(6H)-one (717 mg, 3,07 mmol) in 10 ml of DMF is added K₂CO₃ (637 mg, 4,61 mmol), followed by a dropwise addition of ethyl iodoacetate (557 µl, 4,61 mmol). The resulting mixture is stirred at rt for 1h. Then, the mixture is diluted with a saturated aqueous solution of NH₄Cl and extracted twice with EtOAc. The combined organic layers are dried over Na₂SO₄, filtered off and concentrated under reduced pressure. The residue is purified by flash chromatography on silica gel using cyclohexane/EtOAc (from 0% to 50%) to give the title compound as a white solid (620 mg, 63% yield).

LCMS (method E): Rt = 1.46 min; MS m/z [M+H]⁺ 320

### Int 4: 2-(8-isopropyl-5-oxothieno[3',2':4,5]pyrrolo[1,2-d][1,2,4]triazin-6(5H)-yl)acetic acid

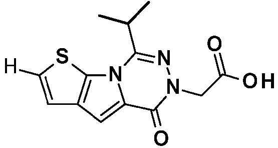

To a solution of ethyl 2-(8-isopropyl-5-oxothieno[3',2':4,5]pyrrolo[1,2-d][1,2,4]triazin-6(5H)-yl)acetate (620 mg, 1,94 mmol) in 20 ml of dioxane is added a solution of NaOH (1M) (2,91 ml, 2,91 mmol). The resulting solution is stirred at rt overnight.

The mixture is acidified with HCI (1M). The precipitate formed is filtered off, washed with water and pentane. After drying overnight under vacuum in presence of P₂O₅, the title compound is obtained as a white solid (433 mg, 77% yield).

LCMS (method E): Rt = 1.22 min; MS m/z [M+H]⁺ 292

### Ex 1: (R)-2-(8-isopropyl-5-oxothieno[3',2':4,5]pyrrolo[1,2-d][1,2,4]triazin-6(5H)-yl)-N-(1-methylpiperidin-3-yl)acetamide

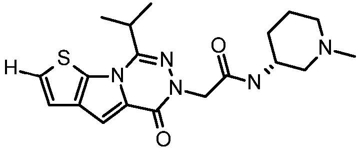

Under nitrogen, to a suspension of (3*R*)-1-methylpiperidin-3-amine dihydrochloride (22 mg, 113 µmol) and 2-(8-isopropyl-5-oxothieno[3',2':4,5]pyrrolo[1,2-d][1,2,4]triazin-6(5H)-yl)acetic acid (30 mg, 103 µmol) in 2 ml of DMF is added DIPEA (54 µl, 0,31 mmol), followed by HATU (51 mg, 134 µmol). The resulting mixture is stirred at rt for 20h. The crude mixture is purified by reverse phase chromatography Waters SunFire PrepC18 OBD 30 x 100 mm column, water containing 0.1% HCO₂H with a gradient of acetonitrile 10 to 80% in 18 min. After evaporation under reduced pressure and drying overnight, the title compound is obtained as a yellow solid (24 mg, 60% yield).

1H NMR (400 MHz, DMSO-d6) δ ppm 1.24 (m, 1 H), 1.35 (d, J=6.8 Hz, 6 H), 1.48 (qd, J=10.2, 3.9 Hz, 1 H), 1.62 - 1.72 (m, 2 H), 1.92 (m, 1 H), 2.02 (m, 1 H), 2,21 (s, 3H), 2.57 (m, 1 H), 2.71 (m, 1 H), 3.37 (m, 1 H), 3.77 (m, 1 H), 4.55 (s, 2 H), 7.32 (d, J=5.5 Hz, 1 H), 7.36 (s, 1 H), 7.58 (d, J=5.5 Hz, 1 H), 7.92 (d, J=7.8 Hz, 1 H), 8.14 (s, 1 H)

LCMS [M+H]⁺ = 388; Rt = 0.65; Method A

The following examples were synthesized analogous to the previous procedure, using the appropriate amines:

| Ex N° | Name | NMR | LCMS Rt (min) | LCMS [M+H]+ | LCMS Analytical method |
|---|---|---|---|---|---|
| 2 | 2-(8-isopropyl-5-oxothieno[3',2':4,5]pyrrolo[1,2 -d][1,2,4]triazin-6(5H)-yl)-N-(thiazol-5-yl)acetamide | 1H NMR (400 MHz, DMSO-d6) δ ppm 1.36 (d, J=6.5 Hz, 6 H), 3.39 (sept, J=6,5 Hz, 1 H), 4.85 (s, 2 H), 7.33 (d, J=5.5 Hz, 1 H), 7.41 (s, 1 H), 7.60 (d, J=5.5 Hz, 1 H), 7.64 (d, J=0.8 Hz, 1 H), 8.60 (br s, 1 H), 11.54 (br s, 1 H) | 1.06 | 374 | A |
| 3 | N-((R)-1-((S)-2-hydroxypropyl)piperidin-3-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.0 (d, J=6.1 Hz, 3 H), 1.35 (d, J=6.8 Hz, 6 H), 1.64 (m, 1 H), 1.71 - 1.85 (m, 2 H), 2.96 - 3.20 (m, 2 H) 3.35 (m, 1 H), 3.87 - 4.08 (m, 2 H), 4.56 (s, 2 H), 4.97 (br s, 1 H), 7.32 (d, J=5.5 Hz, 1 H), 7.37 (s, 1 H), 7.59 (d, J=5.5 Hz, 1 H), 8.04 (br d, J=6.4 Hz, 1 H) | 0.69 | 432 | A |
| 4 | (R)-N-(1-cyclopropylpiperidin-3-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 0.74 - 0.99 (m, 4 H), 1.36 (d, J=6.7 Hz, 6 H), 1.46 (m, 1 H), 1.64 (m, 1 H), 1.75 - 2.00 (m, 2 H), 2.89 (m, 2 H), 3.04 (m, 1 H), 3.37 (sept, *J*=6.7 Hz, 1 H), 3.45 (m, 2 H), 3.94 (m, 1 H), 4.58 (s, 2 H), 7.32 (d, J=5.4 Hz, 1 H), 7.38 (s, 1 H), 7.59 (d, J=5.4 Hz, 1 H), 8.29 (br d, *J*=6.7 Hz, 1 H), 9.06 (br s, 1 H) | 0.65 | 414 | A |
| 11 | (R)-N-(1-cyclobutylpiperidin-3-yl)-2-(8-isopropyl-5-oxothieno[3',2':4,5]pyrrolo[1,2 -d][1,2,4]triazin-6(5H)-yl)acetamide formate | 1H NMR (400 MHz, DMSO-d6) δ ppm 1.23 (br d, J=9 Hz, 1 H), 1.35 (d, J=7 Hz, 6 H), 1.40 - 2.00 (m, 12 H), 2.60 - 2.71 (m, 2 H), 3.21 - 3.37 (m, 1 H), 3.62 - 3.76 (m, 1 H), 4.55 (d, J=3 Hz, 2 H), 7.32 (d, J=5 Hz, 1 H), 7.37 (s, 1 H), 7.58 (d, J=6 Hz, 1 H), 7.83 (br d, J=8 Hz, 1 H) | 0.70 | 428 | A |
| 12 | N-(benzo[d]thiazol-6-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.37 (d, J=7 Hz, 6 H), 3.36 - 3.47 (m, 1 H), 4.84 (s, 2 H), 7.33 (d, J=5 Hz, 1 H), 7.41 (s, 1 H), 7.56 - 7.65 (m, 2 H), 8.03 (d, J=9 Hz, 1 H), 8.49 (d, J=2 Hz, 1 H), 9.27 (s, 1 H), 10.44 (s, 1 H) | 1.21 | 424 | A |
| 14 | (R)-N-(1-isobutylpiperidin-3-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 0.77 (d, J=6 Hz, 6 H), 1.10 - 1.55 (m, 8 H), 1.59 - 2.12 (m, 7 H), 2.65 (br d, J=12 Hz, 2 H), 3.35 - 3.43 (m, 1 H), 3.69 - 3.79 (m, 1 H), 4.47 - 4.64 (m, 2 H), 7.29 - 7.40 (m, 2 H), 7.58 (d, J=5 Hz, 1 H), 7.73 (br d, J=8 Hz, 1 H) | 0.74 | 430 | A |
| 15 | N-(benzo[d]thiazol-5-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.38 (d, J=7 Hz, 6 H), 3.37 - 3.47 (m, 1 H), 4.85 (s, 2 H), 7.33 (d, J=5 Hz, 1 H), 7.41 (s, 1 H), 7.56 - 7.67 (m, 2 H), 8.09 (d, J=9 Hz, 1 H), 8.40 - 8.47 (m, 1 H), 9.37 (s, 1 H), 10.44 (s, 1 H) | 1.28 | 424 | A |
| 16 | 2-(8-isopropyl-5-oxothieno[3',2':4,5]pyrrolo[1,2 -d][1,2,4]triazin-6(5H)-yl)-N-(6-methoxypyridin-3-yl)acetamide | 1H NMR (400 MHz, DMSO-d6) δ ppm 1.37 (d, J=7 Hz, 6 H), 3.34 - 3.44 (m, 1 H), 3.82 (s, 3 H), 4.78 (s, 2 H), 6.81 (d, J=9 Hz, 1 H), 7.33 (d, J=6 Hz, 1 H), 7.40 (s, 1 H), 7.60 (d, J=5 Hz, 1 H), 7.86 (dd, J=9, 3 Hz, 1 H), 8.34 (d, J=3 Hz, 1 H), 10.15 (s, 1 H) | 1.16 | 398 | A |
| 17 | (R)-N-(1-(cyclopentylmethyl)piperidin-3-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.09 (br dd, J=13, 7 Hz, 2 H), 1.27 - 1.52 (m, 12 H), 1.55 - 1.76 (m, 4 H), 1.98 - 2.09 (m, 2 H), 2.13 - 2.22 (m, 1 H), 2.29 - 2.48 (m, 2 H), 2.66 - 2.86 (m, 2 H), 3.27 - 3.38 (m, 1 H), 3.81 (br s, 1 H), 4.49 - 4.62 (m, 2 H), 7.32 (d, J=5 Hz, 1 H), 7.38 (s, 1 H), 7.59 (d, J=5 Hz, 1 H), 7.79 (br s, 1 H) | 0.82 | 456 | A |
| 18 | N-((3R)-1-((2,2-difluorocyclopropyl)methyl)pip eridin-3-yl)-2-(8-isopropyl-5-oxothieno[3',2':4,5]pyrrolo[1,2 -d][1,2,4]triazin-6(5H)-yl)acetamide compound with formaldehyde | 1H NMR (400 MHz, DMSO-d6) δ ppm 1.08 - 1.30 (m, 2 H), 1.35 (d, J=7 Hz, 6 H), 1.42 - 1.59 (m, 2 H), 1.62 - 1.81 (m, 3 H), 1.86 - 2.07 (m, 2 H), 2.27 - 2.39 (m, 1 H), 2.53 - 2.69 (m, 2 H), 2.76 (br dd, J=16, 11 Hz, 1 H), 3.37 - 3.46 (m, 1 H), 3.76 (br d, J=9 Hz, 1 H), 4.50 - 4.57 (m, 2 H), 7.32 (d, J=5 Hz, 1 H), 7.37 (s, 1 H), 7.58 (d, J=5 Hz, 1 H), 7.87 (br d, J=8 Hz, 1 H) | 0.70 | 464 | A |
| 19 | (R)-N-(1-(2-hydroxyethyl)piperidin-3-yl)-2-(8-isopropyl-5-oxothieno[3',2':4,5]pyrrolo[1,2 -d][1,2,4]triazin-6(5H)-yl)acetamide compound with formaldehyde | 1H NMR (400 MHz, DMSO-d6) δ ppm 1.19 - 1.31 (m, 1 H), 1.35 (d, J=7 Hz, 6 H), 1.41 - 1.52 (m, 1 H), 1.61 - 1.70 (m, 2 H), 1.99 (br t, J=10 Hz, 1 H), 2.04 - 2.14 (m, 1 H), 2.38 - 2.45 (m, 2 H), 2.62 - 2.69 (m, 1 H), 2.73 - 2.80 (m, 1 H), 3.35 (br dd, J=13, 7 Hz, 1 H), 3.48 (br t, J=6 Hz, 2 H), 3.72 - 3.83 (m, 1 H), 4.55 (d, J=2 Hz, 2 H), 7.32 (d, J=6 Hz, 1 H), 7.37 (s, 1 H), 7.58 (d, J=5 Hz, 1 H), 7.88 (d, J=8 Hz, 1 H) | 0.61 | 418 | A |
| 23 | (R)-N-(1-(2-hydroxy-2-methylpropyl)piperidin-3-yl)-2-(8-isopropyl-5-oxothieno[3',2':4,5]pyrrolo[1,2 -d][1,2,4]triazin-6(5H)-yl)acetamide formate | 1H NMR (400 MHz, DMSO-d6) δ ppm 0.98 (s, 6 H), 1.22 (br d, J=9 Hz, 1 H), 1.35 (br d, J=7 Hz, 6 H), 1.46 (br d, J=9 Hz, 1 H), 1.62 (br d, J=9 Hz, 2 H), 2.06 - 2.21 (m, 4 H), 2.60 - 2.82 (m, 2 H), 3.39 - 3.51 (m, 1 H), 3.75 (br s, 1 H), 4.48 - 4.60 (m, 2 H), 7.29 - 7.39 (m, 2 H), 7.58 (d, J=5 Hz, 1 H), 7.73 (br d, J=8 Hz, 1 H) | 0.65 | 446 | A |
| 25 | N-((R)-1-cyclobutylpiperidin-3-yl)-2-(8-isopropyl-5-oxothieno[3',2':4,5]pyrrolo[1,2 -d][1,2,4]triazin-6(5H)-yl)propanamide formate | 1H NMR (400 MHz, DMSO-d6) δ ppm 1.14 - 1.29 (m, 1 H), 1.33 - 1.38 (m, 6 H), 1.41 - 1.78 (m, 12 H), 1.91 (dt, J=6, 3 Hz, 2 H), 2.54 - 2.61 (m, 1 H), 2.63 - 2.72 (m, 2 H), 3.33 - 3.39 (m, 1 H), 3.69 - 3.81 (m, 1 H), 5.32 (dd, J=7, 5 Hz, 1 H), 7.32 (d, J=5 Hz, 1 H), 7.37 (s, 1 H), 7.40 - 7.54 (m, 1 H), 7.58 (dd, J=5, 1 Hz, 1 H) | 0.75 | 442 | A |
| 26 | N-((R)-1-cyclopropylpiperidin-3-yl)-2-(8-isopropyl-5-oxothieno[3',2':4,5]pyrrolo[1,2 -d][1,2,4]triazin-6(5H)-yl)propanamide formate | 1H NMR (400 MHz, DMSO-d6) δ ppm 0.14 - 0.26 (m, 2 H), 0.30 - 0.40 (m, 2 H), 1.15 - 1.27 (m, 1 H), 1.28 - 1.42 (m, 7 H), 1.42 - 1.67 (m, 6 H), 1.90 - 2.15 (m, 2 H), 2.69 - 2.87 (m, 2 H), 3.33 - 3.39 (m, 1 H), 3.61 - 3.73 (m, 1 H), 5.30 (q, J=7 Hz, 1 H), 7.32 (d, J=5 Hz, 1 H), 7.37 (d, J=1 Hz, 1 H), 7.39 - 7.52 (m, 1 H), 7.58 (d, J=5 Hz, 1 H) | 0.72 | 428 | A |
| 27 | 2-(8-isopropyl-5-oxothieno[3',2':4,5]pyrrolo[1,2 -d][1,2,4]triazin-6(5H)-yl)-N-(1-methyl-1H-indazol-6-yl)acetamide formate | 1H NMR (400 MHz, DMSO-d6) δ ppm 1.38 (d, J=7 Hz, 6 H), 3.38 - 3.50 (m, 1 H), 3.94 (s, 3 H), 4.85 (s, 2 H), 7.13 (dd, J=9, 2 Hz, 1 H), 7.34 (d, J=5 Hz, 1 H), 7.42 (s, 1 H), 7.61 (d, J=6 Hz, 1 H), 7.70 (d, J=9 Hz, 1 H), 7.95 (s, 1 H), 8.08 (s, 1 H), 10.39 (s, 1 H) | 1.23 | 421 | A |
| 28 | (R)-N-(1-(cyclopropylmethyl)piperidin-3-yl)-2-(8-isopropyl-5-oxothieno[3',2':4,5]pyrrolo[1,2 -d][1,2,4]triazin-6(5H)-yl)acetamide formate | 1H NMR (400 MHz, DMSO-d6) δ ppm 0.08 - 0.19 (m, 2 H), 0.48 (dd, J=8, 2 Hz, 2 H), 0.85 (br d, J=5 Hz, 1 H), 1.35 (d, J=7 Hz, 7 H), 1.54 (br d, J=11 Hz, 1 H), 1.69 - 1.77 (m, 2 H), 2.05 - 2.30 (m, 2 H), 2.38 - 2.44 (m, 2 H), 2.86 - 3.06 (m, 2 H), 3.26 - 3.40 (m, 1 H), 3.75 - 3.93 (m, 1 H), 4.52 - 4.58 (m, 2 H), 7.32 (d, J=5 Hz, 1 H), 7.37 (s, 1 H), 7.58 (d, J=6 Hz, 1 H), 7.95 (br d, J=8 Hz, 1 H) | 0.70 | 428 | A |
| 35 | N-(5,5-difluoro-1-methylpiperidin-3-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.35 (d, J=7 Hz, 6 H), 1.63 - 1.88 (m, 1 H), 1.95 (t, J=10 Hz, 1 H), 2.09 - 2.39 (m, 5 H), 2.70 - 3.00 (m, 2 H), 3.34 - 3.42 (m, 1 H), 3.91 - 4.06 (m, 1 H), 4.56 (s, 2 H), 7.32 (d, J=6 Hz, 1 H), 7.38 (s, 1 H), 7.58 (d, J=6 Hz, 1 H), 7.99 (d, J=8 Hz, 1 H) | 0.74 | 424 | A |
| 36 | (R)-N-(1-(2-fluoroethyl)piperidin-3-yl)-2-(8-isopropyl-5-oxothieno[3',2':4,5]pyrrolo[1,2 -d][1,2,4]triazin-6(5H)-yl)acetamide formate | 1H NMR (400 MHz, DMSO-d6) δ ppm 1.18 - 1.51 (m, 8 H), 1.62 - 1.73 (m, 2 H), 1.90 - 2.08 (m, 2 H), 2.58 (t, J=5 Hz, 2 H), 2.63 - 2.73 (m, 2 H), 2.80 (dd, J=10, 3 Hz, 1 H), 3.70 - 3.79 (m, 1 H), 4.41 - 4.65 (m, 4 H), 7.32 (d, J=5 Hz, 1 H), 7.37 (s, 1 H), 7.59 (d, J=5 Hz, 1 H), 7.86 (d, J=8 Hz, 1 H) | 0.63 | 420 | A |
| 37 | N-(1-(tert-butyl)piperidin-3-yl)-2-(8-isopropyl-5-oxothieno[3',2':4,5]pyrrolo[1,2 -d][1,2,4]triazin-6(5H)-yl)acetamide formate | 1H NMR (400 MHz, DMSO-d6) δ ppm 0.98 (s, 9 H), 1.35 (d, J=7 Hz, 8 H), 1.67 (br d, J=10 Hz, 2 H), 1.97 - 2.25 (m, 2 H), 2.73 - 2.93 (m, 2 H), 3.46 - 3.56 (m, 1 H), 3.66 - 3.79 (m, 1 H), 4.48 - 4.63 (m, 2 H), 7.32 (d, J=5 Hz, 1 H), 7.38 (s, 1 H), 7.58 (d, J=6 Hz, 1 H), 7.77 (br d, J=8 Hz, 1 H) | 0.67 | 430 | A |
| 38 | 2-(8-isopropyl-5-oxothieno[3',2':4,5]pyrrolo[1,2 -d][1,2,4]triazin-6(5H)-yl)-N-(1-(2,2,2-trifluoroethyl)piperidin-3-yl)acetamide 2,2,2-trifluoroacetate | 1H NMR (400 MHz, DMSO-d6) δ ppm 1.16 - 1.30 (m, 1 H), 1.35 (d, J=7 Hz, 6 H), 1.39 - 1.53 (m, 1 H), 1.61 - 1.74 (m, 2 H), 2.18 (t, J=10 Hz, 1 H), 2.25 - 2.36 (m, 1 H), 2.70 - 2.80 (m, 1 H), 2.87 - 2.98 (m, 1 H), 3.12 - 3.26 (m, 2 H), 3.32 - 3.41 (m, 1 H), 3.72 (br d, J=9 Hz, 1 H), 4.49 - 4.64 (m, 2 H), 7.32 (d, J=5 Hz, 1 H), 7.37 (s, 1 H), 7.58 (d, J=5 Hz, 1 H), 7.85 (br d, J=8 Hz, 1 H) | 1.25 | 456 | A |
| 39 | 2-(8-isopropyl-5-oxothieno[3',2':4,5]pyrrolo[1,2 -d][1,2,4]triazin-6(5H)-yl)-N-(quinuclidin-3-yl)acetamide | 1H NMR (400 MHz, DMSO-d6) δ ppm 1.35 (d, J=7 Hz, 6 H), 1.68 - 1.93 (m, 3 H), 2.01 - 2.12 (m, 2 H), 2.97 (br dd, J=13, 4 Hz, 1 H), 3.14 - 3.26 (m, 4 H), 3.36 - 3.45 (m, 1 H), 3.52 - 3.67 (m, 1 H), 4.13 (br d, J=5 Hz, 1 H), 4.57 - 4.67 (m, 2 H), 7.32 (d, J=5 Hz, 1 H), 7.38 (s, 1 H), 7.59 (d, J=5 Hz, 1 H), 8.41 (d, J=6 Hz, 1 H) | 0.62 | 400 | A |
| 40 | (R)-2-(8-isopropyl-5-oxothieno[3',2':4,5]pyrrolo[1,2 -d][1,2,4]triazin-6(5H)-yl)-N-(1-(oxetan-3-yl)piperidin-3-yl)acetamide | 1H NMR (400 MHz, DMSO-d6) δ ppm 1.22(m, 1 H), 1.34 (d, J=7 Hz, 6 H), 1.48 (m, 1 H), 1.63 - 1.77 (m, 3 H), 1.83 (t, J=10 Hz, 1 H), 2.41 - 2.47 (m, 1 H), 2.52 - 2.59 (m, 1 H), 3.38 (m, 2 H), 3.76 (m, 1 H), 4.38 (dt, J=9, 6 Hz, 2 H), 4.50 (td, J=6, 1 Hz, 2 H), 4.55 (d, J=2 Hz, 2 H), 7.32 (d, J=6 Hz, 1 H), 7.37 (s, 1 H), 7.59 (d, J=5 Hz, 1 H), 7.92 (d, J=8 Hz, 1 H) | 1,02 | N/A | B |
| 41 | (R)-N-(1-(3-fluoropropyl)piperidin-3-yl)-2-(8-isopropyl-5-oxothieno[3',2':4,5]pyrrolo[1,2 -d][1,2,4]triazin-6(5H)-yl)acetamide formate | 1H NMR (400 MHz, DMSO-d6) δ ppm 1.20 - 1.50 (m, 8 H), 1.62 - 1.99 (m, 6 H), 2.32 - 2.44 (m, 2 H), 2.53 - 2.62 (m, 1 H), 2.66 - 2.73 (m, 1 H), 3.39 - 3.48 (m, 1 H), 3.70 - 3.78 (m, 1 H), 4.36 - 4.60 (m, 4 H), 7.32 (d, J=5 Hz, 1 H), 7.38 (s, 1 H), 7.59 (d, J=5 Hz, 1 H), 7.84 (d, J=8 Hz, 1 H) | 0.65 | 434 | A |
| 42 | (R)-N-(1-acetylpiperidin-3-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) Mixture of Isomers majo 60% : δ ppm 1.25 (m, 1 H), 1.35 (m, 6 H), 1.39 - 1.58 (m, 2 H), 1.62 - 1.86 (m, 2 H), 1.92 - 1.98 (m, 3 H), 2.47 (m, 1 H), 3.16 (m, 1 H), 3.38 (m, 1 H), 3.53 - 3.70 (m, 2 H), 4.49 - 4.66 (m, 2 H), 7.32 (d, J=5 Hz, 1 H), 7.37 (s, 1 H), 7.59 (d, J=5 Hz, 1 H), 8.13 (d, J=7 Hz, 1 H) | 1.38 | N/A | B |
| 43 | (R)-N-(1-((3,3-difluorocyclobutyl)methyl)pipe ridin-3-yl)-2-(8-isopropyl-5-oxothieno[3',2':4,5]pyrrolo[1,2 -d][1,2,4]triazin-6(5H)-yl)acetamide formate | 1H NMR (400 MHz, DMSO-d6) δ ppm 1.18 - 1.31 (m, 1 H), 1.35 (d, J=7 Hz, 6 H), 1.45 (br dd, J=10, 3 Hz, 1 H), 1.61 - 1.71 (m, 2 H), 1.89 - 2.07 (m, 2 H), 2.13 - 2.38 (m, 3 H), 2.39 - 2.46 (m, 2 H), 2.52 - 2.73 (m, 4 H), 3.33 - 3.45 (m, 1 H), 3.70 - 3.78 (m, 1 H), 4.45 - 4.64 (m, 2 H), 7.32 (d, J=6 Hz, 1 H), 7.37 (s, 1 H), 7.58 (d, J=5 Hz, 1 H), 7.81 (br d, J=8 Hz, 1 H) | 0.72 | 478 | A |
| 45 | (R)-2-(8-isopropyl-5-oxothieno[3',2':4,5]pyrrolo[1,2 -d][1,2,4]triazin-6(5H)-yl)-N-(1-(1-methyl-1H-imidazol-2-yl)piperidin-3-yl)acetamide | 1H NMR (400 MHz, DMSO-d6) δ ppm 1.31 (d, J=8 Hz, 3 H), 1.34 (d, J=8 Hz, 3 H), 1.45 (m, 1 H), 1.61 (m, 1 H), 1.70 - 1.83 (m, 2 H), 2.62 - 2.76 (m, 2 H), 3.02(m, 1 H), 3.12 (dd, J=12, 3 Hz, 1 H), 3.36 (m, 1 H), 3.40 (s, 3 H), 3.88 (m, 1 H), 4.58 (q, J=15 Hz, 2 H), 6.41 (d, J=1 Hz, 1 H), 6.80 (d, J=1 Hz, 1 H), 7.32 (d, J=5 Hz, 1 H), 7.38 (s, 1 H), 7.59 (d, J=5 Hz, 1 H), 8.20 (d, J=8 Hz, 1 H) | 1,08 | 454 | B |
| 46 | 2-(8-isopropyl-5-oxothieno[3',2':4,5]pyrrolo[1,2 -d][1,2,4]triazin-6(5H)-yl)-N-(1-methyl-2-oxopiperidin-4-yl)acetamide | 1H NMR (400 MHz, DMSO-d6) δ ppm 1.36 (d, J=7 Hz, 6 H), 1.69 - 1.79 (m, 1 H), 1.89 - 1.97 (m, 1 H), 2.16 (dd, J=17, 8 Hz, 1 H), 2.44 - 2.47 (m, 1 H), 2.81 (s, 3 H), 3.22 - 3.30 (m, 2 H), 3.36 - 3.46 (m, 1 H), 4.00 - 4.09 (m, 1 H), 4.57 (s, 2 H), 7.32 (d, J=5 Hz, 1 H), 7.38 (s, 1 H), 7.59 (d, J=6 Hz, 1 H), 8.15 (d, J=7 Hz, 1 H) | 0.91 | 402 | A |
| 47 | 2-(8-isopropyl-5-oxothieno[3',2':4,5]pyrrolo[1,2 -d][1,2,4]triazin-6(5H)-yl)-N-(1-methyl-6-oxopiperidin-3-yl)acetamide | 1H NMR (400 MHz, DMSO-d6) δ ppm 1.35 (d, J=7 Hz, 6 H), 1.74 - 1.91 (m, 2 H), 2.21 - 2.42 (m, 2 H), 2.79 (s, 3 H), 3.10 (dd, J=12, 7 Hz, 1 H), 3.34 - 3.46 (m, 2 H), 4.04 - 4.13 (m, 1 H), 4.53 - 4.64 (m, 2 H), 7.32 (d, J=5 Hz, 1 H), 7.37 (s, 1 H), 7.58 (d, J=6 Hz, 1 H), 8.25 (d, J=7 Hz, 1 H) | 0.89 | 402 | A |
| 48 | 2-(12-isopropyl-9-oxo-3-thia-1,10,11-triazatricyclo[6.4.0.02,6]dodec a-2(6),4,7,11-tetraen-10-yl)-N-[(3R)-1-isopropyl-3-piperidyl]acetamide | 1H NMR (400 MHz, DMSO-d6) δ ppm 1.11 (br d, J=5 Hz, 6 H), 1.35 (d, J=7 Hz, 7 H), 1.60 (br d, J=10 Hz, 1 H), 1.81 (br s, 2 H), 2.47 - 2.58 (m, 2 H), 2.98 - 3.17 (m, 2 H), 3.24 - 3.30 (m, 1 H), 3.37 - 3.42 (m, 1 H), 3.91 (br s, 1 H), 4.52 - 4.63 (m, 2 H), 7.32 (d, J=5 Hz, 1 H), 7.38 (s, 1 H), 7.59 (d, J=5 Hz, 1 H), 8.05 (br s, 1 H) | 0.66 | 416 | A |
| 49 | N-[(3R)-1-cyclopentyl-3-piperidyl]-2-(12-isopropyl-9-oxo-3-thia-1,10,11-triazatricyclo[6.4.0.02,6]dodec a-2(6),4,7,11-tetraen-10-yl)acetamide | 1H NMR (400 MHz, DMSO-d6) δ ppm 1.35 (d, J=7 Hz, 14 H), 1.71 - 1.80 (m, 2 H), 1.84 (br s, 2 H), 2.18 - 2.44 (m, 2 H), 3.01 (br d, J=13 Hz, 3 H), 3.34 - 3.46 (m, 1 H), 3.84 (br s, 1 H), 4.52 - 4.62 (m, 2 H), 7.32 (d, J=5 Hz, 1 H), 7.38 (s, 1 H), 7.59 (d, J=6 Hz, 1 H), 7.98 (br s, 1 H) | 0.71 | 442 | A |
| 53 | 2-(12-isopropyl-9-oxo-3-thia-1,10,11-triazatricyclo [6.4.0.02,6]dodeca-2(6),4,7,11-tetraen-10-yl)-N-(2-methylpyrazol-3-yl)acetamide | 1H NMR (400 MHz, DMSO-d6) δ ppm 1.37 (d, J=7 Hz, 6 H), 3.33 - 3.44 (m, 1 H), 3.67 (s, 3 H), 4.82 (s, 2 H), 6.15 (d, J=2 Hz, 1 H), 7.31 - 7.35 (m, 2 H), 7.36 - 7.43 (m, 1 H), 7.59 (d, J=5 Hz, 1 H), 10.12 (s, 1 H) | 0.99-1.02 | 371 | A |
| 54 | 2-(12-isopropyl-9-oxo-3-thia-1,10,11-triazatricyclo[6.4.0.02,6]dodec a-2(6),4,7,11-tetraen-10-yl)-N-(1-methylpyrazol-3-yl)acetamide | 1H NMR (400 MHz, DMSO-d6) δ ppm 1.36 (d, J=7 Hz, 6 H), 3.33 - 3.43 (m, 1 H), 3.74 (s, 3 H), 4.73 (s, 2 H), 6.39 (d, J=2 Hz, 1 H), 7.32 (d, J=5 Hz, 1 H), 7.38 (s, 1 H), 7.54 (d, J=2 Hz, 1 H), 7.59 (d, J=6 Hz, 1 H), 10.60 (s, 1 H) | 1.02 | 371 | A |
| 55 | N-[(1-tert-butyl-5-oxopyrrolidin-3-yl)methyl]-2-(12-isopropyl-9-oxo-3-thia-1,10,11-triazatricyclo [6.4.0.02,6]dodeca-2(6),4,7,11-tetraen-10-yl)acetamide | 1H NMR (500 MHz, DMSO-d6) δ ppm 1.29 (s, 9 H), 1.35 (dd, J=7, 2 Hz, 6 H), 1.90 - 2.02 (m, 1 H), 2.25 - 2.35 (m, 2 H), 3.03 - 3.15 (m, 3 H), 3.34 - 3.40 (m, 1 H), 3.45 (dd, J=10, 7 Hz, 1 H), 4.55 (s, 2 H), 7.32 (d, J=6 Hz, 1 H), 7.37 (s, 1 H), 7.58 (d, J=6 Hz, 1 H), 8.09 (br t, J=6 Hz, 1 H) | 1.09 | 444 | A |
| 56 | 2-(12-isopropyl-9-oxo-3-thia-1,10,11-triazatricyclo[6.4.0.02,6]dodec a-2(6),4,7,11-tetraen-10-yl)-N-(3-pyridyl)acetamide | 1H NMR (400 MHz, DMSO-d6) δ ppm 1.37 (d, J=7 Hz, 6 H), 3.34 - 3.44 (m, 1 H), 4.82 (s, 2 H), 7.30 - 7.38 (m, 2 H), 7.40 (s, 1 H), 7.60 (d, J=5 Hz, 1 H), 8.01 (d, J=9 Hz, 1 H), 8.29 (br d, J=4 Hz, 1 H), 8.73 (br s, 1 H), 10.37 (s, 1 H) | 0.79 | 368 | A |
| 57 | 2-(12-isopropyl-9-oxo-3-thia-1,10,11-triazatricyclo[6.4.0.02,6]dodec a-2(6),4,7,11-tetraen-10-yl)-N-[rac-(3R)-1-(2,2-dimethylcyclobutyl)-3-piperidyl]acetamide | 1H NMR (500 MHz, DMSO-d6) δ ppm 0.85 - 0.98 (m, 2 H), 1.03 (br d, J=10 Hz, 3 H), 1.23 - 1.41 (m, 9 H), 1.43 - 1.87 (m, 6 H), 2.41 - 2.70 (m, 6 H), 3.33 - 3.41 (m, 1 H), 3.76 (br s, 1 H), 4.49 - 4.61 (m, 2 H), 7.32 (d, J=6 Hz, 1 H), 7.38 (d, J=3 Hz, 1 H), 7.59 (d, J=6 Hz, 1 H) | 0.75 | 456 | A |
| 58 | 2-(12-isopropyl-9-oxo-3-thia-1,10,11-triazatricyclo[6.4.0.02,6]dodec a-2(6),4,7,11-tetraen-10-yl)-N-(2-pyridyl)acetamide | 1H NMR (500 MHz, DMSO-d6) δ ppm 1.36 (d, J=7 Hz, 6 H), 3.36 - 3.42 (m, 1 H), 4.86 (s, 2 H), 7.12 (ddd, J=7, 5, 1 Hz, 1 H), 7.33 (d, J=6 Hz, 1 H), 7.39 (s, 1 H), 7.60 (d, J=6 Hz, 1 H), 7.75 - 7.87 (m, 1 H), 8.00 (br d, J=8 Hz, 1 H), 8.29 - 8.42 (m, 1 H), 10.75 (s, 1 H) | 1.10 | 368 | A |
| 61 | N-[(3R)-1-cyclopropylazepan-3-yl]-2-(12-isopropyl-9-oxo-3-thia-1,10,11-triazatricyclo [6.4.0.02,6]dodeca-2(6),4,7,11-tetraen-10-yl)acetamide | 1H NMR (500 MHz, DMSO-d6) δ ppm -0.05 - 0.05 (m, 2 H), 0.13 (dd, J=6, 1 Hz, 2 H), 1.15 (d, J=7 Hz, 6 H), 1.18 - 1.53 (m, 6 H), 1.66 - 1.71 (m, 1 H), 2.38 - 2.51 (m, 3 H), 2.64 (dd, J=13, 4 Hz, 1 H), 3.17 (spt, J=7 Hz, 1 H), 3.57 - 3.64 (m, 1 H), 4.28 - 4.39 (m, 2 H), 7.12 (d, J=6 Hz, 1 H), 7.18 (s, 1 H), 7.38 (d, J=5 Hz, 1 H), 7.44 (br d, J=8 Hz, 1 H) | 0.64 | 428 | A |
| 62 | 2-(12-isopropyl-9-oxo-3-thia-1,10,11-triazatricyclo [6.4.0.02,6]dodeca-2(6),4,7,11-tetraen-10-yl)-N-[(3R)-1-methylazepan-3-yl]acetamide | 1H NMR (500 MHz, DMSO-d6) δ ppm 1.35 (d, J=7 Hz, 6 H), 1.42 - 1.64 (m, 5 H), 1.69 - 1.75 (m, 1 H), 2.27 (s, 3 H), 2.43 (dd, J=13, 8 Hz, 1 H), 2.50 - 2.53 (m, 2 H), 2.63 (dd, J=13, 4 Hz, 1 H), 3.30 - 3.41 (m, 1 H), 3.84 - 3.91 (m, 1 H), 4.53 - 4.58 (m, 2 H), 7.32 (d, J=5 Hz, 1 H), 7.37 (s, 1 H), 7.58 (d, J=5 Hz, 1 H), 7.85 (br d, J=8 Hz, 1 H) | 0.64 | 402 | A |
| 63 | N-(1-cyclopropyl-5,5-difluoropiperidin-3-yl)-2-(8-isopropyl-5-oxothieno[3',2':4,5]pyrrolo[1,2 -d][1,2,4]triazin-6(5H)-yl)acetamide 2,2,2-trifluoroacetate | 1H NMR (400 MHz, DMSO-d6) δ ppm 0.25 - 0.36 (m, 2 H), 0.39 - 0.48 (m, 2 H), 1.35 (d, J=7 Hz, 6 H), 1.73 - 1.89 (m, 2 H), 2.14 - 2.27 (m, 2 H), 2.52 - 2.68 (m, 1 H), 2.89 - 3.03 (m, 2 H), 3.33 - 3.42 (m, 1 H), 3.89 (br s, 1 H), 4.56 (s, 2 H), 7.32 (d, J=6 Hz, 1 H), 7.38 (s, 1 H), 7.59 (d, J=6 Hz, 1 H), 7.93 (d, J=8 Hz, 1 H) | 1.26 | 450 | A |
| 64 | 2-(8-isopropyl-5-oxothieno[3',2':4,5]pyrrolo[1,2 -d][1,2,4]triazin-6(5H)-yl)-N-((3R)-1-(1-methoxypropan-2-yl)piperidin-3-yl)acetamide | 1H NMR (400 MHz, DMSO-d6) δ ppm 0.90 (d, J=7 Hz, 3 H), 1.22 (m, 1 H), 1.36 (d, J=7 Hz, 6 H), 1.42 (m, 1 H), 1.58 - 1.72 (m, 2 H), 2.05 - 2.29 (m, 2 H), 2.61 (m, 1 H), 2.69 - 2.79 (m, 2 H), 3.13 - 3.22 (m, 4 H), 3.34 - 3.41 (m, 2 H), 3.69 (m, 1 H), 4.48 - 4.62 (m, 2 H), 7.33 (d, J=5 Hz, 1 H), 7.38 (s, 1 H), 7.59 (d, J=6 Hz, 1 H), 7.78 (dd, J=8, 3 Hz, 1 H) | 1.07 | 446 | B |
| 65 | 2-(8-isopropyl-5-oxothieno[3',2':4,5]pyrrolo[1,2 -d][1,2,4]triazin-6(5H)-yl)-N-(2-methoxypyridin-4-yl)acetamide | 1H NMR (400 MHz, DMSO-d6) δ ppm 1.36 (d, J=7 Hz, 6 H), 3.33 - 3.41 (m, 1 H), 3.82 (s, 3 H), 4.81 (s, 2 H), 7.05 - 7.13 (m, 2 H), 7.33 (d, J=6 Hz, 1 H), 7.40 (s, 1 H), 7.60 (d, J=5 Hz, 1 H), 8.05 (d, J=6 Hz, 1 H), 10.57 (s, 1 H) | 1.07 | 398 | A |
| 66 | 2-(12-isopropyl-9-oxo-3-thia-1,10,11-triazatricyclo [6.4.0.02,6]dodeca-2(6),4,7,11-tetraen-10-yl)-N-pyrimidin-4-yl-acetamide | 1H NMR (500 MHz, DMSO-d6) δ ppm 1.36 (d, J=7 Hz, 6 H), 3.35 - 3.43 (m, 1 H), 4.90 (s, 2 H), 7.33 (d, J=6 Hz, 1 H), 7.40 (s, 1 H), 7.60 (d, J=5 Hz, 1 H), 7.99 (dd, J=6, 1 Hz, 1 H), 8.66 (d, J=6 Hz, 1 H), 8.91 (d, J=1 Hz, 1 H), 11.22 (br s, 1 H) | 1.03 | 369 | A |
| 67 | 2-(12-isopropyl-9-oxo-3-thia-1,10,11-triazatricyclo [6.4.0.02,6]dodeca-2(6),4,7,11-tetraen-10-yl)-N-(4-pyridyl)acetamide | 1H NMR (500 MHz, DMSO-d6) δ ppm 1.36 (d, J=7 Hz, 2 H), 3.35 - 3.44 (m, 1 H), 4.84 (s, 2 H), 7.33 (d, J=5 Hz, 1 H), 7.41 (s, 1 H), 7.57 - 7.61 (m, 3 H), 8.46 (d, J=6 Hz, 2 H), 10.66 (s, 1 H) | 0.67 | 368 | A |
| 68 | 2-(12-isopropyl-9-oxo-3-thia-1,10,11-triazatricyclo [6.4.0.02,6]dodeca-2(6),4,7,11-tetraen-10-yl)-N-(6-methyl-3-pyridyl)acetamide | 1H NMR (500 MHz, DMSO-d6) δ ppm 1.36 (d, J=7 Hz, 6 H), 2.45 (s, 3 H), 3.37 - 3.42 (m, 1 H), 4.81 (s, 2 H), 7.30 - 7.35 (m, 2 H), 7.40 (s, 1 H), 7.60 (d, J=5 Hz, 1 H), 7.95 (dd, J=8, 2 Hz, 1 H), 8.66 - 8.68 (m, 1 H), 10.38 (br s, 1 H) | 0.72 | 382 | A |
| 70 | N-(5-fluoropyrimidin-4-yl)-2-(12-isopropyl-9-oxo-3-thia-1,10,11-triazatricyclo [6.4.0.02,6]dodeca-2(6),4,7,11-tetraen-10-yl)acetamide | 1H NMR (400 MHz, DMSO-d6) δ ppm 1.37 (d, J=7 Hz, 6 H), 3.45 - 3.61 (m, 1 H), 4.99 (s, 2 H), 7.33 (d, J=5 Hz, 1 H), 7.42 (s, 1 H), 7.60 (d, J=5 Hz, 1 H), 8.82 (dd, J=7, 3 Hz, 2 H), 11.08 (s, 1 H) | 1.02 | 387 | A |
| 71 | 2-(12-isopropyl-9-oxo-3-thia-1,10,11-triazatricyclo[6.4.0.02,6]dodec a-2(6),4,7,11-tetraen-10-yl)-N-[(3R)-1-(1-methylcyclopentyl)-3-piperidyl]acetamide | 1H NMR (400 MHz, DMSO-d6) δ ppm 0.85 (s, 3 H), 1.21 - 1.34 (m, 3 H), 1.37 (dd, J=7, 1 Hz, 6 H), 1.39 - 1.55 (m, 7 H), 1.55 - 1.69 (m, 2 H), 2.13 (t, J=9 Hz, 1 H), 2.26 (t, J=10 Hz, 1 H), 2.58 (m, 1 H), 2.72(m, 1 H), 3.45 (m hidden, 1 H), 3.75 (m, 1 H), 4.46 - 4.66 (m, 2 H), 7.33 (d, J=6 Hz, 1 H), 7.40 (s, 1 H), 7.60 (d, J=6 Hz, 1 H), 7.62 (br d, J=8 Hz, 1 H) | 0.73 | 456 | A |
| 74 | N-[3-(acetamidomethyl) bicyclo[1.1.1]pentan-1-yl]-2-(8-isopropyl-5-oxothieno [3',2':4,5] pyrrolo[1,2-d][1,2,4]triazin-6(5H)-yl)acetamide | 1H NMR (400 MHz, DMSO-d6) δ ppm 1.35 (d, J=7 Hz, 6 H), 1.80 (s, 3 H), 1.84 (s, 6 H), 3.11 - 3.26 (m, 2 H), 3.35 (m, 1 H), 4.48 (s, 2 H), 7.32 (d, J=5 Hz, 1 H), 7.36 (s, 1 H), 7.58 (d, J=6 Hz, 1 H), 7.72 (t, J=6 Hz, 1 H), 8.48 (s, 1 H) | 0.98 | 428 | A |
| 75 | N-[(3R)-1-(cyanomethyl)piperidin-3-yl]-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.13 - 1.30 (m, 1 H), 1.35 (d, J=7 Hz, 6 H), 1.50 (m, 1 H), 1.65 - 1.79 (m, 2 H), 2.04 (br t, J=10 Hz, 1 H), 2.14 (t, J=11 Hz, 1 H), 2.62 (m, 1 H), 2.75 (m, 1 H), 3.38 (m, 1 H), 3.73 (d, J=4 Hz, 2 H), 3.81 (m, 1 H), 4.55 (d, J=2 Hz, 2 H), 7.32 (d, J=6 Hz, 1 H), 7.37 (s, 1 H), 7.58 (d, J=5 Hz, 1 H), 7.94 (d, J=8 Hz, 1 H) | 1.06 | 413 | A |
| 76 | N-[(3R)-1-methylpyrrolidin-3-yl]-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.35 (d, J=7 Hz, 6 H), 1.69 (m, 1 H), 2.03 - 2.30 (m, 1 H), 2.43 (s, 3 H), 2.56 - 2.65 (m, 2 H), 2.82 - 2.91 (m, 2 H), 3.26 - 3.31 (m hidden, 1 H), 4.25 (m, 1 H), 4.55 (d, J=3 Hz, 2 H), 7.32 (d, J=6 Hz, 1 H), 7.37 (s, 1 H), 7.58 (d, J=6 Hz, 1 H), 8.29 (br d, J=7 Hz, 1 H) | 0.60 | 374 | A |
| 77 | N-[(3R)-1-(2-cyanoethyl)piperidin-3-yl]-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.23 (m, 1 H), 1.35 (d, J=7 Hz, 6 H), 1.49 (m, 1 H), 1.59 - 1.79 (m, 2 H), 1.82 - 2.09 (m, 2 H), 2.31 - 2.90 (m partially hidden, 6 H), 3.35 - 3.49 (m partially hidden, 1 H), 3.76 (br s, 1 H), 4.48 - 4.61 (m, 2 H), 7.32 (d, J=6 Hz, 1 H), 7.37 (s, 1 H), 7.58 (d, J=6 Hz, 1 H), 7.86 (br s, 1 H) | 0.64 | 427 | A |
| 78 | N-[(3R)-1-[(2R)-2-hydroxypropyl]piperidin-3-yl]-2-(8-isopropyl-5-oxothieno [3',2':4,5]pyrrolo[1,2-d][1,2,4]triazin-6(5H)-yl)acetamide | 1H NMR (400 MHz, DMSO-d6) δ ppm 1.01 (d, J=6 Hz, 3 H), 1.28 (m, 1 H), 1.35 (d, J=7 Hz, 6 H), 1.57 (m, 1 H), 1.63 - 1.75 (m, 2 H), 2.08 - 3.01 (m partially hidden, 7 H), 3.40 (m, 1 H), 3.74 - 3.92 (m, 2 H), 4.46 - 4.65 (m, 2 H), 7.32 (d, J=5 Hz, 1 H), 7.37 (s, 1 H), 7.59 (d, J=6 Hz, 1 H), 7.96 (br d, J=8 Hz, 1 H) | 0.60 | 432 | A |
| 79 | N-[(3R)-1-(1-hydroxy-2-methylpropan-2-yl)piperidin-3-yl]-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 0.92 (s, 6 H), 1.24 (m, 1 H), 1.35 (d, J=7 Hz, 6 H), 1.45 (m, 1 H), 1.65 (d, J=9 Hz, 2 H), 2.17 (br dd, J=11, 9 Hz, 1 H), 2.26 - 2.34 (m, 1 H), 2.71 (m, 1 H), 2.85 (m, 1 H), 3.31 - 3.42 (m partially hidden, 3 H), 3.71 - 3.78 (m, 2 H), 4.47 - 4.63 (m, 2 H), 7.32 (d, J=6 Hz, 1 H), 7.37 (s, 1 H), 7.58 (d, J=6 Hz, 1 H), 7.83 (d, J=8 Hz, 1 H) | 2.55 | 446 | C |
| 80 | ethyl-2-methyl-2-[3-[[2-(8-isopropyl-5-oxothieno[3',2':4,5]pyrrolo[1,2 -d][1,2,4]triazin-6(5H)-yl)acetyl]amino] piperidin-1-yl]propanoate | 1H NMR (400 MHz, DMSO-d6) δ ppm 1.13 - 1.20 (m, 9 H), 1.25 (m, 1 H), 1.35 (d, J=7 Hz, 6 H), 1.43 (m, 1 H), 1.55 - 1.76 (m, 2 H), 2.02 (t, J=10 Hz, 1 H), 2.15 (t, J=10 Hz, 1 H), 2.67 (m, 1 H), 2.79(m, 1 H), 3.36 (spt, J=6 Hz, 1 H), 3.67 (m, 1 H), 4.03 (q, J=7 Hz, 2 H), 4.48 - 4.61 (m, 2 H), 7.32 (d, J=5 Hz, 1 H), 7.37 (s, 1 H), 7.58 (d, J=5 Hz, 1 H), 7.74 (br d, J=8 Hz, 1 H) | 0.84 | 488 | A |
| 82 | N-[(1R,4R)-2-methyl-2-azabicyclo[2.2.1]heptan-6-yl]-2-(8-isopropyl-5-oxothieno[3',2':4,5] pyrrolo[1,2-d][1,2,4]triazin-6(5H)-yl)acetamide | 1H NMR (400 MHz, DMSO-d6) δ ppm 0.81 (m, 1 H), 1.27 (d, J=10 Hz, 1 H), 1.36 (d, J=7 Hz, 6 H), 1.57 (m, 1 H), 1.88 (m, 1 H), 2.19 (m, 1 H), 2.22 (s, 3 H), 2.30 - 2.37 (m, 2 H), 2.69 (d, J=9 Hz, 1 H), 2.89 (br s, 1 H), 3.94 (m, 1 H), 4.53 - 4.68 (m, 2 H), 7.32 (d, J=6 Hz, 1 H), 7.38 (s, 1 H), 7.58 (d, J=5 Hz, 1 H), 7.72 (br d, J=8 Hz, 1 H) | 0.62 | 400 | A |
| 83 | N-(2-methyl-2-azaspiro[3.3]heptan-6-yl)-2-(8-isopropyl-5-oxothieno[3',2':4,5]pyrrolo[1,2 -d][1,2,4]triazin-6(5H)-yl)acetamide | 1H NMR (400 MHz, DMSO-d6) δ ppm 1.34 (d, J=7 Hz, 6 H), 1.96 - 2.07 (m, 2 H), 2.17 - 2.30 (m, 3 H), 2.31 - 2.38 (m, 2 H), 3.13 - 3.22 (m, 2 H), 3.28 (br s, 2 H), 3.37(m, 1 H), 4.07 (m, 1 H), 4.49 (s, 2 H), 7.32 (d, J=6 Hz, 1 H), 7.36 (s, 1 H), 7.58 (d, J=5 Hz, 1 H), 8.14 (d, J=8 Hz, 1 H) | 0.63 | 400 | A |
| 84 | 2-(8-isopropyl-5-oxothieno[3',2':4,5]pyrrolo[1,2 -d][1,2,4]triazin-6(5H)-yl)-N-pyrimidin-5-ylacetamide | 1H NMR (500 MHz, DMSO-d6) δ ppm 1.37 (d, J=7 Hz, 6 H), 3.40 (spt, J=7 Hz, 1 H), 4.85 (s, 2 H), 7.33 (d, J=5 Hz, 1 H), 7.41 (s, 1 H), 7.60 (d, J=5 Hz, 1 H), 8.90 (s, 1 H), 8.99 (s, 2 H), 10.63 (br s, 1 H) | 0.97 | 369 | A |
| 85 | 2-(12-isopropyl-9-oxo-3-thia-1,10,11-triazatricyclo[6.4.0.02,6]dodec a-2(6),4,7,11-tetraen-10-yl)-N-(2-oxo-4-piperidyl) acetamide | 1H NMR (400 MHz, DMSO-d6) δ ppm 1.35 (dd, J=7, 2 Hz, 6 H), 1.70 - 1.88 (m, 2 H), 2.03 - 2.27 (m, 2 H), 3.02 - 3.13 (m, 1 H), 3.24 - 3.32 (m, 1 H), 3.55 - 3.61 (m, 1 H), 3.89 - 4.05 (m, 1 H), 4.58 (s, 2 H), 7.32 (dd, J=5, 1 Hz, 1 H), 7.37 (d, J=3 Hz, 1 H), 7.58 (d, J=5 Hz, 1 H), 8.06 (br t, J=6 Hz, 1 H), 8.18 (d, J=7 Hz, 1 H) | 2.86-2.91 | 388 | C |
| 86 | N-(1-cyclopropylpyrrolidin-3-yl)-2-(12-isopropyl-9-oxo-3-thia-1,10,11-triazatricyclo[6.4.0.02,6]dodec a-2(6),4,7,11-tetraen-10-yl)acetamide | 1H NMR (400 MHz, DMSO-d6) δ ppm 0.59 - 1.06 (m, 4 H), 1.35 (d, J=7 Hz, 6 H), 1.72 - 2.27 (m, 2 H), 2.75 - 3.76 (m, 5 H), 4.25 - 4.48 (m, 1 H), 4.59 (s, 2 H), 7.32 (d, J=5 Hz, 1 H), 7.38 (s, 1 H), 7.59 (d, J=5 Hz, 1 H), 8.39 (br s, 1 H), 9.65 (br s, 1 H) | 0.63 | 400 | A |
| 87 | 2-(12-isopropyl-9-oxo-3-thia-1,10,11-triazatricyclo[6.4.0.02,6]dodec a-2(6),4,7,11-tetraen-10-yl)-N-[rac-(3R)-1-tert-butylpyrrolidin-3-yl]acetamide | 1H NMR (400 MHz, DMSO-d6) δ ppm 1.09 (s, 9 H), 1.35 (d, J=7 Hz, 6 H), 1.57 - 1.73 (m, 1 H), 1.98 - 2.14 (m, 1 H), 2.52 - 2.62 (m, 1 H), 2.71 - 2.83 (m, 1 H), 2.84 - 2.95 (m, 1 H), 3.04 (br dd, J=10, 7 Hz, 1 H), 3.30 - 3.43 (m, 1 H), 4.15 - 4.28 (m, 1 H), 4.50 - 4.60 (m, 2 H), 7.32 (d, J=5 Hz, 1 H), 7.37 (s, 1 H), 7.58 (d, J=6 Hz, 1 H), 8.24 (br d, J=7 Hz, 1 H) | 0.66 | 416 | A |

### Example 5 ; (R)-2-(2-chloro-8-isopropyl-5-oxothieno[3',2':4,5]pyrrolo[1,2-d][1,2,4]triazin-6(5H)-yl)-N-(1-methylpiperidin-3-yl)acetamide 2,2,2-trifluoroacetate

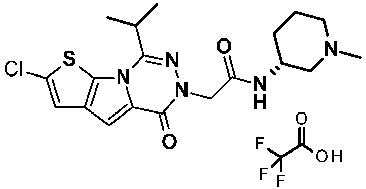

### Int 5: 2-chloro-6H-thieno[2,3-b]pyrrole-5-carbohydrazide

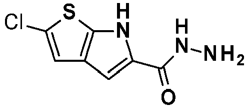

To a solution of methyl 2-chloro-6H-thieno[2,3-b]pyrrole-5-carboxylate (1.5 g, 6.96 mmol) in 10 ml of MeOH, hydrazine (1.31 ml, 20.87 mmol) is added, and this mixture is heated at reflux overnight. After cooling down the solution, the precipitate formed is filtered off, washed with ether and dried under vacuum. The title compound is obtained as a beige solid (1.39 g, 92% yield).

LCMS (method F): Rt = 1.14 min; MS m/z [M+H]⁺ 216/218

### Int 6: 2-chloro-8-isopropylthieno[3',2':4,5]pyrrolo[1,2-d][1,2,4]triazin-5(6H)-one

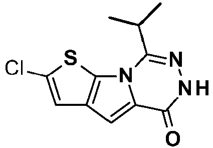

Under nitrogen, to a solution of 2-chloro-6H-thieno[2,3-b]pyrrole-5-carbohydrazide (1.39 g, 6.45 mmol) in 20 ml of DMF is added methyl 2-methylpropanimidoate hydrochloride (1.10 g, 7.73 mmol). This mixture is stirred at rt for 30 min. Then solid tBuOK (2.15g, 18.82 mmol) is added and the resulting mixture is stirred in a preheated oil bath at 90°C for 24 h. After cooling down to 0°C, the solution is acidified to pH 5 with a saturated NH₄Cl aqueous solution. The precipitate formed is filtered off and purified by flash chromatography on silica gel using Cyclohexane/EtOAc (from 10% to 50%) to give the title compound (750 mg, 43% yield) as a brown solid.

LCMS (method F): Rt = 1.82 min; MS m/z [M+H]⁺ 268/270

### Int 7: ethyl 2-(2-chloro-8-isopropyl-5-oxothieno[3',2':4,5]pyrrolo[1,2-d][1,2,4]triazin-6(5H)-yl)acetate

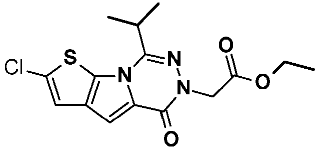

Under nitrogen, to a solution of, 2-chloro-8-isopropylthieno[3',2':4,5]pyrrolo[1,2-d][1,2,4]triazin-5(6H)-one (750 mg, 2.80 mmol) in 20ml of DMF is added K₂CO₃ (581 mg, 4.20 mmol), followed by a dropwise addition of ethyl iodoacetate (508 µl, 4.20 mmol). The mixture is stirred at rt for 12h.

The reaction mixture is diluted with water then extracted twice with EtOAc. The combined organic layers are washed with water, then with brine, dried over Na₂SO₄, filtered off and concentrated under reduced pressure. The residue is purified by Flash chromatography on silica gel using Cyclohexane/EtOAc (from 10% to 30%) to give the title compound as a white solid (800 mg, 80% yield).

LCMS (method F): Rt = 2.18 min; MS m/z [M+H]⁺ 354/356

### Int 8: 2-(2-chloro-8-isopropyl-5-oxothieno[3',2':4,5]pyrrolo[1,2-d][1,2,4]triazin-6(5H)-yl)acetic acid

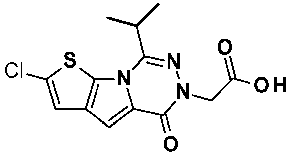

To a solution of ethyl 2-(2-chloro-8-isopropyl-5-oxothieno[3',2':4,5]pyrrolo[1,2-d][1,2,4]triazin-6(5H)-yl)acetate (808 mg, 2.28 mmol) in 20ml of dioxane is added a solution of NaOH (1M) (6.85 ml, 6.85 mmol). The resulting solution is stirred at rt overnight.

The mixture is acidified with HCl (1M). The precipitate formed is filtered off, washed with water and pentane. After drying overnight under vacuum in presence of P₂O₅, the title compound is obtained as a white solid (613 mg, 82% yield).

LCMS (method F): Rt = 1.84 min; MS m/z [M+H]⁺ 326/328

### Ex 5: (R)-2-(2-chloro-8-isopropyl-5-oxothieno[3',2':4,5]pyrrolo[1,2-d][1,2,4]triazin-6(5H)-yl)-N-(1-methylpiperidin-3-yl)acetamide 2,2,2-trifluoroacetate

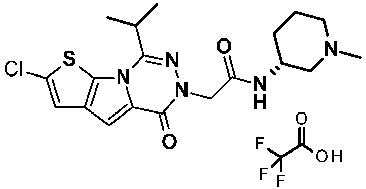

Under nitrogen, to a suspension of (3R)-1-methylpiperidin-3-amine dihydrochloride (73 mg, 368 µmol) and 2-(2-chloro-8-isopropyl-5-oxothieno[3',2':4,5]pyrrolo[1,2-d][1,2,4]triazin-6(5H)-yl)acetic acid (100 mg, 307 µmol) in 2 ml of DMF is added DIPEA (268 µl, 1.53 mmol) followed by HATU (175 mg, 460 µmol). The resulting mixture is stirred at rt for 12h. Some drops of TFA are added and the resulting solution is purified by reverse layer chromatography Waters SunFire PrepC18 OBD 30 x 100 mm column, water containing 0.1% TFA with a gradient of acetonitrile 20 to 80% in 18 min.

After evaporation under reduced pressure and drying overnight, the title compound is obtained as a solid (116 mg, 71% yield).

1H NMR (400 MHz, DMSO-d6) δ ppm 1.24 - 1.50 (m, 7 H), 1.67 (m, 1 H), 1.79 - 1.95 (m, 2 H), 2.64 (m, 1 H), 2.73 - 2.89 (m, 1 H), 3.22 (sept, J=6.6 Hz, 1H), 3.32 - 3.46 (m, 4 H), 3.94 (m, 1 H), 4.57 (s, 2 H), 7.34 (s, 1 H), 7.50 (s, 1 H), 8.26 (d, J=7.5 Hz, 1 H), 9.54 (br s, 1 H) LCMS [M+H]⁺ = 422; Rt =0.76; Method A

The following examples were synthesized analogous to the previous procedure, using the appropriate amines:

| Ex N° | Name | NMR | LCMS Rt(min) | LCMS [M+H]+ | LCMS Analytical method |
|---|---|---|---|---|---|
| 6 | (R)-2-(2-chloro-8-isopropyl-5-oxothieno[3',2':4,5]pyrrolo[1,2-d][1,2,4]triazin-6(5H)-yl)-N-(1-cyclopropylpiperidin-3-yl)acetamide 2,2,2-trifluoroacetate | 1H NMR (400 MHz, DMSO-d6) δ ppm 0.76 - 1.01 (m, 4 H), 1.34 (d, J=6.7, 6 H), 1.45 (m, 1 H), 1.65 (m, 1 H), 1.75 - 1.99 (m, 2 H), 2.89 (m, 2 H), 3.05 (m, 1 H), 3.23 (sept, *J*=6.7, 1 H), 3.46 (m, 2 H), 3.92 (m, 1 H), 4.58 (s, 2 H), 7.35 (s, 1 H), 7.50 (s, 1 H), 8.30 (br d, J=7.3 Hz, 1 H), 9.02 (br s, 1 H) | 0.80 | 448 | A |
| 7 | 2-(2-chloro-8-isopropyl-5-oxothieno[3',2':4,5]pyrrolo[1,2-d][1,2,4]triazin-6(5H)-yl)-N-(thiazol-5-yl)acetamide | 1H NMR (400 MHz, DMSO-d6) δ ppm 1.34 (d, J=6.5 Hz, 6 H), 3.24 (sept, J=6,5 Hz, 1 H), 4.85 (s, 2 H), 7.38 (s, 1 H), 7,51 (s, 1 H), 7.64 (d, J=0.8 Hz, 1 H), 8.60 (br s, 1 H), 11.55 (s, 1 H) | 1.27 | 408 | A |
| 10 | (R)-2-(2-chloro-8-isopropyl-5-oxothieno[3',2':4,5]pyrrolo[1,2-d][1,2,4]triazin-6(5H)-yl)-N-(1-cyclobutylpiperidin-3-yl)acetamide formate | 1H NMR (500 MHz, DMSO-d6) δ ppm 1.17 - 1.26 (m, 1 H), 1.33 (d, J=7 Hz, 6 H), 1.38 - 1.46 (m, 1 H), 1.54 - 1.77 (m, 8 H), 1.92 (br d, J=6 Hz, 2 H), 2.60 - 2.68 (m, 3 H), 3.19 - 3.25 (m, 1 H), 3.67 - 3.74 (m, 1 H), 4.53 - 4.58 (m, 2 H), 7.34 (s, 1 H), 7.49 (s, 1 H), 7.82 (br d, J=8 Hz, 1 H) | 0.83 | 462 | A |
| 34 | 2-(2-chloro-8-isopropyl-5-oxothieno[3',2':4,5]pyrrolo[1,2-d][1,2,4]triazin-6(5H)-yl)-N-(5,5-difluoro-1-methylpiperidin-3-yl)acetamide 2,2,2-trifluoroacetate | 1H NMR (400 MHz, DMSO-d6) δ ppm 1.34 (d, J=7 Hz, 6 H), 1.81 - 2.08 (m, 2 H), 2.28 - 2.43 (m, 4 H), 2.59 - 2.80 (m, 3 H), 3.19 - 3.33 (m, 1 H), 4.12 (br s, 1 H), 4.59 (s, 2 H), 7.35 (s, 1 H), 7.50 (s, 1 H), 8.32 (br d, J=3 Hz, 1 H) | 0.89 | 458 | A |

### Example 8 ; (R)-2-(2-bromo-8-isopropyl-5-oxothieno[3',2':4,5]pyrrolo[1,2-d][1,2,4]triazin-6(5H)-yl)-N-(1-methylpiperidin-3-yl)acetamide

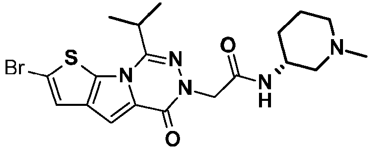

### Int 9: 2-(2-bromo-8-isopropyl-5-oxothieno[3',2':4,5]pyrrolo[1,2-d][1,2,4]triazin-6(5H)-yl)acetic acid

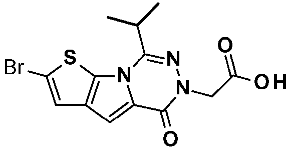

Under nitrogen, to a solution of ethyl 2-(8-isopropyl-5-oxothieno[3',2':4,5]pyrrolo[1,2-d][1,2,4]triazin-6(5H)-yl)acetate (260 mg, 814 µmol) in 10 ml of CHCl₃ is added 1 ml of acetic acid and NBS (159 mg, 896 µmol). The mixture is stirred at rt overnight. A solution of Na₂S₂O₃ (1M) is added and the mixture is extracted with DCM. The organic layer is dried over Na₂SO₄, filtered and concentrated under reduced pressure.

The residue obtained is dissolved in 10 ml of THF and 1 ml of MeOH, and 0.5 ml of a solution of NaOH (1N) is added. The solution is stirred at rt 1h, then the mixture is acidified with a solution of HCI (1N) followed by extraction with EtOAc, dried over Na₂SO₄ then filtered off and concentrated. The corresponding acid is obtained as a white solid (270 mg, 89% yield).

LCMS (method E): Rt = 1.43 min; MS m/z [M+H]⁺ 370/372

### Ex 8: (R)-2-(2-bromo-8-isopropyl-5-oxothieno[3',2':4,5]pyrrolo[1,2-d][1,2,4]triazin-6(5H)-yl)-N-(1-methylpiperidin-3-yl)acetamide

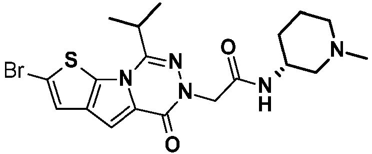

Under nitrogen, to a suspension of (3*R*)-1-methylpiperidin-3-amine dihydrochloride (74 mg, 375 µmol) and 2-(2-bromo-8-isopropyl-5-oxothieno[3',2':4,5]pyrrolo[1,2-d][1,2,4]triazin-6(5H)-yl)acetic acid (126 mg, 340 µmol) in 2 ml of DMF is added DIPEA (180 µl, 1,03 mmol) followed by HATU (168 mg, 442 µmol). The mixture is stirred at rt for 12h. The crude mixture is purified by reverse layer chromatography Waters SunFire PrepC18 OBD 30 x 100 mm column, water containing 0.1% HCO₂H with a gradient of acetonitrile 10 to 80% in 18 min. After evaporation under reduced pressure and drying overnight, the title compound is obtained as a solid (51 mg, 32% yield).

1H NMR (500 MHz, DMSO-d6) δ ppm 1.33 (d, J=6.8 Hz, 6 H), 1.39 (m, 1 H), 1.66 (m, 1 H), 1.85 (m, 1 H), 1.91 (m, 1 H), 2.63 (m, 1 H), 2.78 - 2.89 (m, 4 H), 3.23 (sept, J=6.8 Hz, 1 H), 3.41 (m, 2 H), 3.94 (m, 1 H), 4.57 (s, 2 H), 7.33 (s, 1 H), 7.58 (s, 1 H), 8.25 (d, J=7.6 Hz, 1 H), 9.45 (br s, 1 H)

LCMS [M+H]⁺ = 466/468; Rt =0.76; Method A

The following examples were synthesized analogous to the previous procedure, using the appropriate amines

| Ex N° | Name | NMR | LCMS Rt (min) | LCMS [M+H]+ | LCMS Analytical method |
|---|---|---|---|---|---|
| 31 | (R)-2-(2-bromo-8-isopropyl-5-oxothieno[3',2':4,5]pyrrolo[1,2-d][1,2,4]triazin-6(5H)-yl)-N-(1-cyclobutylpiperidin-3-yl) acetamide 2,2,2-trifluoroacetate | 1H NMR (400 MHz, DMSO-d6) δ ppm 1.33 (d, J=7 Hz, 6 H), 1.43 (br dd, J=13, 4 Hz, 1 H), 1.59 - 1.81 (m, 3 H), 1.83 - 1.98 (m, 2 H), 2.08 - 2.22 (m, 4 H), 2.39 - 2.46 (m, 1 H), 2.57 - 2.70 (m, 1 H), 3.19 - 3.34 (m, 3 H), 3.68 (q, J=8 Hz, 1 H), 3.84 - 4.01 (m, 1 H), 4.52 - 4.65 (m, 2 H), 7.34 (s, 1 H), 7.59 (s, 1 H), 8.28 (d, J=8 Hz, 1 H), 9.49 (br d, J=7 Hz, 1 H) | 2.86 | 506 | A |
| 59 | 2-(4-bromo-12-isopropyl-9-oxo-3-thia-1,10,11-triazatricyclo[6.4.0.02,6]dodeca-2(6),4,7,11-tetraen-10-yl)-N-(1-tert-butyl-3-piperidyl)acetamide | 1H NMR (400 MHz, DMSO-d6) δ ppm 1.06 (s, 9 H), 1.20 - 1.38 (m, 7 H), 1.43 - 1.54 (m, 1 H), 1.67 - 1.77 (m, 2 H), 2.15 (br t, J=10 Hz, 1 H), 2.24 - 2.38 (m, 1 H), 2.88 - 3.02 (m, 2 H), 3.22 (spt, J=7 Hz, 1 H), 3.71 - 3.88 (m, 1 H), 4.50 - 4.61 (m, 2 H), 7.33 (s, 1 H), 7.58 (s, 1 H), 7.90 (br d, J=8 Hz, 1 H) | 0.80 | 508 | A |

### Example 9 ; (R)-2-(8-isopropyl-2-methyl-5-oxothieno[3',2':4,5]pyrrolo[1,2-d][1,2,4]triazin-6(5H)-yl)-N-(1-methylpiperidin-3-yl)acetamide 2,2,2-trifluoroacetate

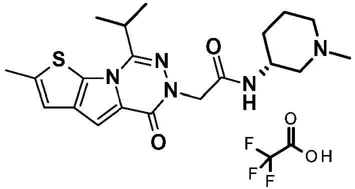

### Int 10: 2-methyl-6H-thieno[2,3-b]pyrrole-5-carbohydrazide

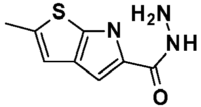

Similarly to 6H-thieno[2,3-b]pyrrole-5-carbohydrazide synthesis, starting from Ethyl 2-methyl-6H-thieno[2,3-b]pyrrole-5-carboxylate (300 mg, 1.43 mmol) and hydrazine (0.270 ml, 4.3 mmol), 150 mg (53% yield) of the title compound is obtained as a solid.

LCMS (method E): Rt = 0.96 min; MS m/z [M+H]⁺ 196

### Int 11: 8-isopropyl-2-methylthieno[3',2':4,5]pyrrolo[1,2-d][1,2,4]triazin-5(6H)-one

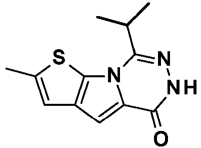

Under nitrogen, to a solution of 2-methyl-6H-thieno[2,3-b]pyrrole-5-carbohydrazide (100 mg, 512 µmol) in 5 ml of DMF is added methyl 2-methylpropanimidoate hydrochloride (87 mg, 615 µmol).

This mixture is stirred at rt for 30 min. Then solid tBuOK (115 mg, 1,02 mmol) is added and the resulting mixture is stirred in a preheated oil bath at 100°C for 1 h. After cooling to 0°C, the mixture is acidified with (1N) HCI to pH 5. The precipitate formed is filtered off, washed with water and dried under vacuum to give the title compound as a violet solid. (110 mg, 86% yield). LCMS (method E): Rt = 1.33 min; MS m/z [M+H]⁺ 248

### Int 12: ethyl 2-(8-isopropyl-2-methyl-5-oxothieno[3',2':4,5]pyrrolo[1,2-d][1,2,4]triazin-6(5H)-yl)acetate

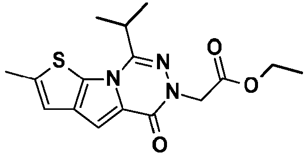

Under nitrogen, to a solution of 8-isopropyl-2-methylthieno[3',2':4,5]pyrrolo[1,2-d][1,2,4]triazin-5(6H)-one (110 mg, 445 µmol) in 5 ml of DMF is added K₂CO₃ (92 mg, 667 µmol), followed by a dropwise addition of ethyl iodoacetate (81 µl, 667 µmol). The resulting mixture is stirred at rt for 12h.

The reaction mixture is diluted with a saturated aqueous solution of NH₄CI, then extracted twice with EtOAc. The combined organic layers are washed with water, then with brine, dried over Na₂SO₄, filtered and concentrated under reduced pressure. The residue is purified by Flash chromatography on silica gel using Cyclohexane/EtOAc (from 10% to 100%) to give the title compound as a white solid (71 mg, 48% yield)

LCMS (method E): Rt = 1.57 min; MS m/z [M+H]⁺ 334

### Ex 9: (R)-2-(8-isopropyl-2-methyl-5-oxothieno[3',2':4,5]pyrrolo[1,2-d][1,2,4]triazin-6(5H)-yl)-N-(1-methylpiperidin-3-yl)acetamide 2,2,2-trifluoroacetate

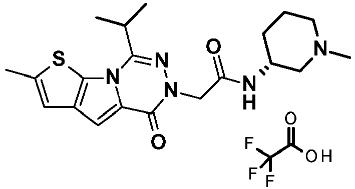

To a solution of ethyl 2-(8-isopropyl-2-methyl-5-oxothieno[3',2':4,5]pyrrolo[1,2-d][1,2,4]triazin-6(5H)-yl)acetate (71 mg, 213 µmol) in 2 ml of dioxane and 5 ml H2O is added solid NaOH (26 mg, 639 µmol). The solution is stirred at rt overnight.

The mixture is acidified with HCI (1N), then extracted with EtOAc. The organic layer is dried over Na₂SO₄, filtered and concentrated under vacuum. Under nitrogen, the residue is solubilized in 5 ml DMF, then (3*R*)-1-methylpiperidin-3-amine dihydrochloride (50 mg, 256 µmol), DIPEA (149 µl, 852 µmol) and HATU (1221 mg, 319 µmol) are added to the solution. The mixture is stirred at rt for 12h. The resulting solution is purified by reverse layer chromatography Waters SunFire PrepC18 OBD 30 x 100 mm column, water containing 0.1% TFA with a gradient of acetonitrile 20 to 80% in 18 min.

After evaporation under reduced pressure and drying overnight, the title compound is obtained as a yellow solid (35 mg, 32% yield).

1H NMR (400 MHz, DMSO-d6) δ ppm 1.34 (d, J=7 Hz, 7 H), 1.66 (br d, J=14 Hz, 1 H), 1.80 - 1.94 (m, 2 H), 2.58 (d, J=1 Hz, 3 H), 2.63 (br d, J=10 Hz, 1 H), 2.77 - 2.85 (m, 4 H), 3.26 - 3.36 (m, 3 H), 3.89 - 3.99 (m, 1 H), 4.56 (s, 2 H), 7.03 (d, J=1 Hz, 1 H), 7.26 (s, 1 H), 8.24 (d, J=8 Hz, 1 H)

LCMS [M+H]⁺ = 402; Rt =0.71; Method A

### Example 24 ; (R)-2-(8-cyclopropyl-5-oxothieno[3',2':4,5]pyrrolo[1,2-d][1,2,4]triazin-6(5H)-yl)-N-(1-methylpiperidin-3-yl)acetamide

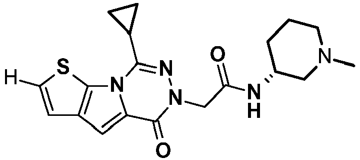

### Int 1: 6H-thieno[2,3-b]pyrrole-5-carbohydrazide

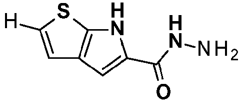

To a solution of methyl 6*H*-Thieno[2,3-*b*]pyrrole-5-carboxylate (4 g, 22.07 mmol) in 100 ml of EtOH is added hydrazine solution (35% in water) (24.7 ml, 176.59 mmol) and this mixture is heated at reflux overnight. After cooling down the solution, the precipitate formed is filtered off, washed with ether and dried under vacuum. The title compound is obtained as a white solid (3.4 g, 85% yield).

LCMS (method F): Rt = 1.15 min; MS m/z [M+H]⁺ 181.2

### Int 13: 8-cylopropylthieno[3',2':4,5]pyrrolo[1,2-d][1,2,4]triazin-5(6H)-one

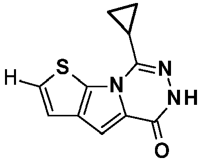

Under nitrogen, to a solution of 6H-thien[2,3-b]pyrrole-5-carbohydrazide (1g, 5.52 mmol) in 40 ml of DMF is added methyl cyclopropanecarboximidate hydrochloride (945 mg, 6.62 mmol) and the mixture is stirred at rt for 1 h.

Then, solid tBuOK (1.26 g, 11.04 mmol) is added and the resulting mixture is stirred in a preheated Dry-syn (90°C) for 1h. The mixture is cooled to RT, diluted with EtOAc and washed with 10% NaHCO₃ aq solution. The organic layer is dried over Na₂SO₄, filtered and concentrated under reduced pressure to provide the title product (490mg, 38% yield). LCMS (method F): Rt = 1.41 min; MS m/z [M+H]⁺ 232.2

### Int 14: Ethyl 2-(8-cyclopropyl-5-oxothieno[3',2':4,5]pyrrolo[1,2-d][1,2,4]triazin-6(5H)-yl)acetate

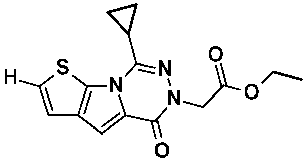

Under nitrogen, to a solution of 8-cylopropylthieno[3',2':4,5]pyrrolo[1,2-d][1,2,4]triazin-5(6H)-one (490 mg, 2.12 mmol) in 5 ml of DMF is added Cs₂CO₃ (828 mg, 2.54 mmol), followed by a dropwise addition of ethyl iodoacetate (557 µl, 4,61 mmol). The resulting mixture is stirred at rt for 20 h. Then, the mixture is diluted with water and extracted twice with EtOAc. The combined organic layers are washed with brine, dried over Na₂SO₄, filtered and concentrated under reduced pressure. The residue is purified by flash chromatography on silica gel using cyclohexane/EtOAc (from 5% to 40%) to give the title compound as a white solid (500 mg, 77% yield).

LCMS (method F): Rt = 1.79 min; MS m/z [M+H]⁺ 318.4

### Int 15: 2-(-8-cyclopropyl-5-oxothieno[3',2':4,5]pyrrolo[1,2-d][1,2,4]triazin-6(5H)-yl)acetic acid

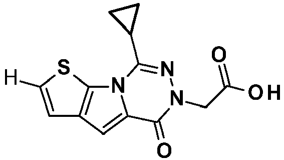

To a solution of ethyl 2-(8-cyclopropyl-5-oxothieno[3',2':4,5]pyrrolo[1,2-d][1,2,4]triazin-6(5H)-yl)acetate (650 mg, 2,14 mmol) in 15 ml of dioxane/MeOH (1/1) is added a solution of (1M) NaOH (6.43 ml 6.43 mmol). The resulting solution is stirred 20 h at rt. The mixture is acidified with HCI (1M) and extracted with DCM. The organic layer is washed with brine, dried over Na₂SO₄, filtered and concentrated under reduced pressure to provide the title compound as a white solid (500 mg, 80% yield).

LCMS (method F): Rt = 1.47 min; MS m/z [M+H]⁺ 290.0

### Ex 24: (R)-2-(8-cyclopropyl-5-oxothieno[3',2':4,5]pyrrolo[1,2-d][1,2,4]triazin-6(5H)-yl)-N-(1-methylpiperidin-3-yl)acetamide

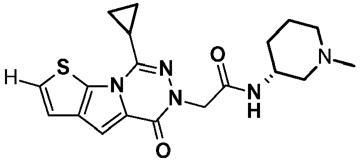

Under nitrogen, to a suspension of (*R*)-1-methylpiperidin-3-amine hydrochloride (38 mg 331 µmol) and 2-(8-cyclopropyl-5-oxothieno[3',2':4,5]pyrrolo[1,2-d][1,2,4]triazin-6(5H)-yl)acetic acid (80 mg, 277 µmol) in 5 ml of DMF is added DIPEA (241 µl,1.38 mmol), followed by HATU (126 mg, 331 µmol). The resulting mixture is stirred at rt for 20 h. EtOAc is added to the mixture and the solution is washed twice with water, then with an aqueous solution of HCI (1M), and twice with brine. The organic layer is dried over Na₂SO₄, filtered off and concentrated under reduced pressure. The residue is purified by reverse layer chromatography using Gilson GX271 system, CSH 50x250mm, 5µm column (Waters^{™}), water/formic acid (0.1% v/v) with a gradient of ACN/FA (0.1% v/v) 18 to 50% in 25 min, to provide the title compound as a white solid (6 mg, 5.2% yield).

1H NMR (400 MHz, DMSO-d6) δ ppm 0.99 - 1.06 (m, 2 H), 1.10 - 1.25 (m, 3 H), 1.46 (br d, J=7 Hz, 1 H), 1.65 (br d, J=9 Hz, 2 H), 1.83 (br t, J=10 Hz, 1 H), 1.95 (br t, J=10 Hz, 1 H), 2.16 (s, 3 H), 2.30 - 2.42 (m, 1 H), 2.50 (d, J=2 Hz, 1 H), 2.62 - 2.72 (m, 1 H), 3.70 - 3.78 (m, 1 H), 4.46 - 4.54 (m, 2 H), 7.29 - 7.34 (m, 2 H), 7.57 (d, J=6 Hz, 1 H), 7.86 (br d, J=8 Hz, 1 H) LCMS [M+H]⁺ = 386; Rt = 0.56 ; Method A

The following examples were synthesized analogous to the previous procedure, using the appropriate amines:

| Ex N° | Name | NMR | LCMS Rt (min) | LCMS [M+H]+ | LCMS Analytical method |
|---|---|---|---|---|---|
| 20 | (R)-N-(1-cyclobutylpiperidin-3-yl)-2-(8-cyclopropyl-5-oxothieno[3',2':4,5]pyrrolo[1,2-d][1,2,4]triazin-6(5H)-yl)acetamide | 1H NMR (400 MHz, DMSO-d6) δ ppm 0.92 - 1.27 (m, 5 H), 1.04 - 1.04 (m, 1 H), 1.35 - 1.81 (m, 9 H), 1.86 - 2.02 (m, 2 H), 2.30 - 2.39 (m, 1 H), 2.57 - 2.67 (m, 2 H), 3.61 - 3.77 (m, 1 H), 4.50 (s, 2 H), 7.30 - 7.34 (m, 2 H), 7.57 (d, J=5 Hz, 1 H), 7.75 (d, J=8 Hz, 1 H) | 0.62 | 426 | A |
| 21 | (R)-2-(8-cyclopropyl-5-oxothieno[3',2':4,5]pyrrolo[1,2-d][1,2,4]triazin-6(5H)-yl)-N-(1-cyclopropylpiperidin-3-yl)acetamide | 1H NMR (500 MHz, DMSO-d6) δ ppm 0.22 - 0.31 (m, 2 H), 0.39 (br d, J=6 Hz, 2 H), 1.00 - 1.05 (m, 2 H), 1.11 - 1.26 (m, 3 H), 1.35 - 1.43 (m, 1 H), 1.58 - 1.70 (m, 3 H), 2.04 (br s, 1 H), 2.17 (br s, 1 H), 2.31 - 2.38 (m, 1 H), 2.71 (br s, 1 H), 2.85 (br d, J=9 Hz, 1 H), 3.62 - 3.69 (m, 1 H), 4.50 (s, 2 H), 7.32 (d, J=6 Hz, 1 H), 7.34 (s, 1 H), 7.58 (d, J=5 Hz, 1 H), 7.78 (br d, J=8 Hz, 1 H) | 0.60 | 412 | A |
| 22 | 2-(8-cyclopropyl-5-oxothieno[3',2':4,5]pyrrolo[1,2-d][1,2,4]triazin-6(5H)-yl)-N-((R)-1-((S)-2-hydroxypropyl)piperidin-3-yl)acetamide | 1H NMR (500 MHz, DMSO-d6) δ ppm 0.98 - 1.06 (m, 5 H), 1.11 - 1.18 (m, 2 H), 1.21 - 1.30 (m, 1 H), 1.44 - 1.52 (m, 1 H), 1.65 (br d, J=9 Hz, 2 H), 1.99 - 2.15 (m, 2 H), 2.17 - 2.29 (m, 2 H), 2.31 - 2.40 (m, 1 H), 2.63 - 2.66 (m, 1 H), 2.71 (br d, J=9 Hz, 1 H), 3.70 - 3.81 (m, 2 H), 4.47 - 4.55 (m, 2 H), 7.32 (d, J=6 Hz, 1 H), 7.34 (s, 1 H), 7.58 (d, J=5 Hz, 1 H), 7.84 (d, J=8 Hz, 1 H) | 0.57 | 429 | A |

### Example 13 ; (R)-2-(2-chloro-8-cyclopropyl-5-oxothieno[3',2':4,5]pyrrolo[1,2-d][1,2,4]triazin-6(5H)-yl)-N-(1-cyclopropylpiperidin-3-yl)acetamide

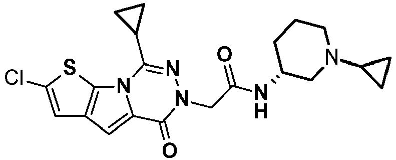

### Int 5: 2-Chloro-6H-thieno[2,3-b]pyrrole-5-carbohydrazide

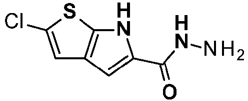

To a solution of methyl 2-chloro-6*H*-thieno[2,3-*b*]pyrrole-5-carboxylate (3.0 g, 13.91 mmol) in 60 ml of EtOH is added a solution of hydrazine hydrate (35% in water) (7.78 ml, 55.65 mmol) and the mixture is heated at reflux overnight. After cooling down the solution, the precipitate formed is filtered off, washed with ether and dried under vacuum. The title compound is obtained as a yellow solid (3g, 100% yield).

LCMS (method F): Rt = 1.15 min; MS m/z [M+H]⁺ 216.0

### Int 16: 2-Chloro-8-cylopropylthieno[3',2':4,5]pyrrolo[1,2-d][1,2,4]triazin-5(6H)-one

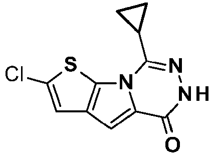

Under nitrogen, to a solution of 2-chloro-6H-thieno[2,3-b]pyrrole-5-carbohydrazide (500 mg, 2.32 mmol) in 10 ml of DMF is added methyl cyclopropanecarboximidate hydrochloride (1.10 g, 7.73 mmol). This mixture is stirred at rt for 1 h.

Then solid tBuOK (531 mg 4.64 mmol) is added and the resulting mixture is stirred in a preheated Dry-syn at 90°C for 18 h. After cooling to 0°C, the solution is acidified to pH 5 with a solution of (1M) HCI and the precipitate formed is filtered off, rinsed with water then DCM, and dried under reduced pressure in the presence of P₂O₅ to give the title compound as a brown solid. (250 mg, 40% yield).

LCMS (method F): Rt = 1.70 min; MS m/z [M+H]⁺ 266.0

### Int 17: Ethyl 2-(2-Chloro-8-cyclopropyl-5-oxothieno[3',2':4,5]pyrrolo[1,2-d][1,2,4]triazin-6(5H)-yl)acetate

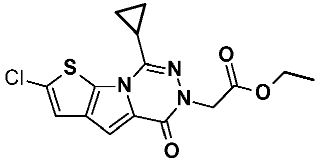

Under nitrogen, to a solution of 2-Chloro-8-cylopropylthieno[3',2':4,5]pyrrolo[1,2-d][1,2,4]triazin-5(6H)-one (650 mg, 2.45 mmol) in 15 ml of DMF is added K₂CO₃ (406 mg, 2.94 mmol) followed by a dropwise addition of ethyl iodoacetate (641 mg, 2.94 mmol). The mixture is stirred at rt for 20h. The reaction mixture is diluted with water and extracted twice with EtOAc. The organic extracts are washed with brine, dried over Na₂SO₄, filtered and concentrated under reduced pressure.

The residue is purified by Flash chromatography on silica gel using Cyclohexane/EtOAc (from 5% to 50%) to give the title compound as a light yellow solid (250 mg, 29% yield).

LCMS (method F): Rt = 2.04 min; MS m/z [M+H]⁺ 352.1

### Int 18: 2-(2-Chloro-8-cyclopropyl-5-oxothieno[3',2':4,5]pyrrolo[1,2-d][1,2,4]triazin-6(5H)-yl)acetic acid

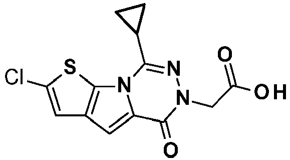

To a solution of Ethyl 2-(2-Chloro-8-cyclopropyl-5-oxothieno[3',2':4,5]pyrrolo[1,2-d][1,2,4]triazin-6(5H)-yl)acetate (220 mg, 0.625 mmol) in 10 ml of dioxane/MeOH (1/1) is added a solution of (1M) NaOH (1.88 ml, 1.88 mmol ) and the resulting solution is stirred 20 h at rt. The mixture is acidified with (1M) HCI and extracted with CH₂Cl₂. The organic layer is washed with brine, dried over Na₂SO₄, filtered and concentrated under reduced pressure to provide the title compound as a white solid (202 mg, 74% yield).

LCMS (method F): Rt = 1.72 min; MS m/z [M+H]⁺ 324.0

### Ex 13: (R)-2-(2-chloro-8-cyclopropyl-5-oxothieno[3',2':4,5]pyrrolo[1,2-d][1,2,4]triazin-6(5H)-yl)-N-(1-cyclopropylpiperidin-3-yl)acetamide

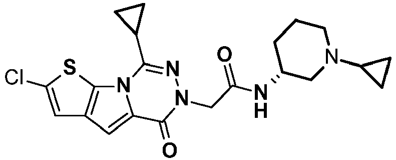

Under nitrogen, to a suspension of (*R*)-1-cyclopropylpiperidin-3-amine hydrochloride (33 mg 185 µmol) and 2-(2 chloro-8-cyclopropyl-5-oxothieno[3',2':4,5]pyrrolo[1,2-d][1,2,4]triazin-6(5H)-yl)acetic acid (50 mg, 154 µmol) in 3 ml de DMF is added DIPEA (134 µl, 772 µmol), followed by HATU (71 mg, 185 µmol). The resulting mixture is stirred at rt for 20 h.

The reaction mixture is diluted with water and extracted twice with EtOAc. The organic extracts are washed with a solution of (1M) HCI, then with brine, dried over Na₂SO₄, filtered and concentrated under reduced pressure.

The residue is purified by reverse layer chromatography using Gilson GX271 system, CSH 50x250mm, 5µm column (Waters^{™}), water/formic acid (0.1% v/v) with a gradient of ACN/FA (0.1% v/v) 18 to 50% in 25 min, to provide the title compound as a white solid (30 mg, 44% yield).

1H NMR (400 MHz, DMSO-d6) δ ppm 0.21 - 0.32 (m, 2 H), 0.35 - 0.42 (m, 2 H), 0.98 - 1.05 (m, 2 H), 1.11 - 1.27 (m, 3 H), 1.33 - 1.44 (m, 1 H), 1.56 - 1.70 (m, 3 H), 2.02 (br t, J=10 Hz, 1 H), 2.15 (br t, J=10 Hz, 1 H), 2.24 - 2.39 (m, 1 H), 2.67 - 2.74 (m, 1 H), 2.84 (br dd, J=10, 3 Hz, 1 H), 3.60 - 3.69 (m, 1 H), 4.50 (s, 2 H), 7.31 (s, 1 H), 7.49 (s, 1 H), 7.78 (br d, J=8 Hz, 1 H) LCMS [M+H]⁺ = 446 ; Rt = 0.73 ; Method A

### Example 33 ; (R)-N-(1-cyclopropylpiperidin-3-yl)-2-(8-(methoxymethyl)-5-oxothieno[3',2':4,5]pyrrolo[1,2-d][1,2,4]triazin-6(5H)-yl)acetamide

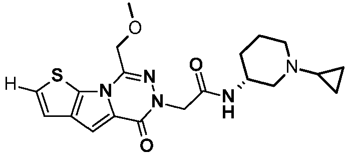

### Int 1: 6H-thieno[2,3-b]pyrrole-5-carbohydrazide

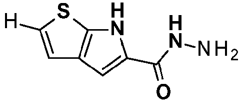

To a solution of methyl 6*H*-Thieno[2,3-*b*]pyrrole-5-carboxylate (4 g, 22.07 mmol) in 100 ml of EtOH is added hydrazine solution (35% in water) (24.7 ml, 176.59 mmol) and this mixture is heated at reflux overnight. After cooling down the solution, the precipitate formed is filtered off, washed with ether and dried under vacuum. The title compound is obtained as a white solid (3.4 g, 85% yield).

LCMS (method F): Rt = 1.15 min; MS m/z [M+H]⁺ 181.2

### Int 19: 8-(methoxymethyl)thieno[3',2':4,5]pyrrolo[1,2-d][1,2,4]triazin-5(6H)-one

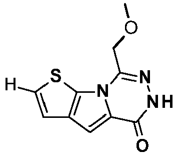

Under nitrogen, to a solution of 6H-thieno[2,3-b]pyrrole-5-carbohydrazide (500 mg, 2.76 mmol) in 20 ml of DMF is added ethyl 2-methoxyethanimidoate hydrochloride (524 mg, 3.31 mmol). This mixture is stirred at rt for 30 min. Then solid tBuOK (632 mg 5.52 mmol) is added and the resulting mixture is stirred in a preheated Dry-syn at 90°C for 1h. After cooling to 0°C, water is added, and the mixture is acidified with HCl (1M) to pH 5. The precipitate formed is filtered off, washed with water and dried under reduced pressure in the presence of P₂O₅ to provide the title product as a soft yellow solid (303 mg, 46% yield).

LCMS (method F): Rt = 1.27 min; MS m/z [M+H]⁺ 236.0

### Int 20: Ethyl 2-(8-(methoxymethyl)-5-oxothieno[3',2':4,5]pyrrolo[1,2-d][1,2,4]triazin-6(5H)-yl)acetate

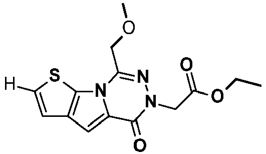

Under nitrogen, to a solution of 8-(methoxymethyl)thieno[3',2':4,5]pyrrolo[1,2-d][1,2,4]triazin-5(6H)-one (300 mg, 1.28 mmol) in 10ml of DMF is added K₂CO₃ (267 mg, 1.91 mmol), followed by a dropwise addition of ethyl iodoacetate (238 µl, 1.91 mmol). The mixture is stirred at RT for 20h. The reaction mixture is diluted with water then extracted twice with EtOAc. The combined organic layers are washed with water, then with brine, dried over Na₂SO₄, filtered off and concentrated under reduced pressure to give 450 mg of an oil which is directly engaged in the next step.

LCMS (method F): Rt = 1.64 min; MS m/z [M+H]⁺322.3

### Int 21: 2-(8-(methoxymethyl)-5-oxothieno[3',2':4,5]pyrrolo[1,2-d][1,2,4]triazin-6(5H)-yl)acetic acid

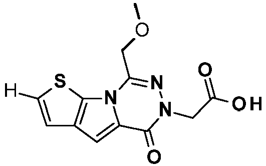

To a solution of ethyl 2-(8-(methoxymethyl)-5-oxothieno[3',2':4,5]pyrrolo[1,2-d][1,2,4]triazin-6(5H)-yl)acetate (450 mg, 1.40 mmol) in 20ml of dioxane and 20ml of MeOH is added a solution of NaOH (1M) (4.2 ml, 4.2 mmol). The resulting solution is stirred at rt overnight. The mixture is acidified with HCl (1M). The precipitate formed is filtered off, washed with water and pentane. After drying overnight under vacuum in presence of P₂O₅, the title compound is obtained as a white solid (270 mg, 65% yield).

LCMS (method F): Rt = 1.33 min; MS m/z [M+H]⁺ 294.0

### Ex 33: (R)-N-(1-cyclopropylpiperidin-3-yl)-2-(8-(methoxymethyl)-5-oxothieno[3',2':4,5]pyrrolo [1,2-d][1,2,4]triazin-6(5H)-yl)acetamide

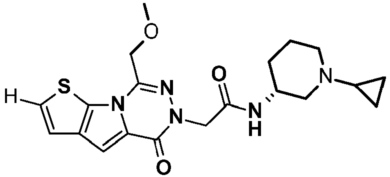

Under nitrogen, to a suspension of (*R*)-1-cyclopropylpiperidin-3-amine hydrochloride (65 mg 368 µmol) and 2-(8-(methoxymethyl)-5-oxothieno[3',2':4,5]pyrrolo[1,2-d][1,2,4]triazin-6(5H)-yl)acetic acid (90 mg, 307 µmol) in 5 ml of DMF is added DIPEA (268 µl, 1,53 mmol) followed by HATU (140 mg, 368 µmol). The resulting mixture is stirred at rt for 12h. Some drops of TFA are added and the residue is purified by reverse layer chromatography using Gilson GX271 system, CSH 50x250mm, 5µm column (Waters^{™}), water/formic acid (0.1% v/v) with a gradient of ACN/FA (0.1% v/v) 18 to 50% in 25 min, to provide after lyophilization the title compound as a white powder (20 mg, 12% yield).

1H NMR (400 MHz, DMSO-d6) δ ppm 0.69 - 0.97 (m, 4 H), 1.35 - 2.04 (m, 4 H), 2.82 - 3.15 (m, 3 H), 3.44 (s, 5 H), 3.81 - 4.01 (m, 1 H), 4.53 - 4.75 (m, 4 H), 7.27 (d, J=6 Hz, 1 H), 7.39 (s, 1 H), 7.56 (d, J=6 Hz, 1 H), 8.36 (br d, J=4 Hz, 1 H)

LCMS [M+H]⁺ = 416; Rt = 0.52 ; Method A

The following examples was synthesized analogous to the previous procedure, using the appropriate amine

| Ex N° | Name | NMR | LCMS Rt (min) | LCMS [M+H]⁺ | LCMS Analytical method |
|---|---|---|---|---|---|
| 32 | (R)-N-(1-cyclobutylpiperidin-3-yl)-2-(8-(methoxymethyl)-5-oxothieno[3',2':4,5]pyrrolo[1,2-d][1,2,4]triazin-6(5H)-yl)acetamide | 1H NMR (400 MHz, DMSO-d6) δ ppm 0.96 - 2.36 (m, 13 H), 2.73 - 3.05 (m, 2 H), 3.44 (s, 3 H), 3.74 - 3.89 (m, 1 H), 4.59 (s, 2 H), 4.66 (s, 2 H), 7.27 (d, J=6 Hz, 1 H), 7.38 (s, 1 H), 7.56 (d, J=6 Hz, 1 H), 7.90 - 8.11 (m, 1 H) | 0.55 | 430 | A |

Starting from 2-Chloro-6H-thieno[2,3-b]pyrrole-5-carbohydrazide, following examples were synthesized analogous to the previous procedure:

| Ex N° | Name | NMR | LCMS Rt (min) | LCMS [M+H]⁺ | LCMS Analytical method |
|---|---|---|---|---|---|
| 29 | (R)-2-(2-chloro-8-(methoxymethyl)-5-oxothieno[3',2':4,5]pyrrolo[1,2-d][1,2,4]triazin-6(5H)-yl)-N-(1-cyclobutylpiperidin-3-yl)acetamide formate | 1H NMR (400 MHz, DMSO-d6) δ ppm 1.14 - 1.26 (m, 1 H), 1.36 - 1.46 (m, 1 H), 1.52 - 1.81 (m, 7 H), 1.86 - 1.98 (m, 2 H), 2.54 - 2.69 (m, 4 H), 3.44 (s, 3 H), 3.63 - 3.75 (m, 1 H), 4.56 (s, 2 H), 4.65 (s, 2 H), 7.35 (s, 1 H), 7.43 (s, 1 H), 7.90 (d, J=8 Hz, 1 H) | 0.69 | 464 | A |
| 30 | (R)-2-(2-chloro-8-(methoxymethyl)-5-oxothieno[3',2':4,5]pyrrolo[1,2-d][1,2,4]triazin-6(5H)-yl)-N-(1-cyclopropylpiperidin-3-yl)acetamide formate | 1H NMR (400 MHz, DMSO-d6) δ ppm 0.27 (br s, 2 H), 0.39 (br d, J=6 Hz, 2 H), 1.13 - 1.47 (m, 2 H), 1.60 (br s, 3 H), 1.96 - 2.22 (m, 2 H), 2.63 - 2.94 (m, 2 H), 3.44 (s, 3 H), 3.56 - 3.72 (m, 1 H), 4.56 (s, 2 H), 4.65 (s, 2 H), 7.35 (s, 1 H), 7.43 (s, 1 H), 7.89 (br d, J=7 Hz, 1 H) | 0.67 | 450 | A |

### Example 60 ; (R)-N-(1-cyclopropylpiperidin-3-yl)-2-(8-(ethyl)-5-oxothieno[3',2':4,5] pyrrolo[1,2-d][1,2,4]triazin-6(5H)-yl)acetamide

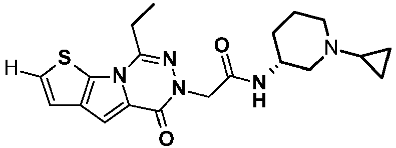

### Int 1: 6H-thieno[2,3-b]pyrrole-5-carbohydrazide

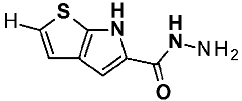

To a solution of methyl 6H-thieno[2,3-b]pyrrole-5-carboxylate (4 g, 22.07 mmol) in 100 ml of EtOH is added hydrazine solution (35% in water) (24.7 ml, 176.59 mmol) and this mixture is heated at reflux overnight. After cooling down the solution, the precipitate formed is filtered off, washed with ether, and dried under vacuum. The title compound is obtained as a white solid (3.4 g, 85% yield).

LCMS (method F): Rt = 1.15 min; MS m/z [M+H]⁺ 181.2

### Int 22: 8-(ethyl)thieno[3',2':4,5]pyrrolo[1,2-d][1,2,4]triazin-5(6H)-one

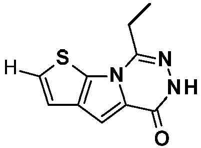

Under nitrogen, to a solution of 6H-thieno[2,3-b]pyrrole-5-carbohydrazide (1.0 g, 6.53 mmol) in 15 ml of DMF is added ethyl propanecarboximidate hydrochloride (1.08 g, 7.83 mmol). This mixture is stirred at rt for 1 h. Then solid tBuOK (1.50 g, 13.05 mmol) is added and the resulting mixture is stirred in a preheated Dry-syn at 90°C for 1h30. After cooling to 0°C, water is added, and the mixture is acidified with HCl (1M) to pH 5. The precipitate formed is filtered off, washed with water and dried under reduced pressure in the presence of P₂O₅ to provide the title product as a soft yellow solid (1.2 g, 84% yield).

LCMS (method F): Rt = 1.38 min; MS m/z [M+H]⁺ 220.1

### Int 23: Ethyl 2-(8-(ethyl)-5-oxothieno[3',2':4,5]pyrrolo[1,2-d][1,2,4]triazin-6(5H)-yl)acetate

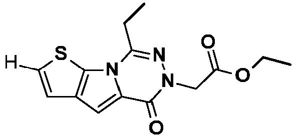

Under nitrogen, to a solution of 8-(ethyl)thieno[3',2':4,5]pyrrolo[1,2-d][1,2,4]triazin-5(6H)-one (1.2 g, 5.5 mmol) in 15 ml of DMF is added Cs₂CO₃ (7.1 g, 22 mmol), followed by a dropwise addition of ethyl iodoacetate (780 µl, 6.6 mmol). The mixture is stirred at RT for 20h.

The reaction mixture is diluted with water then extracted twice with EtOAc. The combined organic layers are washed with water, then with brine, dried over Na₂SO₄, filtered off and concentrated under reduced pressure to give 900 mg of an oil which is used without further purification in the next step.

LCMS (method F): Rt = 1.78 min; MS m/z [M+H]⁺ 306.2

### Int 24: 2-(8-(ethyl)-5-oxothieno[3',2':4,5]pyrrolo[1,2-d][1,2,4]triazin-6(5H)-yl)acetic acid

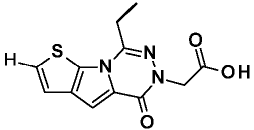

To a solution of ethyl 2-(8-(ethyl)-5-oxothieno[3',2':4,5]pyrrolo[1,2-d][1,2,4]triazin-6(5H)-yl)acetate (900 mg, 2.95 mmol) in 6 ml of THF is added a solution of NaOH (1M) (3 ml, 3 mmol). The resulting solution is stirred at rt overnight. The mixture is acidified with HCl (1M). The organic layer is extracted with DCM, concentrated under reduced pressure and the residue is triturated with Et₂O, then filtered. After drying overnight under vacuum in presence of P₂O₅ the title compound is obtained as a yellow solid (830 mg, 100% yield).

LCMS (method F): Rt = 1.42 min; MS m/z [M+H]⁺ 278.0

### Ex 60: (R)-N-(1-cyclopropylpiperidin-3-yl)-2-(8-(ethyl)-5-oxothieno[3',2':4,5]pyrrolo[1,2-d][1,2,4]triazin-6(5H)-yl)acetamide

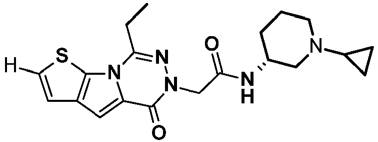

Under nitrogen, to a suspension of (R)-1-cyclopropylpiperidin-3-amine hydrochloride (46 mg, 216 µmol) and 2-(8-(ethyl)-5-oxothieno[3',2':4,5]pyrrolo[1,2-d][1,2,4]triazin-6(5H)-yl)acetic acid (50 mg, 180 µmol) in 5 ml of ACN is added DIPEA (157 µl, 0.900 mmol) followed by HATU (82 mg, 216 µmol). The resulting mixture is stirred at rt for 1h30. After evaporation under reduced pressure, the residue is purified by reverse layer chromatography using Gilson GX271 system, CSH 50x250mm, 5µm column (Waters^{™}), water/formic acid (0.1% v/v) with a gradient of ACN/FA (0.1% v/v) 14 to 45% in 20 min, to provide after lyophilization the title compound as a white powder (10 mg, 14% yield).

1H NMR (400 MHz, DMSO-d6) δ ppm 0.20 - 0.29 (m, 2 H), 0.34 - 0.42 (m, 2 H), 1.19 - 1.43 (m, 5 H), 1.55 - 1.71 (m, 3 H), 2.04 (br t, J=10 Hz, 1 H), 2.11 - 2.21 (m, 1 H), 2.61 - 2.73 (m, 1 H), 2.84 (br d, J=7 Hz, 1 H), 3.05 (q, J=7 Hz, 2 H), 3.65 (br d, J=9 Hz, 1 H), 4.54 (s, 2 H), 7.31 (d, J=5 Hz, 1 H), 7.34 (s, 1 H), 7.57 (d, J=5 Hz, 1 H), 7.81 (br d, J=8 Hz, 1 H)

LCMS [M+H]⁺ = 400 ; Rt = 0.57 ; Method A

The following examples were synthesized analogous to the previous procedure, using the appropriate amines

| Ex N° | Name | NMR | LCMS Rt (min) | LCMS [M+H]⁺ | LCMS Analytical method |
|---|---|---|---|---|---|
| 73 | 2-(12-ethyl-9-oxo-3-thia-1,10,11-triazatricyclo [6.4.0.02,6]dodeca-2(6),4,7,11-tetraen-10-yl)-N-[(3R)-1-methyl-3-piperidyl]acetamide | 1H NMR (400 MHz, DMSO-d6) δ ppm 1.19 - 1.35 (m, 4 H), 1.42 - 1.55 (m, 1 H), 1.68 (br d, J=9 Hz, 2 H), 1.92 (br d, J=7 Hz, 1 H), 2.04 (br s, 1 H), 2.22 (s, 3 H), 2.54 - 2.63 (m, 1 H), 2.67 - 2.75 (m, 1 H), 3.04 (q, J=7 Hz, 2 H), 3.77 (br d, J=8 Hz, 1 H), 4.55 (s, 2 H), 7.31 (d, J=5 Hz, 1 H), 7.34 (s, 1 H), 7.57 (d, J=5 Hz, 1 H), 7.95 (br d, J=8 Hz, 1 H) | 0.54 | 374 | A |
| 69 | 2-(12-ethyl-9-oxo-3-thia-1,10,11-triazatricyclo[6.4.0.02,6]dodeca-2(6),4,7,11-tetraen-10-yl)-N-[(3R)-1-[(2S)-2-hydroxypropyl]-3-piperidyl]acetamide | 1H NMR (400 MHz, DMSO-d6) δ ppm 1.10 (br d, J=6 Hz, 3 H), 1.32 (t, J=7 Hz, 3 H), 1.37 - 1.47 (m, 1 H), 1.68 - 1.93 (m, 3 H), 2.63 - 2.71 (m, 1 H), 2.80 (br s, 1 H), 2.94 - 3.15 (m, 4 H), 3.49 (br s, 2 H), 3.90 - 4.31 (m, 2 H), 4.57 (s, 2 H), 5.40 (br s, 1 H), 7.32 (d, J=5 Hz, 1 H), 7.35 (s, 1 H), 7.58 (d, J=5 Hz, 1 H), 8.21 (br d, J=7 Hz, 1 H) | 0.55 | 418 | A |

The following example was synthesized analogous to the previous procedure, using the appropriate imidate:

| Ex N° | Name | NMR | LCMS Rt (min) | LCMS [M+H]⁺ | LCMS Analytical method |
|---|---|---|---|---|---|
| 72 | N-[(3R)-1-cyclopropylpiperidin-3-yl]-2-(9-oxo-12-propyl-3-thia-1,10,11-triazatricyclo [6.4.0.02,6]dodeca-2(6),4,7,11-tetraen-10-yl)acetamide | 1H NMR (400 MHz, DMSO-d6) δ ppm 0.25 (d, J = 1.1 Hz, 2H), 0.38 (d, J = 5.8 Hz, 2H), 1.06 (t, J = 6.8 Hz, 3H), 1.23 (brs, 1H), 1.30 - 1.46 (m, 1H), 1.59 (d, J = 2.6 Hz, 3H), 1.79 (d, J = 7.0 Hz, 2H), 1.97 - 2.06 (m, 1H), 2.11 - 2.20 (m, 1H), 2.69 (ddd, J = 2.2, 4.8, 7.9 Hz, 1H), 2.72 (d, J = 9.0 Hz, 1H), 2.96 (t, J = 7.0 Hz, 2H), 3.63 - 3.65 (m, 1H), 4.54 (brs, 2H), 7.31 - 7.35 (m, 2H), 7.57 (d, J = 4.9 Hz, 1H), 7.86 (d, J = 7.4 Hz, 1H). | 0.66 | 414 | A |

### Example 44 and 51; N-((R)-1-cyclopropylpiperidin-3-yl)-2-(8-(1-hydroxyethyl)-5-oxothieno[3',2':4,5]pyrrolo[1,2-d][1,2,4]triazin-6(5H)-yl)acetamide

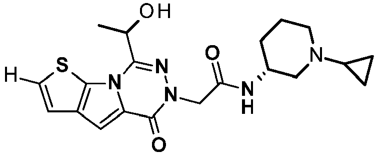

### Int 1: 6H-thieno[2,3-b]pyrrole-5-carbohydrazide

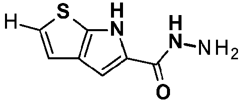

To a solution of methyl 6*H*-Thieno[2,3-*b*]pyrrole-5-carboxylate (4 g, 22.07 mmol) in 100 ml of EtOH is added hydrazine solution (35% in water) (24.7 ml, 176.59 mmol) and this solution is heated at reflux overnight. After cooling down the solution, the precipitate formed is filtered off, washed with ether and dried under vacuum. The title compound is obtained as a white solid (3.4 g, 85% yield).

LCMS (method F): Rt = 1.15 min; MS m/z [M+H]⁺ 181.2

### Int 25: 8-(1-hydroxyethyl)-thieno[3',2':4,5]pyrrolo[1,2-d][1,2,4]triazin-5(6H)-one

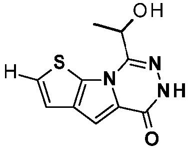

Under nitrogen, to a solution of 6H-thieno[2,3-b]pyrrole-5-carbohydrazide (2,5g, 13,80 mmol) in 50 ml of DMF is added ethyl 2-hydroxypropanimidoate hydrochloride (2,74 g, 16,97 mmol). This mixture is stirred at rt for 1h30. Then, solid tBuOK (3.16g, 27.59 mmol) is added and the resulting mixture is stirred in a preheated Dry-syn at 90°C for 1h30.

After cooling to 0°C, the mixture is acidified with HCl (1M). The precipitate formed is filtered off, washed with water and dried under reduced pressure in the presence of P₂O₅ to provide the title compound as a soft yellow solid (800 mg, 25% yield).

LCMS (method F): Rt = 1.25 min; MS m/z [M+H]⁺ 236.1

### Int 26: Ethyl 2-(8-(1-hydroxyethyl)-5-oxothieno[3',2':4,5]pyrrolo[1,2-d][1,2,4]triazin-6(5H)-yl)acetate

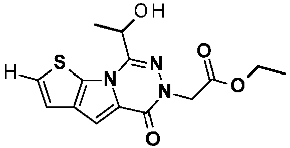

To a solution of 8-(1-hydroxyethyl)thieno[3',2':4,5]pyrrolo[1,2-d][1,2,4]triazin-5(6H)-one (350 mg, 1,49 mmol) in 6 ml of DMF is added Cs₂CO₃ (581 mg, 1,79 mmol), followed by a dropwise addition of ethyl iodoacetate (222 µl, 1.79 mmol). The resulting mixture is stirred at rt for 20h. Then, the mixture is diluted with water and extracted twice with EtOAc. The combined organic layers are dried over Na₂SO₄, filtered off and concentrated under reduced pressure to give the title compound as a light marron powder (400 mg, 83% yield).

LCMS (method F): Rt = 1.54 min; MS m/z [M+H]⁺322.1

### Int 27: 2-(8-(1-hydroxyethyl)-5-oxothieno[3',2':4,5]pyrrolo[1,2-d][1,2,4]triazin-6(5H)-yl)acetic acid

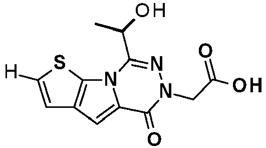

To a solution of ethyl 2-(8-(1-hydroxyethyl)-5-oxothieno[3',2':4,5]pyrrolo[1,2-d][1,2,4]triazin-6(5H)-yl)acetate (250 mg, 778 µmol) in 10 ml of THF and 3 ml of MeOH is added a solution of NaOH (1M) (3.89 ml 3.89 mmol). The resulting solution is stirred at rt overnight. The mixture is acidified with (1M) HCl and extracted with CH₂Cl₂. The organic layer is washed with brine, dried over Na₂SO₄, filtered and evaporated to provide the title compound as a brown oil (230 mg, 100% yield).

LCMS (method F): Rt = 1.20 min; MS m/z [M+H]⁺ 294.1

### Ex 44: N-((R)-1-cyclopropylpiperidin-3-yl)-2-(8-(1-hydroxyethyl)-5-oxothieno[3',2':4,5] pyrrolo[1,2-d][1,2,4]triazin-6(5H)-yl)acetamide (Dia 1)

### Ex 51: N-((R)-1-cyclopropylpiperidin-3-yl)-2-(8-(1-hydroxyethyl)-5-oxothieno[3',2':4,5] pyrrolo[1,2-d][1,2,4]triazin-6(5H)-yl)acetamide (Dia 2)

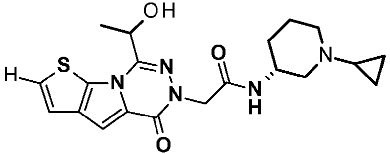

Under nitrogen, to a suspension of (R)-1-cyclopropylpiperidin-3-amine hydrochloride (60 mg, 341 µmol) and 2-(8-(1-hydroxyethyl)-5-oxothieno[3',2':4,5]pyrrolo[1,2-d][1,2,4]triazin-6(5H)-yl)acetic acid (100 mg, 341 µmol) in 5 ml de DMF is added DIPEA (298 µl, 1,70 mmol) followed by HATU (1565 mg, 409 µmol). The resulting mixture is stirred at rt for 20 h. After evaporation under reduced pressure, the residue is purified by reverse layer chromatography using Gilson GX271 system, CSH 50x250mm, 5µm column (Waters^{™}), water/formic acid (0.1% v/v) with a gradient of ACN/FA (0.1% v/v) 18 to 50% in 25 min, to provide the two diastereoisomers: Dia 1 as a yellow powder (15 mg, 10% yield) and Dia 2 as an orange powder (12 mg, 8% yield).

### 1^{st} diastereoisomer (Dia 1):

1H NMR (400 MHz, DMSO-d6) δ ppm 0.71 - 0.96 (m, 4 H), 1.38 - 1.71 (m, 5 H), 1.75 - 2.02 (m, 2 H), 2.84 - 3.14 (m, 3 H), 3.37 - 3.54 (m, 2 H), 3.88 (br s, 1 H), 4.94 - 5.01 (m, 1 H), 5.16 - 5.30 (m, 2 H), 6.01 (br s, 1 H), 7.07 - 7.13 (m, 2 H), 7.19 (d, J=5 Hz, 1 H), 8.58 (br s, 1 H) LCMS [M+H]⁺ = 416; Rt = 0.46 ; Method A

### 2^{nd} diastereoisomer (Dia 2):

1H NMR (400 MHz, DMSO-d6) δ ppm 0.71 - 0.95 (m, 4 H), 1.53 (d, J=7 Hz, 5 H), 1.75 - 2.01 (m, 2 H), 2.81 - 2.99 (m, 2 H), 3.01 - 3.16 (m, 1 H), 3.37 - 3.56 (m, 2 H), 3.88 (br s, 1 H), 4.94 - 5.01 (m, 1 H), 5.16 - 5.30 (m, 2 H), 6.01 (br s, 1 H), 7.07 - 7.13 (m, 2 H), 7.19 (d, J=5 Hz, 1 H), 8.58 (br s, 1 H)
LCMS [M+H]⁺ = 416; Rt = 0.49 ; Method A

The following examples were synthesized analogous to the previous procedure, using the appropriate amine:

| Ex N° | Name | NMR | LCMS Rt (min) | LCMS [M+H]+ | LCMS Analytical method |
|---|---|---|---|---|---|
| 50 Dial | N-((R)-1-cyclobutylpiperidin-3-yl)-2-(8-(1-hydroxyethyl)-5-oxothieno[3',2':4,5]pyrrolo[1,2-d][1,2,4]triazin-6(5H)-yl)acetamide | 1H NMR (400 MHz, DMSO-d6) δ ppm 1.38 - 1.48 (m, 1 H), 1.52 (d, J=6 Hz, 3 H), 1.61 - 1.81 (m, 3 H), 1.84 - 1.98 (m, 2 H), 2.07 - 2.24 (m, 4 H), 2.44 - 2.50 (m, 1 H), 2.60 - 2.73 (m, 1 H), 3.31 - 3.36 (m, 2 H), 3.61 - 3.74 (m, 1 H), 3.85 - 4.01 (m, 1 H), 4.52 - 4.71 (m, 2 H), 4.95 - 5.04 (m, 1 H), 6.21 (br d, J=4 Hz, 1 H), 7.25 (d, J=6 Hz, 1 H), 7.38 (s, 1 H), 7.53 (d, J=6 Hz, 1 H), 8.33 (br d, J=7 Hz, 1 H) | 0.48 | 430 | A |
| 81 Dia2 | N-((R)-1-cyclobutylpiperidin-3-yl)-2-(8-(1-hydroxyethyl)-5-oxothieno[3',2':4,5]pyrrolo[1,2-d][1,2,4]triazin-6(5H)-yl)acetamide | 1H NMR (400 MHz, DMSO-d6) δ ppm 1.43 - 1.57 (m, 4 H), 1.59 - 1.80 (m, 3 H), 1.83 - 2.02 (m, 2 H), 2.03 - 2.22 (m, 4 H), 2.53 - 2.59 (m, 1 H), 2.62 - 2.74 (m, 1 H), 3.19 - 3.30 (m, 2 H), 3.68 (br d, J=8 Hz, 1 H), 3.90 (br s, 1 H), 4.88 - 5.08 (m, 1 H), 5.21 (s, 2 H), 6.00 (br d, J=5 Hz, 1 H), 7.07 - 7.14 (m, 2 H), 7.19 (d, J=5 Hz, 1 H), 8.55 (br d, J=7 Hz, 1 H) | 0.51 | 430 | A |

### Example 52; N-((R)-1-cyclopropylpiperidin-3-yl)-2-(8-(2-hydroxypropan-2-yl)-5-oxothieno[3',2':4,5]pyrrolo[1,2-d][1,2,4]triazin-6(5H)-yl)acetamide

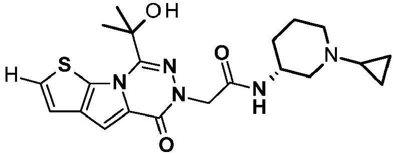

### Int 28: 8-acetylthieno[3',2':4,5]pyrrolo[1,2-d][1,2,4]triazin-5(6H)-one

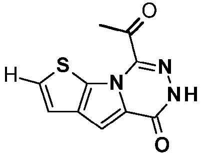

To a solution of 8-(1-hydroxyethyl)-thieno[3',2':4,5]pyrrolo[1,2-d][1,2,4]triazin-5(6H)-one (int 28) (400 mg, 1,70 mmol) in 15 ml of DMF is added Dess-Martin periodinane (1,14 g, 2,55 mmol) and the mixture is stirred at rt for 30 min. Then, an aqueous saturated solution of NaHCO₃ is added and the precipitate formed is filtered off, rinsed with DCM to provide the title compound as a marron solid (395 mg, 100% yield).

LCMS (method F): Rt = 1.36 min; MS m/z [M+H]⁺ 234

### Int 29: 8-(2-hydroxypropan-2-yl)thieno[3',2':4,5]pyrrolo[1,2-d][1,2,4]triazin-5(6H)-one

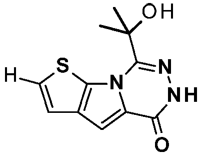

Under nitrogen, a solution (0.6M in THF) of lanthanum(III) chloride bis(lithium chloride) complex (11,43 ml, 6,86 mmol) is added to a solution of 8-acetylthieno[3',2':4,5]pyrrolo[1,2-d][1,2,4]triazin-5(6H)-one (400 mg, 1,71 mmol) in 8 ml of anhydrous THF. The temperature is decreased to 0°C, then a solution (3M in THF) of methylmagnesium chloride is added dropwise with a control of the temperature between 5 and 8°C. After total addition, the mixture is stirred 1h at RT. Then, an aqueous solution of NH₄Cl (10%) is added, plus acidification with HCl (1M) and the resulting mixture is extracted with DCM (3 x). The combined organic layers are dried over Na₂SO₄, filtered off and concentrated under reduced pressure to give the title compound as a brown solid (220 mg, 51% yield).

LCMS (method F): Rt = 1.42 min; MS m/z [M+H]⁺ 250.1

### Int 30: 2-(8-(2-hydroxypropan-2-yl)-5-oxothieno[3',2':4,5]pyrrolo[1,2-d][1,2,4]triazin-6(5H)-yl)acetic acid

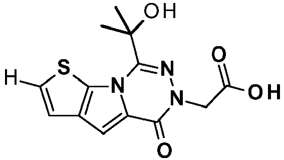

To a solution of 8-(2-hydroxypropan-2-yl)thieno[3',2':4,5]pyrrolo[1,2-d][1,2,4]triazin-5(6H)-one (220 mg, 0.882 mmol) in 10 ml of DMF is added Cs₂CO₃ (345 mg, 1.06 mmol), followed by a dropwise addition of ethyl iodoacetate (128 µl, 1.06 mmol). The resulting mixture is stirred at rt for 20h and 3h at 60°C. After cooling to rt, the mixture is diluted with water and extracted twice with EtOAc. The combined organic layers are dried over Na₂SO₄, filtered off and concentrated under reduced pressure to give the title compound as a light marron wax (290 mg, purity 64%), used without further purification in the next step.

LCMS (method F): Rt = 1.32 min; MS m/z [M+H]⁺ 308

### Ex 52: N-((R)-1-cyclopropylpiperidin-3-yl)-2-(8-(2-hydroxypropan-2-yl)-5-oxothieno[3',2':4,5]pyrrolo [1,2-d][1,2,4]triazin-6(5H)-yl)acetamide

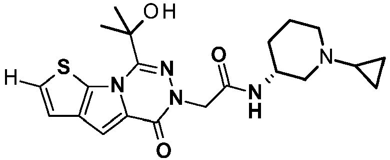

Under nitrogen, to a suspension of (*R*)-1-cyclopropylpiperidin-3-amine hydrochloride (44 mg, 246 µmol) and 2-(8-(2-hydroxypropan-2-yl)-5-oxothieno[3',2':4,5]pyrrolo[1,2-d][1,2,4]triazin-6(5H)-yl)acetic acid (63 mg, 205 µmol) in 2 ml de DMF is added DIPEA (179 µl, 1,02 mmol) followed by HATU (94 mg, 246 µmol). The resulting mixture is stirred at rt for 20 h. After evaporation under reduced pressure, the residue is purified by reverse layer chromatography using Gilson GX271 system, CSH 50x250mm, 5µm column (Waters^{™}), water/formic acid (0.1% v/v) with a gradient of ACN/FA (0.1% v/v) 2 to 30% in 40 min, to provide after lyophilization the title compound as a white powder (3 mg, 3.5% yield).
1H NMR (500 MHz, DMSO-d6) δ ppm 0.15 - 0.48 (m, 4 H), 1.20 - 1.43 (m, 3 H), 1.58 (d, J=11 Hz, 9 H), 1.95 - 2.89 (m, 3 H), 3.58 - 3.73 (m, 1 H), 4.54 (s, 2 H), 6.33 (s, 1 H), 7.22 (d, J=6 Hz, 1 H), 7.39 (s, 1 H), 7.45 (d, J=6 Hz, 1 H), 7.79 - 8.06 (m, 1 H)
LCMS [M+H]⁺ = 430 ; Rt = 1.98-2.00 ; Method C

### Example 79; N-[(3R)-1-(1-hydroxy-2-methylpropan-2-yl)piperidin-3-yl]-2-(8-isopropyl-5-oxothieno[3',2':4,5]pyrrolo[1,2-d][1,2,4]triazin-6(5H)-yl)acetamide

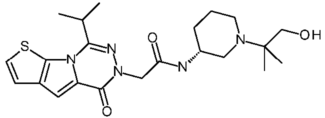

At 0°C, to a solution of ethyl-2-methyl-2-[3-[[2-(8-isopropyl-5-oxothieno[3',2':4,5]pyrrolo[1,2-d][1,2,4]triazin-6(5H)-yl)acetyl]amino]piperidin-1-yl]propanoate - (Example 80,130 mg, 0,27 mmol) in 20 ml of THF is added dropwise a (2M in THF) solution of LAH (0,2 ml, 0,4 mmol) and the resulting mixture is stirred at RT for 2 hours. The reaction mixture is quenched at 0°C by addition of a saturated aqueous solution of NH₄Cl. After addition of EtOAc, the organic layer is washed with NaCl (3 times), dried over MgSO₄, filtered and concentrated under reduced pressure.

The residue is purified by reverse phase chromatography Waters SunFire PrepC18 OBD 30 x 100 mm column, water containing 0.1% HCO₂H with a gradient of acetonitrile 10 to 60% in 15 min.

After concentration under vacuum and drying overnight, the title compound is obtained as a white solid (95 mg, 80% yield)

1H NMR (400 MHz, DMSO-d6) δ ppm 0.92 (s, 6 H), 1.24 (m, 1 H), 1.35 (d, J=7 Hz, 6 H), 1.45 (m, 1 H), 1.65 (d, J=9 Hz, 2 H), 2.17 (br dd, J=11, 9 Hz, 1 H), 2.26 - 2.34 (m, 1 H), 2.71 (m, 1 H), 2.85 (m, 1 H), 3.31 - 3.42 (m partially hidden, 3 H), 3.71 - 3.78 (m, 2 H), 4.47 - 4.63 (m, 2 H), 7.32 (d, J=6 Hz, 1 H), 7.37 (s, 1 H), 7.58 (d, J=6 Hz, 1 H), 7.83 (d, J=8 Hz, 1 H) LCMS [M+H]⁺ = 446 ; Rt = 2.55 ; Method A

### Example 88 ; N-[(1S,4R)-2-azabicyclo[2.2.1]heptan-6-yl]-2-(12-isopropyl-9-oxo-3-thia-1,10,11-triazatricyclo[6.4.0.02,6]dodeca-2(6),4,7,11-tetraen-10-yl)acetamide hydrochloride

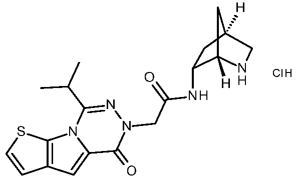

A solution of tert-butyl (1R,4R)-6-[[2-(12-isopropyl-9-oxo-3-thia-1,10,11-triazatricyclo [6.4.0.02,6]dodeca-2(6),4,7,11-tetraen-10-yl)acetyl]amino]-2-azabicyclo[2.2.1]heptane-2-carboxylate obtained following process as described in example 1 and using appropriate amine (1,1-Dimethylethyl 6-amino-2-azabicyclo[2.2.1]heptane-2-carboxylate) (676 mg, 1,39mmol) in 10 ml of DCM is treated with hydrochloric acid 4M in dioxane (1,5 ml, 6 mmol) and stirred at rt overnight. The reaction mixture is concentrated under reduced pressure. The precipitate formed is filtered off to give 830 mg of the title as a brown solid.

200 mg of this solid is purified by reverse phase chromatography Waters SunFire PrepC18 OBD 30 x 100 mm column, water containing 0.1% HCO₂H with a gradient of acetonitrile 10 to 60% in 15 min.

After concentration under vacuum and drying overnight, 80 mg of the title compound is obtained as a beige solid.

1H NMR (400 MHz, DMSO-d6) δ ppm 1.09 (br d, J=13 Hz, 1 H), 1.35 (dd, J=7, 2 Hz, 6 H), 1.48 - 1.56 (m, 1 H), 1.59 - 1.66 (m, 1 H), 1.97 - 2.07 (m, 1 H), 2.39 - 2.43 (m, 1 H), 2.64 - 2.74 (m, 1 H), 2.92 (br d, J=9 Hz, 1 H), 3.29 - 3.41 (m, 1 H), 3.53 (br s, 1 H), 4.03 (br dd, J=7, 4 Hz, 1 H), 4.54 - 4.68 (m, 2 H), 7.32 (d, J=5 Hz, 1 H), 7.37 (s, 1 H), 7.59 (d, J=6 Hz, 1 H), 8.25 (s, 2 H)

LCMS [M+H]⁺ = 386 ; Rt = 0.62 ; Method A

### Example 82 ; 2-(12-isopropyl-9-oxo-3-thia-1,10,11-triazatricyclo[6.4.0.02,6]dodeca-2(6),4,7,11-tetraen-10-yl)-N-[(1S,4S)-2-methyl-2-azabicyclo[2.2.1]heptan-6-yl]acetamide

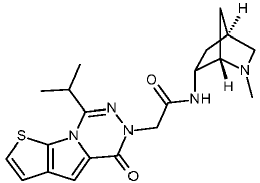

To a solution of N-[(1R,4S)-2-azabicyclo[2.2.1]heptan-6-yl]-2-(12-isopropyl-9-oxo-3-thia-1,10,11-triazatricyclo[6.4.0.02,6]dodeca-2(6),4,7,11-tetraen-10-yl)acetamide - example 88 (150 mg, 0,39 mmol) in 15 ml of MeOH, Formaldehyde solution (0,22 mL, 2,14 mmol), sodium cyanoborohydride (97,8 mg, 1,56 mmol) and acetic acid (0,018 ml, 0,32 mmol) are subsequently added and the mixture is stirred at 45°C overnight.

The reaction mixture is concentrated under reduced pressure and the residue diluted with AcOEt. The organic layers are washed with NaHCO₃ sat, water, dried over MgSO₄, filtered and concentrated under reduced pressure.

The residue is purified by reverse phase chromatography Waters SunFire PrepC18 OBD 30 x 100 mm column, water containing 0.1% HCO₂H with a gradient of acetonitrile 10 to 60% in 15 min.

After concentration under vacuum and drying overnight, the title compound is obtained as a white solid (62 mg, 40% yield)

1H NMR (400 MHz, DMSO-d6) δ ppm 0.82 (dt, J=12, 3 Hz, 1 H), 1.28 (br d, J=10 Hz, 1 H), 1.37 (d, J=7 Hz, 6 H), 1.58 (br d, J=10 Hz, 1 H), 1.85 - 1.93 (m, 1 H), 2.18 - 2.25 (m, 4 H), 2.30 - 2.36 (m, 1 H), 2.67 - 2.72 (m, 1 H), 2.90 (br s, 1 H), 3.37 - 3.41 (m, 1 H), 3.91 - 3.98 (m, 1 H), 4.53 - 4.66 (m, 2 H), 7.32 (d, J=6 Hz, 1 H), 7.39 (s, 1 H), 7.59 (d, J=6 Hz, 1 H), 7.73 (br d, J=8 Hz, 1 H)

LCMS [M+H]⁺ = 400 ; Rt = 0.62 ; Method A

### Synthesis of amines building block

### Intermediate 31 : (3R)-1-(1-methylcyclopentyl)piperidin-3-aminehydrochloride

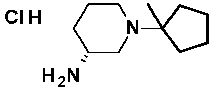

### Int 32: tert-butyl N-[(3R)-1-(1-cyanocyclopentyl)-3-piperidyl]carbamate

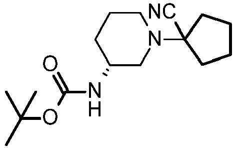

To a solution of tert-butyl N-[(3R)-3-piperidyl]carbamate ( 2 g, 9,98 mmol) and cyclopentanone (0,88 ml, 9,98 mmol) in 20 mL of DCM was added titanium(IV) isopropoxide (3,0 ml , 9,98 mmol) and the mixture was stirred at room temperature for 40 hours. Diethylaluminum cyanide (1M in toluene) (11,98 ml, 11,98 mmol) was added and the solution is stirred for an additional 20 hours. After dilution with DCM, water is added and the resulting mixture is filtered, washed with DCM and water. The aqueous layer is extracted with DCM. The organic layers are grouped, dried over MgSO4, filtered and concentrated under reduced pressure.

The residue is purified by flash chromatography on silica gel using Heptane/AcOEt (from 0% to 50%) to give the title compound (944 mg, 33% yield) as a yellow oil.

LCMS (method A): Rt = 1.17 min; MS m/z [M+H]+ 294

### Int 33: tert-butyl N-[(3R)-1-(1-methylcyclopentyl)-3-piperidyl]carbamate

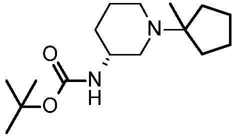

Under N2, a solution of tert-butyl N-[(3R)-1-(1-cyanocyclopentyl)-3-piperidyl]carbamate (300 mg, 1,02 mmol) in 5 mL of THF is cooled to 0°C and methylmagnesium bromide 3M in Et2O (1,70 ml , 5,11 mmol) is added dropwise. The mixture is stirred at 0°C for 1 hours and at rt overnight. The reaction is quenched with saturated aqueous NH4Cl and the aqueous layer is extracted with AcOEt. The organic layers are dried over MgSO4, filtered and concentrated under reduced pressure.

The residue is purified by Flash chromatography on silica gel using DCM/MeOH (from 0% to 10%) to give the title compound (85 mg, 30% yield) as a yellow oil.

LCMS (method A): Rt = 0.54 min; MS m/z [M+H]+ 283

### Int 31: (3R)-1-(1-methylcyclopentyl)piperidin-3-aminehydrochloride

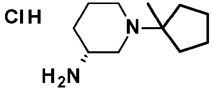

A solution of tert-butyl N-[(3R)-1-(1-methylcyclopentyl)-3-piperidyl]carbamate ( 85 mg, 0,30 mmol) in 5 mL of DCM was treated with hydrochloric acid 4M in dioxane (0.15 mL, 0,60mmol) and stirred 4 hours at rt. The reaction mixture concentrated under reduced pressure. The precipitate formed is filtered off to give 68 mg of an orange oil which is directly engaged in the next step.

### Intermediate 34: ethyl 2-[(3R)-3-amino-1-piperidyl]-2-methyl-propanoate; hydrochloride

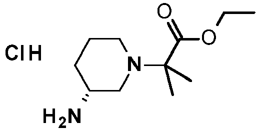

### Int 35: ethyl 2-[(3R)-3-(tert-butoxycarbonylamino)-1-piperidyl]-2-methyl-propanoate

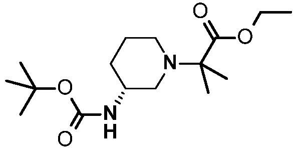

To a solution of tert-butyl N-[(3R)-3-piperidyl]carbamate (4 g, 20 mmol) in 80 ml of acetonitrile is added K2CO3 (6,8 g, 49,5 mmol) and Ethyl 2-bromoisobutyrate (5,76 g, 29,56 mmol).The mixture is stirred at 80°C for 72 hours. After cooling to rt, the mixture is diluted with water and extracted twice with EtOAc. The combined organic layers are washed with brine and dried over Na₂SO₄, filtered off and concentrated under reduced pressure.

The residue is purified by flash chromatography on silica gel using heptane/EtOAc (from 0% to 30%) to give the title compound as a colorless oil (4,2 g, 66% yield).

LCMS (method D): Rt = 1.71 min; MS m/z [M+H]⁺ 315

### Int 34: ethyl 2-[(3R)-3-amino-1-piperidyl]-2-methyl-propanoate; hydrochloride

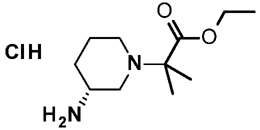

To a solution of ethyl 2-[(3R)-3-(tert-butoxycarbonylamino)-1-piperidyl]-2-methyl-propanoate (Int 35) (4,2 g, 13,4 mmol) in 40 ml of dioxane is added a solution of hydrochloric acid 4M in dioxane (34 ml, 136 mmol) and the mixture is stirred at room temperature for 20 hours. The mixture is then concentrated under reduced pressure and the residue is triturated in Et₂O. After drying overnight under vacuum in presence of P₂O₅, the title compound is obtained as a white solid (3,5 g, 98% yield).

LCMS (method B): Rt = 0.16 min; MS m/z [M+H]⁺ 215

### Intermediate 36: N-[(3-amino-1-bicyclo[1.1.1]pentanyl)methyl]-N-acetamide ; hydrochloride

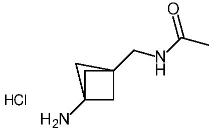

### Int 37: tert-butyl N-[3-(acetamidomethyl)-1-bicyclo[1.1.1]pentanyl]carbamate

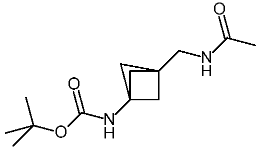

To a solution of tert-butyl N-[3-(aminomethyl)bicyclo[1.1.1]pentan-1-yl]carbamate (300 mg, 1.41 mmol) and DIPEA (1.23 ml, 7.07 mmol) in 10 ml of DCM is dropwise added acetyl chloride (80 µl, 1.13 mmol). The mixture is stirred at RT for 20 hours. The mixture is neutralized with a saturated aqueous solution of NH4Cl, then extracted with DCM. The organic layer is dried over Na₂SO₄, filtered off and concentrated under reduced pressure. After drying overnight under vacuum in presence of P₂O₅, the title compound is obtained as a white solid (300 mg, 83% yield).

1H NMR (400 MHz, DMSO-d6) δ ppm 1.37 (s, 9 H), 1.73 (s, 6 H), 1.80 (s, 3 H), 3.17 (d, J=6 Hz, 2 H), 7.40 (br m, 1 H), 7.70 (m, 1H)

### Int 36: N-[(3-amino-1-bicyclo[1.1.1]pentanyl)methyl]-N-acetamide ; hydrochloride

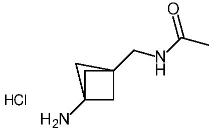

To a solution of tert-butyl N-[3-(acetamidomethyl)-1-bicyclo[1.1.1]pentanyl]carbamate (200 mg, 0.79 mmol) in 5 ml of dioxane is added a solution of hydrochloric acid 4M in dioxane (2 ml, 8 mmol) and the mixture is stirred at room temperature for 20 hours. The mixture is then concentrated under reduced pressure and the residue is triturated in Et2O. After drying overnight under vacuum in presence of P₂O₅, the title compound is obtained as a white solid (160 mg, 98% yield).

1H NMR (400 MHz, DMSO-d6) δ ppm 1.81 (s, 3 H), 1.83 (s, 6 H), 3.23 (d, J=6 Hz, 2 H), 7.86 (br m, 1 H), 8.56 (m, 3H)

Compounds of formula (I) underwent biochemical studies in order to assess their activities using the following *in vitro* methods:

### IL-1β secretion assay

Monocytic THP-1 cells (DSMZ) were maintained in RPMI media (RPMI 1640 Medium (1×) +10% FBS, Eurobio CVFSVFOO-OU). Cells were then plated at 16,000 cells per well in 96-well round bottom cell culture plates (TPP, ref 92097) and maintained with RPMI and 0.1% Bovine Serum Albumin solution. Activation of the NLRP3 inflammasome requires both an NF-kB-dependent priming step and the addition of a NLRP3 activator. The priming step was induced by LPS (10ng/mL, Sigma, ref:L4391) for 3h at 37°C, then compound, in a 1:3.10 serial dilution series in DMSO 1:100, and the activator nigericin (Sigma Aldrich, ref : SML1779) 10µM (final concentration) were added to the cells and co-incubated for 2 h. 50 µL supernatant was removed, and IL-1β levels were monitored using an MSD assay (Electro Chemical Luminescence Immunoassay (ECLI) according to manufacturers' instructions.

### TNF-α secretion assay

Monocytic THP-1 cells (DSMZ) were maintained in RPMI media (RPMI 1640 Medium (1×) +10% FBS, biowest S181 H). Cells were then differentiated at 160,000 cells per well in 96-well flat bottom cell culture plates with RPMI + 0.1% Bovine Serum Albumin solution and 0.5µM phorbol 12-myristate 13-acetate (PMA; Sigma, ref:P8139) overnight at 37°C. Experimental compounds were prepared and added as described above. TNF-α secretion was triggered by the addition of 0.5µg/mL LPS (invivoGen, tlrl-3pelps) and cells were incubated for 24 h. 2.5 µL supernatant was removed, and TNF-α levels were monitored using an MSD assay (mesoscale, K15049) according to manufacturers' instructions.

### Data interpretation

IC₅₀ values were calculated from the plot of percentage of inhibition versus the inhibitor concentration by a logistics fit according to: y = A2 + (A1 - A2)/ (1 + (x/ IC₅₀)^p) where y is the %-inhibition at the inhibitor concentration, x. A1 is the lowest inhibition value, *i.e.,* 0 %, and A2 the maximum inhibition value, *i.e.,* 100 %. The exponent, p, is the Hill coefficient. The curve fitting was conducted with an internally developed software.

### Brain over plasma ratio evaluation

A solution of 3 mg of compound in a generic vehicle (Glycofurol/solutol/Dextrose 5%: 10%/5%/85%, adjusted at pH3), final concentration 0.2 mg/ml, are stirred overnight at ambient temperature whilst protected from light.

Female C57BI6 mice between the ages of 8 -12 weeks are administered with the prepared solution, as single intravenous dose of 1 mg/kg into the tail vein with a dose volume of 5 ml/kg. A total of 3 animal replicates are evaluated for each compound utilizing a terminal sampling approach. After 0.25h post dosing, under anesthesia, 400 µL of blood and the whole brain are collected. Blood sample is centrifuged at 4°C for 10 minutes at 1500 g; brain is weighted, homogenized using deionized water to give a final ratio of (1/2: w/v). Plasma and brain concentrations are measured by an exploratory LC-MS/MS method.

Individual brain over plasma concentration ratio is calculated for each animal, and geometric mean value is reported.

The IC₅₀ values for the compounds disclosed herein were generally less than 10 µM, more particularly less than 1 µM, as indicated in the table below:

| Exp Nr | IL1b IC₅₀ (µM) | TNF-a inhibition @ 10µM | TNF-a inhibition @ 3µM | Ratio brain/plasma |
|---|---|---|---|---|
| 1 | 0.661 | **-17,7** | **-5,1** | |
| 2 | 0.577 | **-51,5** | **3,3** | |
| 3 | 0.335 | **-8,2** | **3,1** | |
| 4 | 0.054 | **7,5** | **21,5** | |
| 5 | 1.230 | **-14,1** | **-18,2** | |
| 6 | 0.158 | | | **7.58** |
| 7 | 1.790 | | | |
| 8 | 0.801 | **-21,8** | **-10,0** | |
| 9 | 9.560 | | | |
| 10 | 0.101 | **-11,9** | **3,0** | **5.84** |
| 11 | 0.045 | **-12,7** | **20,1** | **3.20** |
| 12 | 0.540 | | | |
| 13 | 0.168 | **22,2** | **5,2** | |
| 14 | 0.237 | **-18,8** | **-0,5** | |
| 15 | 2.740 | | | |
| 16 | 1.520 | | | |
| 17 | 0.300 | **31,4** | **11,2** | |
| 18 | 0.236 | **21,6** | **9,6** | **1.56** |
| 19 | 0.225 | **-22,7** | **27,7** | |
| 20 | 0.185 | **-26,0** | **-6,1** | |
| 21 | 0.060 | **13,5** | **11,7** | |
| 22 | 0.400 | **14,5** | **-2,9** | |
| 23 | 0.311 | **3,3** | **1,8** | |
| 24 | 2.890 | **15,5** | **12,0** | |
| 25 | 0.768 | | | |
| 26 | 1.990 | | | |
| 27 | 1.030 | | | |
| 28 | 0.775 | **-45,5** | **34,4** | |
| 29 | 1.140 | | | |
| 30 | 1.200 | | | |
| 31 | 0.030 | **32,5** | **10,5** | |
| 32 | 3.060 | | | |
| 33 | 2.750 | | | |
| 34 | 6.160 | | | |
| 35 | 2.380 | **5,7** | **23,0** | |
| 35 | 0.516 | | | |
| 37 | 0.045 | | | |
| 38 | 1.300 | | | |
| 39 | 2.230 | | | |
| 40 | 0.259 | | | |
| 41 | 0.037 | | | **1.56** |
| 42 | 1.150 | | | |
| 43 | 0.133 | | | **0.42** |
| 44 | 0,278 | | | |
| 45 | 1.530 | | | |
| 46 | 8.200 | | | |
| 47 | 1.030 | | | |
| 48 | 0,0589 | | | |
| 49 | 0,027 | | | |
| 50 | 0,650 | | | |
| 51 | >10.000 | | | |
| 52 | 0,082 | | | |
| 53 | 1,07 | | | |
| 54 | 1,71 | | | |
| 55 | 3,23 | | | |
| 56 | 1,47 | | | |
| 57 | 1,01 | | | |
| 58 | 0,657 | | | |
| 59 | 0,034 | | | **1.15** |
| 60 | 0,130 | | | |
| 61 | 0,159 | | | |
| 62 | 0,850 | | | |
| 63 | 1,66 | | | |
| 64 | 0,109 | | | |
| 65 | 3,80 | | | |
| 66 | 2,07 | | | |
| 67 | 0,446 | | | |
| 68 | 2,28 | | | |
| 69 | 3,43 | | | |
| 70 | 0,148 | | | |
| 71 | 0,07 | | | |
| 72 | >10,00 | | | |
| 73 | >10,00 | | | |
| 74 | 0.344 | | | |
| 75 | 0.157 | | | |
| 76 | 0.978 | | | |
| 77 | 0.078 | | | |
| 78 | 0.326 | | | |
| 79 | 0.029 | | | |
| 80 | 0.322 | | | |
| 81 | >10,00 | | | |
| 82 | 4.50 | | | |
| 83 | 1.88 | | | |
| 84 | 5.95 | | | |
| 85 | >10,00 | | | |
| 86 | >10,00 | | | |
| 87 | >10,00 | | | |
| 88 | >10,00 | | | |

It is therefore apparent that the compounds of formula (I) have an inhibitory activity on NOD-like receptor protein 3 (NLRP3) inflammasome.

The compounds of formula (I) may thus be used as inhibitors of NOD-like receptor protein 3 (NLRP3) inflammasome pathway.

The compounds of formula (I) may thus be used as medicaments, especially medicaments which are inhibitors of NOD-like receptor protein 3 (NLRP3) inflammasome pathway.

Thus, according to another of its aspects, a subject of the present disclosure is medicaments that comprise a compound of formula (I), or an addition salt thereof with a pharmaceutically acceptable acid.

These medicaments are employed therapeutically in the treatment of neurodegenerative diseases, in particular Parkinson's disease, Multiple System Atrophy, Alzheimer's Disease, Multiple Sclerosis, Amyotrophic Lateral Sclerosis or Brain injury.

According to another embodiment, the present disclosure relates to pharmaceutical compositions comprising as active principle, a compound of formula (I). These pharmaceutical compositions contain an effective dose of at least one compound of formula (I), or a pharmaceutically acceptable salt of the said compound.

These pharmaceutical compositions may also contain at least one pharmaceutically acceptable excipient.

The said excipients are chosen, according to the pharmaceutical form and the desired mode of administration, from the usual excipients known to those skilled in the art.

The compounds of formula (I) may be used in the treatment of pathologies involving NOD-like receptor protein 3 (NLRP3) inflammasome pathway.

The present disclosure, according to another of its aspects, also provides the compound of formula (I) for use in a method of treating the pathologies indicated above.

Thus, described is also the compound of formula (I) for use in a method of treating Parkinson's disease, Multiple System Atrophy, Alzheimer's Disease, Multiple Sclerosis, Amyotrophic Lateral Sclerosis or Brain injury, including administering to a subject in need thereof a therapeutically effective amount of at least one compound of formula (I) or a pharmaceutically acceptable salt thereof.

## Claims

1. Compound of the formula (I) 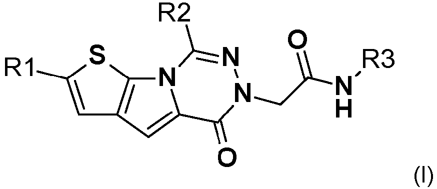 in which:
R1 represents a hydrogen atom, a halogen atom, or a -(C₁-C₂)-alkyl group,
R2 represents a -(C₁-C₃)alkyl group being unsubstituted or substituted with 1 to 3 substituents independently selected from a hydroxy group, or a -(C₁-C₃)-alkoxy group; or represents a -(C₃-C₄)cycloalkyl group,
**R3** is selected from
➢ a -(C₅-C₈)bicycloalkyl group being unsubstituted or substituted with one -(C₁-C₃)alkyl group being unsubstituted or substituted with one or two -NH(CO)Me groups,
➢ a -(C₄-C₇)heterocycloalkyl group with nitrogen as heteroatom, being unsubstituted or substituted with one or more substituents independently selected from
• a halogen atom,
• an oxo group,
• -(C₁-C₄)-alkyl group, being unsubstituted or substituted with 1 to 3 substituents independently selected from a hydroxy group, -(C₁-C₂)-alkyl group, a halogen atom, a -(C₁-C₂)-alkoxy group, a nitrile group, a -C(O)O(C₁-C₃) group, or a - (C₃-C₈)-cycloalkyl group being unsubstituted or substituted by one or more halogen group,
• -(C₃-C₆)-cycloalkyl group, being unsubstituted or substituted by one or more (C₁-C₂) alkyl group,
• -(CO)-(C₁-C₂)-alkyl group,
• a heterocycloalkyl group, or
• a heteroaryl group being unsubstituted or substituted with one or more -(C₁-C₄)alkyl group,
➢ a -hetero(C₆-C₉)bicycloalkyl group with nitrogen as heteroatom, optionally substituted with one or more substituents independently selected from
• a -(C₃-C₄)cycloalkyl group, or
• a -(C₁-C₃)alkyl group,
or
➢ a mono, or bicycloheteroaryl group, being unsubstituted or substituted with one or more substituents independently selected from
➢ a -(C₁-C₄)-alkyl group,
➢ a -(C₁-C₄)-alkoxy group, or
➢ a halogen atom,
or a pharmaceutically acceptable salt thereof.

2. Compound according to claim 1 wherein:
**R1** represents a hydrogen atom, a halogen atom chosen between bromine and chlorine atom, or a methyl group,
**R2** represents a -(C₁-C₃)alkyl group chosen between methyl, ethyl, propyl and isopropyl group being unsubstituted or substituted with 1 substituent independently selected from a hydroxy group, or a methoxy group; or represents a cyclopropyl group,
**R3** is selected from
➢ a bicyclopentyl group being substituted with one -(C₁-C₃)alkyl group being unsubstituted or substituted with one -NH(CO)Me group,
➢ a -(C₄-C₆) heterocycloalkyl group with nitrogen as heteroatom, being unsubstituted or substituted with one or more substituents independently selected from
• a fluorine atom,
• an oxo group,
• a -(C₁-C₄)-alkyl group, being unsubstituted or substituted with 1 to 3 substituents independently selected from a hydroxy group, a methyl group, a fluorine atom, a methoxy group, a nitrile group, a -C(O)O(C₁-C₂) group, or a -(C₃-C₆)-cycloalkyl group being unsubstituted or substituted by one fluorine atom,
• a -(C₃-C₅)-cycloalkyl group, being unsubstituted or substituted by one or more methyl group,
• a -(CO)-methyl group,
• a heterocyclopropyl group, or
• an imidazole group, being unsubstituted or substituted with one methyl group,
➢ a -hetero(C₆-C₉)bicycloalkyl group with nitrogen as heteroatom, optionally substituted with one or more substituents independently selected from
• a -(C₃-C₄) cycloalkyl group, or
• a methyl group,
or
➢ a mono, or bicycloheteroaryl group, being unsubstituted or substituted with one or two substituents independently selected from
➢ a methyl group,
➢ a methoxy group, or
➢ a fluorine atom,
or a pharmaceutically acceptable salt thereof.

3. Compound according to any one of claims 1 or 2 wherein
**R1** represents a hydrogen atom, or a halogen atom chosen between a bromine and a chlorine atom,
**R2** represents a -(C₁-C₃)alkyl group being unsubstituted or substituted with 1 to 3 substituents independently selected from a hydroxy group, or represents a -(C₃-C₄)cycloalkyl group,
**R3** is selected from
➢ -(C₅-C₈)bicycloalkyl group being unsubstituted or substituted with one -(C₁-C₃)alkyl group being unsubstituted or substituted with one or two -NH(CO)Me groups,
➢ a -(C₄-C₇)heterocycloalkyl group with nitrogen as heteroatom, being unsubstituted or substituted with one or more substituents independently selected from
• -(C₁-C₄)-alkyl group being unsubstituted or substituted with 1 to 3 substituents independently selected from a hydroxy group, a methyl group, a fluorine atom,
a methoxy group, a nitrile group, a -C(O)O(C₁-C₃) group, or a -(C₃-C₅)-cycloalkyl group being unsubstituted or substituted by one or more fluorine atom,
• -(C₃-C₆)-cycloalkyl group, being unsubstituted or substituted by one or more - (C₁-C₂)-alkyl group, or
• a heterocycloalkyl group,
➢ -hetero(C₆-C₈) bicycloalkyl group with nitrogen as heteroatom, optionally substituted with one or more -(C₃-C₄)cycloalkyl group,
or
➢ a mono, or bicycloheteroaryl group, being unsubstituted or substituted with one fluorine atom,
or a pharmaceutically acceptable salt thereof.

4. Compound according to any one of claims 1 to 3 wherein R1 represents a hydrogen atom or a pharmaceutically acceptable salt thereof.

5. Compound according to any one of claims 1 to 3 wherein R1 represents a bromine atom or a pharmaceutically acceptable salt thereof.

6. Compound according to any one of claims 1 to 3 wherein R1 represents a chlorine atom or a pharmaceutically acceptable salt thereof.

7. Compound according to any one of claims 1 to 6 wherein R2 represents a -iPr group or a pharmaceutically acceptable salt thereof.

8. Compound according to any one of claims 1 to 6 wherein R2 represents a -cyclopropyl group or a pharmaceutically acceptable salt thereof.

9. Compound according to any one of claims 1 to 8 wherein R3 represents a -(C₄-C₇)heterocycloalkyl group with nitrogen as heteroatom, being unsubstituted or substituted with one or more substituents independently selected from
• -(C₁-C₄)-alkyl group, being unsubstituted or substituted with 1 to 3 substituents independently selected from a hydroxy group, -(C₁-C₂)-alkyl group, a fluorine atom, -(C₁-C₂)-alkoxy group, a nitrile group, a -C(O)O(C₁-C₃) group, or a -(C₃-C₅)-cycloalkyl group being unsubstituted or substituted by one or more fluorine atom,
• -(C₃-C₆)-cycloalkyl group, being unsubstituted or substituted by one or more (C₁-C₂) alkyl group, or
• a heterocyclopropyl group.
or a pharmaceutically acceptable salt thereof.

10. Compounds of formula (I) according to claim 1 which are selected from the following list:
1 (R)-2-(8-isopropyl-5-oxothieno[3',2':4,5]pyrrolo[1,2-d][1,2,4]triazin-6(5H)-yl)-N-(1-methylpiperidin-3-yl)acetamide
2 2-(8-isopropyl-5-oxothieno[3',2':4,5]pyrrolo[1,2-d][1,2,4]triazin-6(5H)-yl)-N-(thiazol-5-yl) acetamide
3 N-((R)-1-((S)-2-hydroxypropyl)piperidin-3-yl)-2-(8-isopropyl-5-oxothieno[3',2':4,5] pyrrolo[1,2-d][1,2,4]triazin-6(5H)-yl)acetamide
4 (R)-N-(1-cyclopropylpiperidin-3-yl)-2-(8-isopropyl-5-oxothieno[3',2':4,5]pyrrolo[1,2-d] [1,2,4]triazin-6(5H)-yl)acetamide
5 (R)-2-(2-chloro-8-isopropyl-5-oxothieno[3',2':4,5]pyrrolo[1,2-d][1,2,4] triazin-6(5H)-yl)-N-(1-methylpiperidin-3-yl)acetamide 2,2,2-trifluoroacetate
6 (R)-2-(2-chloro-8-isopropyl-5-oxothieno[3',2':4,5]pyrrolo[1,2-d][1,2,4]triazin-6(5H)-yl)-N-(1-cyclopropylpiperidin-3-yl)acetamide 2,2,2-trifluoroacetate
7 2-(2-chloro-8-isopropyl-5-oxothieno[3',2':4,5]pyrrolo[1,2-d][1,2,4]triazin-6(5H)-yl)-N-(thiazol-5-yl)acetamide
8 (R)-2-(2-bromo-8-isopropyl-5-oxothieno[3',2':4,5]pyrrolo[1,2-d][1,2,4] triazin-6(5H)-yl)-N-(1-methylpiperidin-3-yl)acetamide
9 (R)-2-(8-isopropyl-2-methyl-5-oxothieno[3',2':4,5]pyrrolo[1,2-d][1,2,4]triazin-6(5H)-yl)-N-(1-methylpiperidin-3-yl)acetamide 2,2,2-trifluoroacetate
10 (R)-2-(2-chloro-8-isopropyl-5-oxothieno[3',2':4,5]pyrrolo[1,2-d][1,2,4] triazin-6(5H)-yl)-N-(1-cyclobutylpiperidin-3-yl)acetamide formate
11 (R)-N-(1-cyclobutylpiperidin-3-yl)-2-(8-isopropyl-5-oxothieno[3',2':4,5]pyrrolo[1,2-d] [1,2,4]triazin-6(5H)-yl)acetamide formate
12 N-(benzo[d]thiazol-6-yl)-2-(8-isopropyl-5-oxothieno[3',2':4,5]pyrrolo[1,2-d][1,2,4]triazin-6(5H)-yl)acetamide
13 (R)-2-(2-chloro-8-cyclopropyl-5-oxothieno[3',2':4,5]pyrrolo[1,2-d][1,2,4]triazin-6(5H)-yl)-N-(1-cyclopropylpiperidin-3-yl)acetamide
14 (R)-N-(1-isobutylpiperidin-3-yl)-2-(8-isopropyl-5-oxothieno[3',2':4,5]pyrrolo[1,2-d][1,2,4] triazin-6(5H)-yl)acetamide
15 N-(benzo[d]thiazol-5-yl)-2-(8-isopropyl-5-oxothieno[3',2':4,5]pyrrolo[1,2-d][1,2,4]triazin-6(5H)-yl)acetamide
16 2-(8-isopropyl-5-oxothieno[3',2':4,5]pyrrolo[1,2-d][1,2,4]triazin-6(5H)-yl)-N-(6-methoxypyridin-3-yl)acetamide
17 (R)-N-(1-(cyclopentylmethyl)piperidin-3-yl)-2-(8-isopropyl-5-oxothieno[3',2':4,5]pyrrolo [1,2-d][1,2,4]triazin-6(5H)-yl)acetamide
18 N-((3R)-1-((2,2-difluorocyclopropyl)methyl)piperidin-3-yl)-2-(8-isopropyl-5-oxothieno [3',2':4,5]pyrrolo[1,2-d][1,2,4]triazin-6(5H)-yl)acetamide compound with formaldehyde
19 (R)-N-(1-(2-hydroxyethyl)piperidin-3-yl)-2-(8-isopropyl-5-oxothieno [3',2':4,5]pyrrolo[1,2-d][1,2,4]triazin-6(5H)-yl)acetamide compound with formaldehyde
20 (R)-N-(1-cyclobutylpiperidin-3-yl)-2-(8-cyclopropyl-5-oxothieno[3',2':4,5]pyrrolo[1,2-d] [1,2,4]triazin-6(5H)-yl)acetamide
21 (R)-2-(8-cyclopropyl-5-oxothieno[3',2':4,5]pyrrolo[1,2-d][1,2,4]triazin-6(5H)-yl)-N-(1-cyclopropylpiperidin-3-yl)acetamide
22 2-(8-cyclopropyl-5-oxothieno[3',2':4,5]pyrrolo[1,2-d][1,2,4]triazin-6(5H)-yl)-N-((R)-1-((S)-2-hydroxypropyl)piperidin-3-yl)acetamide
23 (R)-N-(1-(2-hydroxy-2-methylpropyl)piperidin-3-yl)-2-(8-isopropyl-5-oxothieno[3',2':4,5] pyrrolo[1,2-d][1,2,4]triazin-6(5H)-yl)acetamide formate
24 (R)-2-(8-cyclopropyl-5-oxothieno[3',2':4,5]pyrrolo[1,2-d][1,2,4]triazin-6(5H)-yl)-N-(1-methylpiperidin-3-yl)acetamide formate
25 N-((R)-1-cyclobutylpiperidin-3-yl)-2-(8-isopropyl-5-oxothieno[3',2':4,5]pyrrolo[1,2-d] [1,2,4]triazin-6(5H)-yl)propanamide formate
26 N-((R)-1-cyclopropylpiperidin-3-yl)-2-(8-isopropyl-5-oxothieno[3',2':4,5]pyrrolo[1,2-d] [1,2,4]triazin-6(5H)-yl)propanamide formate
27 2-(8-isopropyl-5-oxothieno[3',2':4,5]pyrrolo[1,2-d][1,2,4]triazin-6(5H)-yl)-N-(1-methyl-1H-indazol-6-yl)acetamide formate
28 (R)-N-(1-(cyclopropylmethyl)piperidin-3-yl)-2-(8-isopropyl-5-oxothieno[3',2':4,5]pyrrolo [1,2-d][1,2,4]triazin-6(5H)-yl)acetamide formate
29 (R)-2-(2-chloro-8-(methoxymethyl)-5-oxothieno[3',2':4,5]pyrrolo[1,2-d][1,2,4]triazin-6(5H)-yl)-N-(1-cyclobutylpiperidin-3-yl)acetamide formate
30 (R)-2-(2-chloro-8-(methoxymethyl)-5-oxothieno[3',2':4,5]pyrrolo[1,2-d][1,2,4]triazin-6(5H)-yl)-N-(1-cyclopropylpiperidin-3-yl)acetamide formate
31 (R)-2-(2-bromo-8-isopropyl-5-oxothieno[3',2':4,5]pyrrolo[1,2-d][1,2,4]triazin-6(5H)-yl)-N-(1-cyclobutylpiperidin-3-yl)acetamide 2,2,2-trifluoroacetate
32 (R)-N-(1-cyclobutylpiperidin-3-yl)-2-(8-(methoxymethyl)-5-oxothieno[3',2':4,5]pyrrolo [1,2-d][1,2,4]triazin-6(5H)-yl)acetamide
33 (R)-N-(1-cyclopropylpiperidin-3-yl)-2-(8-(methoxymethyl)-5-oxothieno[3',2':4,5]pyrrolo [1,2-d][1,2,4]triazin-6(5H)-yl)acetamide 2,2,2-trifluoroacetate
34 2-(2-chloro-8-isopropyl-5-oxothieno[3',2':4,5]pyrrolo[1,2-d][1,2,4]triazin-6(5H)-yl)-N-(5,5-difluoro-1-methylpiperidin-3-yl)acetamide 2,2,2-trifluoroacetate
35 N-(5,5-difluoro-1-methylpiperidin-3-yl)-2-(8-isopropyl-5-oxothieno[3',2':4,5]pyrrolo[1,2-d] [1,2,4]triazin-6(5H)-yl)acetamide
36 (R)-N-(1-(2-fluoroethyl)piperidin-3-yl)-2-(8-isopropyl-5-oxothieno[3',2':4,5]pyrrolo[1,2-d] [1,2,4]triazin-6(5H)-yl)acetamide formate
37 N-(1-(tert-butyl)piperidin-3-yl)-2-(8-isopropyl-5-oxothieno[3',2':4,5]pyrrolo[1,2-d][1,2,4] triazin-6(5H)-yl)acetamide formate
38 2-(8-isopropyl-5-oxothieno[3',2':4,5]pyrrolo[1,2-d][1,2,4]triazin-6(5H)-yl)-N-(1-(2,2,2-trifluoroethyl)piperidin-3-yl)acetamide 2,2,2-trifluoroacetate
39 2-(8-isopropyl-5-oxothieno[3',2':4,5]pyrrolo[1,2-d][1,2,4]triazin-6(5H)-yl)-N-(quinuclidin-3-yl)acetamide
40 (R)-2-(8-isopropyl-5-oxothieno[3',2':4,5]pyrrolo[1,2-d][1,2,4]triazin-6(5H)-yl)-N-(1-(oxetan-3-yl)piperidin-3-yl)acetamide
41 (R)-N-(1-(3-fluoropropyl)piperidin-3-yl)-2-(8-isopropyl-5-oxothieno [3',2':4,5] pyrrolo[1,2-d][1,2,4]triazin-6(5H)-yl)acetamide formate
42 (R)-N-(1-acetylpiperidin-3-yl)-2-(8-isopropyl-5-oxothieno[3',2':4,5]pyrrolo[1,2-d][1,2,4] triazin-6(5H)-yl)acetamide
43 (R)-N-(1-((3,3-difluorocyclobutyl)methyl)piperidin-3-yl)-2-(8-isopropyl-5-oxothieno[3',2':4,5]pyrrolo[1,2-d][1,2,4]triazin-6(5H)-yl)acetamide formate
44 N-((R)-1-cyclopropylpiperidin-3-yl)-2-(8-(1-hydroxyethyl)-5-oxothieno[3',2':4,5]pyrrolo [1,2-d][1,2,4]triazin-6(5H)-yl)acetamide
45 (R)-2-(8-isopropyl-5-oxothieno[3',2':4,5]pyrrolo[1,2-d][1,2,4]triazin-6(5H)-yl)-N-(1-(1-methyl-1H-imidazol-2-yl)piperidin-3-yl)acetamide
46 2-(8-isopropyl-5-oxothieno[3',2':4,5]pyrrolo[1,2-d][1,2,4]triazin-6(5H)-yl)-N-(1-methyl-2-oxopiperidin-4-yl)acetamide
47 2-(8-isopropyl-5-oxothieno[3',2':4,5]pyrrolo[1,2-d][1,2,4]triazin-6(5H)-yl)-N-(1-methyl-6-oxopiperidin-3-yl)acetamide
48 2-(12-isopropyl-9-oxo-3-thia-1,10,11-triazatricyclo[6.4.0.02,6]dodeca-2(6),4,7,11-tetraen-10-yl)-N-[(3R)-1-isopropyl-3-piperidyl]acetamide
49 N-[(3R)-1-cyclopentyl-3-piperidyl]-2-(12-isopropyl-9-oxo-3-thia-1,10,11-triazatricyclo [6.4.0.02,6]dodeca-2(6),4,7,11-tetraen-10-yl)acetamide
50 N-((R)-1-cyclobutylpiperidin-3-yl)-2-(8-(1-hydroxyethyl)-5-oxothieno[3',2':4,5]pyrrolo [1,2-d][1,2,4]triazin-6(5H)-yl)acetamide
51 N-((R)-1-cyclopropylpiperidin-3-yl)-2-(8-(1-hydroxyethyl)-5-oxothieno[3',2':4,5]pyrrolo [1,2d][1,2,4]triazin-6(5H)-yl)acetamide
52 (R)-N-(1-cyclopropylpiperidin-3-yl)-2-(8-(2-hydroxypropan-2-yl)-5-oxothieno [3',2':4,5]pyrrolo[1,2-d][1,2,4]triazin-6(5H)-yl)acetamide
53 2-(12-isopropyl-9-oxo-3-thia-1,10,11-triazatricyclo[6.4.0.02,6]dodeca-2(6),4,7,11-tetraen-10-yl)-N-(2-methylpyrazol-3-yl)acetamide
54 2-(12-isopropyl-9-oxo-3-thia-1,10,11-triazatricyclo[6.4.0.02,6]dodeca-2(6),4,7,11-tetraen-10-yl)-N-(1-methylpyrazol-3-yl)acetamide
55 N-[(1-tert-butyl-5-oxo-pyrrolidin-3-yl)methyl]-2-(12-isopropyl-9-oxo-3-thia-1,10,11-triazatricyclo[6.4.0.02,6]dodeca-2(6),4,7,11-tetraen-10-yl)acetamide
56 2-(12-isopropyl-9-oxo-3-thia-1,10,11-triazatricyclo[6.4.0.02,6]dodeca-2(6),4,7,11-tetraen-10-yl)-N-(3-pyridyl)acetamide
57 2-(12-isopropyl-9-oxo-3-thia-1,10,11-triazatricyclo[6.4.0.02,6]dodeca-2(6),4,7,11-tetraen-10-yl)-N-[rac-(3R)-1-(2,2-dimethylcyclobutyl)-3-piperidyl]acetamide
58 2-(12-isopropyl-9-oxo-3-thia-1,10,11-triazatricyclo[6.4.0.02,6]dodeca-2(6),4,7,11-tetraen-10-yl)-N-(2-pyridyl)acetamide
59 2-(4-bromo-12-isopropyl-9-oxo-3-thia-1,10,11-triazatricyclo[6.4.0.02,6]dodeca-2(6),4,7,11-tetraen-10-yl)-N-(1-tert-butyl-3-piperidyl)acetamide
60 N-[(3R)-1-cyclopropyl-3-piperidyl]-2-(12-ethyl-9-oxo-3-thia-1,10,11-triazatricyclo [6.4.0.02,6]dodeca-2(6),4,7,11-tetraen-10-yl)acetamide
61 N-[(3R)-1-cyclopropylazepan-3-yl]-2-(12-isopropyl-9-oxo-3-thia-1,10,11-triazatricyclo [6.4.0.02,6]dodeca-2(6),4,7,11-tetraen-10-yl)acetamide
62 2-(12-isopropyl-9-oxo-3-thia-1,10,11-triazatricyclo[6.4.0.02,6]dodeca-2(6),4,7,11-tetraen-10-yl)-N-[(3R)-1-methylazepan-3-yl]acetamide
63 N-(1-cyclopropyl-5,5-difluoropiperidin-3-yl)-2-(8-isopropyl-5-oxothieno[3',2':4,5]pyrrolo [1,2-d][1,2,4]triazin-6(5H)-yl)acetamide 2,2,2-trifluoroacetate
64 2-(8-isopropyl-5-oxothieno[3',2':4,5]pyrrolo[1,2-d][1,2,4]triazin-6(5H)-yl)-N-((3R)-1-(1-methoxypropan-2-yl)piperidin-3-yl)acetamide
65 2-(8-isopropyl-5-oxothieno[3',2':4,5]pyrrolo[1,2-d][1,2,4]triazin-6(5H)-yl)-N-(2-methoxypyridin-4-yl)acetamide
66 2-(12-isopropyl-9-oxo-3-thia-1,10,11-triazatricyclo[6.4.0.02,6]dodeca-2(6),4,7,11-tetraen-10-yl)-N-pyrimidin-4-yl-acetamide
67 2-(12-isopropyl-9-oxo-3-thia-1,10,11-triazatricyclo[6.4.0.02,6]dodeca-2(6),4,7,11-tetraen-10-yl)-N-(4-pyridyl)acetamide
68 2-(12-isopropyl-9-oxo-3-thia-1,10,11-triazatricyclo[6.4.0.02,6]dodeca-2(6),4,7,11-tetraen-10-yl)-N-(6-methyl-3-pyridyl)acetamide
69 2-(12-ethyl-9-oxo-3-thia-1,10,11-triazatricyclo[6.4.0.02,6]dodeca-2(6),4,7,11-tetraen-10-yl)-N-[(3R)-1-[(2S)-2-hydroxypropyl]-3-piperidyl]acetamide
70 N-(5-fluoropyrimidin-4-yl)-2-(12-isopropyl-9-oxo-3-thia-1,10,11-triazatricyclo[6.4.0.02,6] dodeca-2(6),4,7,11-tetraen-10-yl)acetamide
71 2-(12-isopropyl-9-oxo-3-thia-1,10,11-triazatricyclo[6.4.0.02,6]dodeca-2(6),4,7,11-tetraen-10-yl)-N-[(3R)-1-(1-methylcyclopentyl)-3-piperidyl]acetamide
72 N-[(3R)-1-cyclopropylpiperidin-3-yl]-2-(9-oxo-12-propyl-3-thia-1,10,11-triazatricyclo [6.4.0.02,6]dodeca-2(6),4,7,11-tetraen-10-yl)acetamide
73 2-(12-ethyl-9-oxo-3-thia-1,10,11-triazatricyclo[6.4.0.02,6]dodeca-2(6),4,7,11-tetraen-10-yl)-N-[(3R)-1-methyl-3-piperidyl]acetamide
74N-[3-(acetamidomethyl)bicyclo[1.1.1]pentan-1-yl]-2-(8-isopropyl-5-oxothieno[3',2':4,5] pyrrolo[1,2-d][1,2,4]triazin-6(5H)-yl)acetamide
75 N-[(3R)-1-(cyanomethyl)piperidin-3-yl]-2-(8-isopropyl-5-oxothieno[3',2':4,5]pyrrolo[1,2-d][1,2,4]triazin-6(5H)-yl)acetamide
76N-[(3R)-1-methylpyrrolidin-3-yl]-2-(8-isopropyl-5-oxothieno[3',2':4,5]pyrrolo[1,2-d][1,2,4] triazin-6(5H)-yl)acetamide
77 N-[(3R)-1-(2-cyanoethyl)piperidin-3-yl]-2-(8-isopropyl-5-oxothieno[3',2':4,5]pyrrolo[1,2-d][1,2,4]triazin-6(5H)-yl)acetamide
78 N-[(3R)-1-[(2R)-2-hydroxypropyl]piperidin-3-yl]-2-(8-isopropyl-5-oxothieno[3',2':4,5]pyrrolo [1,2-d][1,2,4]triazin-6(5H)-yl)acetamide
79N-[(3R)-1-(1-hydroxy-2-methylpropan-2-yl)piperidin-3-yl]-2-(8-isopropyl-5-oxothieno [3',2':4,5] pyrrolo[1,2-d][1,2,4]triazin-6(5H)-yl)acetamide
80 ethyl 2-methyl-2-[3-[[2-(8-isopropyl-5-oxothieno[3',2':4,5]pyrrolo[1,2-d][1,2,4]triazin-6(5H)-yl)acetyl]amino]piperidin-1-yl]propanoate
81 N-((R)-1-cyclobutylpiperidin-3-yl)-2-(8-(1-hydroxyethyl)-5-oxothieno[3',2':4,5]pyrrolo[1,2-d][1,2,4]triazin-6(5H)-yl)acetamide
82 N-[(1R,4R)-2-methyl-2-azabicyclo[2.2.1]heptan-6-yl]-2-(8-isopropyl-5-oxothieno[3',2':4,5] pyrrolo[1,2-d][1,2,4]triazin-6(5H)-yl)acetamide
83 N-(2-methyl-2-azaspiro[3.3]heptan-6-yl)-2-(8-isopropyl-5-oxothieno[3',2':4,5]pyrrolo[1,2-d][1,2,4]triazin-6(5H)-yl)acetamide
84 2-(8-isopropyl-5-oxothieno[3',2':4,5]pyrrolo[1,2-d][1,2,4]triazin-6(5H)-yl)-N-pyrimidin-5-ylacetamide
85 2-(12-isopropyl-9-oxo-3-thia-1,10,11-triazatricyclo[6.4.0.02,6]dodeca-2(6),4,7,11-tetraen-10-yl)-N-(2-oxo-4-piperidyl)acetamide
86 N-(1-cyclopropylpyrrolidin-3-yl)-2-(12-isopropyl-9-oxo-3-thia-1,10,11-triazatricyclo [6.4.0.02,6]dodeca-2(6),4,7,11-tetraen-10-yl)acetamide
87 2-(12-isopropyl-9-oxo-3-thia-1,10,11-triazatricyclo[6.4.0.02,6]dodeca-2(6),4,7,11-tetraen-10-yl)-N-[rac-(3R)-1-tert-butylpyrrolidin-3-yl]acetamide
88 N-[(1S,4R)-2-azabicyclo[2.2.1]heptan-6-yl]-2-(12-isopropyl-9-oxo-3-thia-1,10,11-triazatricyclo[6.4.0.02,6]dodeca-2(6),4,7,11-tetraen-10-yl)acetamidehydrochloride.

11. Medicament, **characterized in that** it comprises a compound of formula (I) according to any one of claims 1 to 10, or pharmaceutically acceptable salt thereof.

12. Pharmaceutical composition, **characterized in that** it comprises a compound of formula (I) according **to** any one of claims 1 to 10.

13. Compound of formula (I) according to any one of claims 1 to 10, for use in the treatment of Parkinson's disease, Multiple System Atrophy, Alzheimer's disease, frontotemporal Dementia, Multiple Sclerosis, Amyotrophic Lateral Sclerosis or brain injury.

## Patentansprüche

1. Verbindung der Formel (I) 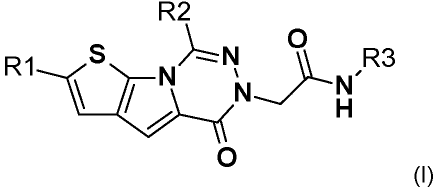 wobei:
R1 für ein Wasserstoffatom, ein Halogenatom oder eine - (C₁-C₂) -Alkylgruppe steht,
R2 für eine -(C₁-C₃)-Alkylgruppe steht, die unsubstituiert oder durch 1 bis 3 Substituenten substituiert ist, die unabhängig aus einer Hydroxygruppe oder einer -(C₁-C₃) -Alkoxygruppe ausgewählt sind; oder
für eine -(C₃-C₄)-Cycloalkylgruppe steht,
R3 ausgewählt ist aus:
➢ einer - (C₅-C₈) -Bicycloalkylgruppe, die unsubstituiert oder durch eine -(C₁-C₃)-Alkylgruppe, die unsubstituiert oder durch eine oder zwei -NH(CO)Me-Gruppen substituiert ist, substituiert ist,
➢ einer -(C₄-C₇)-Heterocycloalkylgruppe mit Stickstoff als Heteroatom, die unsubstituiert ist oder durch einen oder mehrere Substituenten substituiert ist, die unabhängig ausgewählt sind aus
• einem Halogenatom,
• einer Oxogruppe,
• einer -(C₁-C₄)-Alkylgruppe, die unsubstituiert oder durch 1 bis 3 Substituenten, die unabhängig aus einer Hydroxygruppe, einer -(C₁-C₂)-Alkylgruppe, einem Halogenatom, einer - (C₁-C₂) -Alkoxygruppe, einer Nitrilgruppe, einer -C(O)O(C₁-C₃)-Gruppe oder einer -(C₃-C₈)-Cycloalkylgruppe, die unsubstituiert oder durch eine oder mehrere Halogengruppen substituiert ist, ausgewählt sind, substituiert ist,
• einer -(C₃-C₆)-Cycloalkylgruppe, die unsubstituiert oder durch eine oder mehrere (C₁-C₂) -Alkylgruppen substituiert ist,
• einer - (CO) - (C₁-C₂) -Alkylgruppe,
• einer Heterocycloalkylgruppe oder
• einer Heteroarylgruppe, die unsubstituiert oder durch ein oder mehrere - (C₁-C₄) -Alkylgruppen substituiert ist,
➢ einer -Hetero- (C₆-C₉) -bicycloalkylgruppe mit Stickstoff als Heteroatom, die gegebenenfalls durch einen oder mehrere Substituenten substituiert ist, die unabhängig ausgewählt sind aus
• einer -(C₃-C₄)-Cycloalkylgruppe oder
• einer -(C₁-C₃)-Alkylgruppe,
oder
➢ einer Mono- oder Bicycloheteroarylgruppe, die unsubstituiert oder durch einen oder mehrere Substituenten substituiert ist, die unabhängig ausgewählt sind aus
➢ einer - (C₁-C₄) -Alkylgruppe,
➢ einer - (C₁-C₄) -Alkoxygruppe oder
➢ einem Halogenatom,
oder ein pharmazeutisch akzeptables Salz davon.

2. Verbindung nach Anspruch 1, wobei:
R1 für ein Wasserstoffatom, ein Halogenatom, das aus einem Brom- und einem Chloratom ausgewählt ist, oder eine Methylgruppe steht,
R2 für eine -(C₁-C₃)-Alkylgruppe steht, die aus einer Methyl-, Ethyl-, Propyl- und Isopropylgruppe, die unsubstituiert oder durch 1 Substituenten, der unabhängig aus einer Hydroxygruppe oder einer Methoxygruppe ausgewählt ist, substituiert ist, ausgewählt ist; oder für eine Cyclopropylgruppe steht,
R3 ausgewählt ist aus:
➢ einer Bicyclopentylgruppe, die durch eine -(C₁-C₃)-Alkylgruppe, die unsubstituiert oder durch eine - NH(CO)Me-Gruppe substituiert ist, substituiert ist,
➢ einer -(C₄-C₆)-Heterocycloalkylgruppe mit Stickstoff als Heteroatom, die unsubstituiert ist oder durch einen oder mehrere Substituenten substituiert ist, die unabhängig ausgewählt sind aus
• einem Fluoratom,
• einer Oxogruppe,
• einer -(C₁-C₄)-Alkylgruppe, die unsubstituiert oder durch 1 bis 3 Substituenten, die unabhängig aus einer Hydroxygruppe, einer Methylgruppe, einem Fluoratom, einer Methoxygruppe, einer Nitrilgruppe, einer -C(O)O(C₁-C₂)-Gruppe oder einer -(C₃-C₆)-Cycloalkylgruppe, die unsubstituiert oder durch ein Fluoratom substituiert ist, ausgewählt sind, substituiert ist,
• einer -(C₃-C₅)-Cycloalkylgruppe, die unsubstituiert oder durch eine oder mehrere Methylgruppen substituiert ist,
• einer -(CO)-Methylgruppe,
• einer Heterocyclopropylgruppe oder
• einer Imidazolgruppe, die unsubstituiert oder durch eine Methylgruppe substituiert ist,
➢ einer -Hetero- (C₆-C₉) -bicycloalkylgruppe mit Stickstoff als Heteroatom, die gegebenenfalls durch einen oder mehrere Substituenten substituiert ist, die unabhängig ausgewählt sind aus
• einer -(C₃-C₄)-Cycloalkylgruppe oder
• einer Methylgruppe,
oder
➢ einer Mono- oder Bicycloheteroarylgruppe, die unsubstituiert oder durch einen oder zwei Substituenten substituiert ist, die unabhängig ausgewählt sind aus
➢ einer Methylgruppe,
➢ einer Methoxygruppe oder
➢ einem Fluoratom,
oder ein pharmazeutisch akzeptables Salz davon.

3. Verbindung nach Anspruch 1 oder 2, wobei
R1 für ein Wasserstoffatom oder ein Halogenatom, das aus einem Brom- und einem Chloratom ausgewählt ist, steht,
R2 für eine -(C₁-C₃)-Alkylgruppe steht, die unsubstituiert oder durch 1 bis 3 Substituenten substituiert ist, die unabhängig aus einer Hydroxygruppe ausgewählt sind, oder für eine -(C₃-C₄)-Cycloalkylgruppe steht,
R3 ausgewählt ist aus:
➢ einer - (C₅-C₈) -Bicycloalkylgruppe, die unsubstituiert oder durch eine -(C₁-C₃)-Alkylgruppe, die unsubstituiert oder durch eine oder zwei -NH(CO)Me-Gruppen substituiert ist, substituiert ist,
➢ einer -(C₄-C₇)-Heterocycloalkylgruppe mit Stickstoff als Heteroatom, die unsubstituiert ist oder durch einen oder mehrere Substituenten substituiert ist, die unabhängig ausgewählt sind aus
• einer -(C₁-C₄)-Alkylgruppe, die unsubstituiert oder durch 1 bis 3 Substituenten, die unabhängig aus einer Hydroxygruppe, einer Methylgruppe, einem Fluoratom, einer Methoxygruppe, einer Nitrilgruppe, einer -C(O)O(C₁-C₃)-Gruppe oder einer -(C₃-C₅)-Cycloalkylgruppe, die unsubstituiert oder durch ein oder mehrere Fluoratome substituiert ist, ausgewählt sind, substituiert ist,
• einer -(C₃-C₆)-Cycloalkylgruppe, die unsubstituiert oder durch eine oder mehrere -(C₁-C₂) -Alkylgruppen substituiert ist, oder
• einer Heterocycloalkylgruppe,
➢ einer -Hetero-(C₆-C₈)-bicycloalkylgruppe mit Stickstoff als Heteroatom, die gegebenenfalls durch eine oder mehrere -(C₃-C₄)-Cycloalkylgruppen substituiert ist, oder
➢ einer Mono- oder Bicycloheteroarylgruppe, die unsubstituiert oder durch ein Fluoratom substituiert ist, oder ein pharmazeutisch akzeptables Salz davon.

4. Verbindung nach einem der Ansprüche 1 bis 3, wobei R1 für ein Wasserstoffatom steht, oder ein pharmazeutisch akzeptables Salz davon.

5. Verbindung nach einem der Ansprüche 1 bis 3, wobei R1 für ein Bromatom steht, oder ein pharmazeutisch akzeptables Salz davon.

6. Verbindung nach einem der Ansprüche 1 bis 3, wobei R1 für ein Chloratom steht, oder ein pharmazeutisch akzeptables Salz davon.

7. Verbindung nach einem der Ansprüche 1 bis 6, wobei R2 für eine -iPr-Gruppe steht, oder ein pharmazeutisch akzeptables Salz davon.

8. Verbindung nach einem der Ansprüche 1 bis 6, wobei R2 für eine -Cyclopropylgruppe steht, oder ein pharmazeutisch akzeptables Salz davon.

9. Verbindung nach einem der Ansprüche 1 bis 8, wobei R3 für eine -(C₄-C₇)-Heterocycloalkylgruppe mit Stickstoff als Heteroatom steht, die unsubstituiert ist oder durch einen oder mehrere Substituenten substituiert ist, die unabhängig ausgewählt sind aus
• einer -(C₁-C₄)-Alkylgruppe, die unsubstituiert oder durch 1 bis 3 Substituenten, die unabhängig aus einer Hydroxygruppe, einer -(C₁-C₂)-Alkylgruppe, einem Fluoratom, einer - (C₁-C₂) -Alkoxygruppe, einer Nitrilgruppe, einer -C(O)O(C₁-C₃)-Gruppe oder einer -(C₃-C₅)-Cycloalkylgruppe, die unsubstituiert oder durch ein oder mehrere Fluoratome substituiert ist, ausgewählt sind, substituiert ist,
• einer -(C₃-C₆)-Cycloalkylgruppe, die unsubstituiert oder durch eine oder mehrere (C₁-C₂) -Alkylgruppen substituiert ist, oder
• einer Heterocyclopropylgruppe,
oder ein pharmazeutisch akzeptables Salz davon.

10. Verbindungen der Formel (I) nach Anspruch 1, die aus der folgenden Liste ausgewählt sind:
1 (R)-2-(8-Isopropyl-5-oxothieno[3',2':4,5]pyrrolo[1,2-d][1,2,4]triazin-6(5H)-yl)-N-(1-methylpiperidin-3-yl)acetamid
2 2-(8-Isopropyl-5-oxothieno[3',2':4,5]pyrrolo[1,2-d][1,2,4]triazin-6(5H)-yl)-N-(thiazol-5-yl)acetamid
3 N-((R)-1-((S)-2-Hydroxypropyl)piperidin-3-yl)-2-(8-isopropyl-5-oxothieno[3',2':4,5]pyrrolo[1,2-d][1,2,4]triazin-6(5H)-yl)acetamid
4 (R)-N-(1-Cyclopropylpiperidin-3-yl)-2-(8-isopropyl-5-oxothieno[3',2':4,5]pyrrolo[1,2-d][1,2,4]triazin-6(5H)-yl)acetamid
5 (R)-2-(2-Chlor-8-isopropyl-5-oxothieno[3',2':4,5]pyrrolo[1,2-d][1,2,4]triazin-6(5H)-yl)-N-(1-methylpiperidin-3-yl)acetamid-2,2,2-trifluoracetat
6 (R)-2-(2-Chlor-8-isopropyl-5-oxothieno[3',2':4,5]pyrrolo[1,2-d][1,2,4]triazin-6(5H)-yl)-N-(1-cyclopropylpiperidin-3-yl)acetamid-2,2,2-trifluoracetat
7 2-(2-Chlor-8-isopropyl-5-oxothieno[3',2':4,5]pyrrolo[1,2-d][1,2,4]triazin-6(5H)-yl)-N-(thiazol-5-yl)acetamid
8 (R)-2-(2-Brom-8-isopropyl-5-oxothieno[3',2':4,5]pyrrolo[1,2-d][1,2,4]triazin-6(5H)-yl)-N-(1-methylpiperidin-3-yl)acetamid
9 (R)-2-(8-Isopropyl-2-methyl-5-oxothieno[3',2':4,5]pyrrolo[1,2-d][1,2,4]triazin-6(5H)-yl)-N-(1-methylpiperidin-3-yl)acetamid-2,2,2-trifluoracetat
10 (R)-2-(2-Chlor-8-isopropyl-5-oxothieno[3',2':4,5]pyrrolo[1,2-d][1,2,4]triazin-6(5H)-yl)-N-(1-cyclobutylpiperidin-3-yl)acetamid-formiat
11 (R)-N-(1-Cyclobutylpiperidin-3-yl)-2-(8-isopropyl-5-oxothieno[3',2':4,5]pyrrolo[1,2-d][1,2,4]triazin-6(5H)-yl)acetamid-formiat
12 N-(Benzo[d]thiazol-6-yl)-2-(8-isopropyl-5-oxothieno[3',2':4,5]pyrrolo[1,2-d][1,2,4]triazin-6(5H)-yl)acetamid
13 (R)-2-(2-Chlor-8-cyclopropyl-5-oxothieno[3',2':4,5]pyrrolo[1,2-d][1,2,4]triazin-6(5H)-yl)-N-(1-cyclopropylpiperidin-3-yl)acetamid
14 (R)-N-(1-Isobutylpiperidin-3-yl)-2-(8-isopropyl-5-oxothieno[3',2':4,5]pyrrolo[1,2-d][1,2,4]triazin-6(5H)-yl)acetamid
15 N-(Benzo[d]thiazol-5-yl)-2-(8-isopropyl-5-oxothieno[3',2':4,5]pyrrolo[1,2-d][1,2,4]triazin-6(5H)-yl)acetamid
16 2-(8-Isopropyl-5-oxothieno[3',2':4,5]pyrrolo[1,2-d][1,2,4]triazin-6(5H)-yl)-N-(6-methoxypyridin-3-yl)acetamid
17 (R)-N-(1-(Cyclopentylmethyl)piperidin-3-yl)-2-(8-isopropyl-5-oxothieno[3',2':4,5]pyrrolo[1,2-d][1,2,4]triazin-6(5H)-yl)acetamid
18 N-((3R)-1-((2,2-Difluorcyclopropyl)methyl)piperidin-3-yl)-2-(8-isopropyl-5-oxothieno[3',2':4,5]pyrrolo[1,2-d][1,2,4]triazin-6(5H)-yl)acetamid Verbindung mit Formaldehyd
19 (R)-N-(1-(2-Hydroxyethyl)piperidin-3-yl)-2-(8-isopropyl-5-oxothieno[3',2':4,5]pyrrolo[1,2-d][1,2,4]triazin-6(5H)-yl)acetamid Verbindung mit Formaldehyd
20 (R)-N-(1-Cyclobutylpiperidin-3-yl)-2-(8-cyclopropyl-5-oxothieno[3',2':4,5]pyrrolo[1,2-d][1,2,4]triazin-6(5H)-yl)acetamid
21 (R)-2-(8-Cyclopropyl-5-oxothieno[3',2':4,5]pyrrolo[1,2-d][1,2,4]triazin-6(5H)-yl)-N-(1-cyclopropylpiperidin-3-yl)acetamid
22 2-(8-Cyclopropyl-5-oxothieno[3',2':4,5]pyrrolo[1,2-d][1,2,4]triazin-6(5H)-yl)-N-((R)-1-((S)-2-hydroxypropyl)piperidin-3-yl)acetamid
23 (R)-N-(1-(2-Hydroxy-2-methylpropyl)piperidin-3-yl)-2-(8-isopropyl-5-oxothieno[3',2':4,5]pyrrolo[1,2-d][1,2,4]triazin-6(5H)-yl)acetamid-formiat
24 (R)-2-(8-Cyclopropyl-5-oxothieno[3',2':4,5]pyrrolo[1,2-d][1,2,4]triazin-6(5H)-yl)-N-(1-methylpiperidin-3-yl)acetamid-formiat
25 N-((R)-1-Cyclobutylpiperidin-3-yl)-2-(8-isopropyl-5-oxothieno[3',2':4,5]pyrrolo[1,2-d][1,2,4]triazin-6(5H)-yl)propanamid-formiat
26 N-((R)-1-Cyclopropylpiperidin-3-yl)-2-(8-isopropyl-5-oxothieno[3',2':4,5]pyrrolo[1,2-d][1,2,4]triazin-6(5H)-yl)propanamid-formiat
27 2-(8-Isopropyl-5-oxothieno[3',2':4,5]pyrrolo[1,2-d][1,2,4]triazin-6(5H)-yl)-N-(1-methyl-1H-indazol-6-yl)acetamid-formiat
28 (R)-N-(1-(Cyclopropylmethyl)piperidin-3-yl)-2-(8-isopropyl-5-oxothieno[3',2':4,5]pyrrolo[1,2-d][1,2,4]triazin-6(5H)-yl)acetamid-formiat
29 (R)-2-(2-Chlor-8-(methoxymethyl)-5-oxothieno[3',2':4,5]pyrrolo[1,2-d][1,2,4]triazin-6(5H)-yl)-N-(1-cyclobutylpiperidin-3-yl)acetamid-formiat
30 (R)-2-(2-Chlor-8-(methoxymethyl)-5-oxothieno[3',2':4,5]pyrrolo[1,2-d][1,2,4]triazin-6(5H)-yl)-N-(1-cyclopropylpiperidin-3-yl)acetamid-formiat
31 (R)-2-(2-Brom-8-isopropyl-5-oxothieno[3',2':4,5]pyrrolo[1,2-d][1,2,4]triazin-6(5H)-yl)-N-(1-cyclobutylpiperidin-3-yl)acetamid-2,2,2-trifluoracetat
32 (R)-N-(1-Cyclobutylpiperidin-3-yl)-2-(8-(methoxymethyl)-5-oxothieno[3',2':4,5]pyrrolo[1,2-d][1,2,4]triazin-6(5H)-yl)acetamid
33 (R)-N-(1-Cyclopropylpiperidin-3-yl)-2-(8-(methoxymethyl)-5-oxothieno[3',2':4,5]pyrrolo[1,2-d][1,2,4]triazin-6(5H)-yl)acetamid-2,2,2-trifluoracetat
34 2-(2-Chlor-8-isopropyl-5-oxothieno[3',2':4,5]pyrrolo[1,2-d][1,2,4]triazin-6(5H)-yl)-N-(5,5-difluor-1-methylpiperidin-3-yl)acetamid-2,2,2-trifluoracetat
35 N-(5,5-Difluor-1-methylpiperidin-3-yl)-2-(8-isopropyl-5-oxothieno[3',2':4,5]pyrrolo[1,2-d][1,2,4]triazin-6(5H)-yl)acetamid
36 (R)-N-(1-(2-Fluorethyl)piperidin-3-yl)-2-(8-isopropyl-5-oxothieno[3',2':4,5]pyrrolo[1,2-d][1,2,4]triazin-6(5H)-yl)acetamid-formiat
37 N-(1-(tert-Butyl)piperidin-3-yl)-2-(8-isopropyl-5-oxothieno[3',2':4,5]pyrrolo[1,2-d][1,2,4]triazin-6(5H)-yl)acetamid-formiat
38 2-(8-Isopropyl-5-oxothieno[3',2':4,5]pyrrolo[1,2-d][1,2,4]triazin-6(5H)-yl)-N-(1-(2,2,2-trifluorethyl)piperidin-3-yl)acetamid-2,2,2-trifluoracetat
39 2-(8-Isopropyl-5-oxothieno[3',2':4,5]pyrrolo[1,2-d][1,2,4]triazin-6(5H)-yl)-N-(chinuclidin-3-yl)acetamid
40 (R)-2-(8-Isopropyl-5-oxothieno[3',2':4,5]pyrrolo[1,2-d][1,2,4]triazin-6(5H)-yl)-N-(1-(oxetan-3-yl)piperidin-3-yl)acetamid
41 (R)-N-(1-(3-Fluorpropyl)piperidin-3-yl)-2-(8-isopropyl-5-oxothieno[3',2':4,5]pyrrolo[1,2-d][1,2,4]triazin-6(5H)-yl)acetamid-formiat
42 (R)-N-(1-Acetylpiperidin-3-yl)-2-(8-isopropyl-5-oxothieno[3',2':4,5]pyrrolo[1,2-d][1,2,4]triazin-6(5H)-yl)acetamid
43 (R)-N-(1-((3,3-Difluorcyclobutyl)methyl)piperidin-3-yl)-2-(8-isopropyl-5-oxothieno[3',2':4,5]pyrrolo[1,2-d][1,2,4]triazin-6(5H)-yl)acetamid-formiat
44 N-((R)-1-Cyclopropylpiperidin-3-yl)-2-(8-(1-hydroxyethyl)-5-oxothieno[3',2' :4,5Jpyrrolo[1,2-d][1,2,4]triazin-6(5H)-yl)acetamid
45 (R)-2-(8-Isopropyl-5-oxothieno[3',2':4,5]pyrrolo[1,2-d][1,2,4]triazin-6(5H)-yl)-N-(1-(1-methyl-1H-imidazol-2-yl)piperidin-3-yl)acetamid
46 2-(8-Isopropyl-5-oxothieno[3',2':4,5]pyrrolo[1,2-d][1,2,4]triazin-6(5H)-yl)-N-(1-methyl-2-oxopiperidin-4-yl)acetamid
47 2-(8-Isopropyl-5-oxothieno[3',2':4,5]pyrrolo[1,2-d][1,2,4]triazin-6(5H)-yl)-N-(1-methyl-6-oxopiperidin-3-yl)acetamid
48 2-(12-Isopropyl-9-oxo-3-thia-1,10,11-triazatricyclo[6.4.0.02,6]dodeca-2(6),4,7,11-tetraen-10-yl)-N-[(3R)-1-isopropyl-3-piperidyl]acetamid
49 N-[(3R)-1-Cyclopentyl-3-piperidyl]-2-(12-isopropyl-9-oxo-3-thia-1,10,11-triazatricyclo[6.4.0.02,6]dodeca-2(6),4,7,11-tetraen-10-yl)acetamid
50 N-((R)-1-Cyclobutylpiperidin-3-yl)-2-(8-(1-hydroxyethyl)-5-oxothieno[3',2':4,5]pyrrolo[1,2-d][1,2,4]triazin-6(5H)-yl)acetamid
51 N-((R)-1-Cyclopropylpiperidin-3-yl)-2-(8-(1-hydroxyethyl)-5-oxothieno[3',2':4,5]pyrrolo[1,2d][1,2,4]triazin-6(5H)-yl)acetamid
52 (R)-N-(1-Cyclopropylpiperidin-3-yl)-2-(8-(2-hydroxypropan-2-yl)-5-oxothieno[3',2':4,5]pyrrolo[1,2-d][1,2,4]triazin-6(5H)-yl)acetamid
53 2-(12-Isopropyl-9-oxo-3-thia-1,10,11-triazatricyclo[6.4.0.02,6]dodeca-2(6),4,7,11-tetraen-10-yl)-N-(2-methylpyrazol-3-yl)acetamid
54 2-(12-Isopropyl-9-oxo-3-thia-1,10,11-triazatricyclo[6.4.0.02,6]dodeca-2(6),4,7,11-tetraen-10-yl)-N-(1-methylpyrazol-3-yl)acetamid
55 N-[(1-tert-Butyl-5-oxopyrrolidin-3-yl)methyl]-2-(12-isopropyl-9-oxo-3-thia-1,10,11-triazatricyclo[6.4.0.02,6]dodeca-2(6),4,7,11-tetraen-10-yl)acetamid
56 2-(12-Isopropyl-9-oxo-3-thia-1,10,11-triazatricyclo[6.4.0.02,6]dodeca-2(6),4,7,11-tetraen-10-yl)-N-(3-pyridyl)acetamid
57 2-(12-Isopropyl-9-oxo-3-thia-1,10,11-triazatricyclo[6.4.0.02,6]dodeca-2(6),4,7,11-tetraen-10-yl)-N-[rac-(3R)-1-(2,2-dimethylcyclobutyl)-3-piperidyl]acetamid
58 2-(12-Isopropyl-9-oxo-3-thia-1,10,11-triazatricyclo[6.4.0.02,6]dodeca-2(6),4,7,11-tetraen-10-yl)-N-(2-pyridyl)acetamid
59 2-(4-Brom-12-isopropyl-9-oxo-3-thia-1,10,11-triazatricyclo[6.4.0.02,6]dodeca-2(6),4,7,11-tetraen-10-yl)-N-(1-tert-butyl-3-piperidyl)acetamid
60 N-[(3R)-1-Cyclopropyl-3-piperidyl]-2-(12-ethyl-9-oxo-3-thia-1,10,11-triazatricyclo[6.4.0.02,6]dodeca-2(6),4,7,11-tetraen-10-yl)acetamid
61 N-[(3R)-1-Cyclopropylazepan-3-yl]-2-(12-isopropyl-9-oxo-3-thia-1,10,11-triazatricyclo[6.4.0.02,6]dodeca-2(6),4,7,11-tetraen-10-yl)acetamid
62 2-(12-Isopropyl-9-oxo-3-thia-1,10,11-triazatricyclo[6.4.0.02,6]dodeca-2(6),4,7,11-tetraen-10-yl)-N-[(3R)-1-methylazepan-3-yl]acetamid
63 N-(1-Cyclopropyl-5,5-difluorpiperidin-3-yl)-2-(8-isopropyl-5-oxothieno[3',2':4,5]pyrrolo[1,2-d][1,2,4]triazin-6(5H)-yl)acetamid-2,2,2-trifluoracetat
64 2-(8-Isopropyl-5-oxothieno[3',2':4,5]pyrrolo[1,2-d][1,2,4]triazin-6(5H)-yl)-N-((3R)-1-(1-methoxypropan-2-yl)piperidin-3-yl)acetamid
65 2-(8-Isopropyl-5-oxothieno[3',2':4,5]pyrrolo[1,2-d][1,2,4]triazin-6(5H)-yl)-N-(2-methoxypyridin-4-yl)acetamid
66 2-(12-Isopropyl-9-oxo-3-thia-1,10,11-triazatricyclo[6.4.0.02,6]dodeca-2(6),4,7,11-tetraen-10-yl)-N-pyrimidin-4-yl-acetamid
67 2-(12-Isopropyl-9-oxo-3-thia-1,10,11-triazatricyclo[6.4.0.02,6]dodeca-2(6),4,7,11-tetraen-10-yl)-N-(4-pyridyl)acetamid
68 2-(12-Isopropyl-9-oxo-3-thia-1,10,11-triazatricyclo[6.4.0.02,6]dodeca-2(6),4,7,11-tetraen-10-yl)-N-(6-methyl-3-pyridyl)acetamid
69 2-(12-Ethyl-9-oxo-3-thia-1,10,11-triazatricyclo[6.4.0.02,6]dodeca-2(6),4,7,11-tetraen-10-yl)-N-[(3R)-1-[(2S)-2-hydroxypropyl]-3-piperidyl]acetamid
70 N-(5-Fluorpyrimidin-4-yl)-2-(12-isopropyl-9-oxo-3-thia-1,10,11-triazatricyclo[6.4.0.02,6]dodeca-2(6),4,7,11-tetraen-10-yl)acetamid
71 2-(12-Isopropyl-9-oxo-3-thia-1,10,11-triazatricyclo[6.4.0.02,6]dodeca-2(6),4,7,11-tetraen-10-yl)-N-[(3R)-1-(1-methylcyclopentyl)-3-piperidyl]acetamid
72 N-[(3R)-1-Cyclopropylpiperidin-3-yl]-2-(9-oxo-12-propyl-3-thia-1,10,11-triazatricyclo[6.4.0.02,6]dodeca-2(6),4,7,11-tetraen-10-yl)acetamid
73 2-(12-Ethyl-9-oxo-3-thia-1,10,11-triazatricyclo[6.4.0.02,6]dodeca-2(6),4,7,11-tetraen-10-yl)-N-[(3R)-1-methyl-3-piperidyl]acetamid
74 N-[3-(Acetamidomethyl)bicyclo[1.1.1]pentan-1-yl]-2-(8-isopropyl-5-oxothieno[3',2':4,5]pyrrolo[1,2-d][1,2,4]triazin-6(5H)-yl)acetamid
75 N-[(3R)-1-(Cyanomethyl)piperidin-3-yl]-2-(8-isopropyl-5-oxothieno[3',2':4,5]pyrrolo[1,2-d][1,2,4]triazin-6(5H)-yl)acetamid
76 N-[(3R)-1-Methylpyrrolidin-3-yl]-2-(8-isopropyl-5-oxothieno[3',2':4,5]pyrrolo[1,2-d][1,2,4]triazin-6(5H)-yl)acetamid
77 N-[(3R)-1-(2-cyanoethyl)piperidin-3-yl]-2-(8-isopropyl-5-oxothieno[3',2':4,5]pyrrolo[1,2-d][1,2,4]triazin-6(5H)-yl)acetamid
78 N-[(3R)-1-[(2R)-2-Hydroxypropyl]piperidin-3-yl]-2-(8-isopropyl-5-oxothieno[3',2':4,5]pyrrolo[1,2-d][1,2,4]triazin-6(5H)-yl)acetamid
79 N-[(3R)-1-(1-Hydroxy-2-methylpropan-2-yl)piperidin-3-yl]-2-(8-isopropyl-5-oxothieno[3',2':4,5]pyrrolo[1,2-d][1,2,4]triazin-6(5H)-yl)acetamid
80 2-Methyl-2-[3-[[2-(8-isopropyl-5-oxothieno[3',2':4,5]pyrrolo[1,2-d][1,2,4]triazin-6(5H)-yl)acetyl]amino]piperidin-1-yl]propansäureethylester
81 N-((R)-1-Cyclobutylpiperidin-3-yl)-2-(8-(1-hydroxyethyl)-5-oxothieno[3',2':4,5]pyrrolo[1,2-d][1,2,4]triazin-6(5H)-yl)acetamid
82 N-[(1R,4R)-2-methyl-2-azabicyclo[2.2.1]heptan-6-yl]-2-(8-isopropyl-5-oxothieno[3',2':4,5]pyrrolo[1,2-d][1,2,4]triazin-6(5H)-yl)acetamid
83 N-(2-Methyl-2-azaspiro[3.3]heptan-6-yl)-2-(8-isopropyl-5-oxothieno[3',2':4,5]pyrrolo[1,2-d][1,2,4]triazin-6(5H)-yl)acetamid
84 2-(8-Isopropyl-5-oxothieno[3',2':4,5]pyrrolo[1,2-d][1,2,4]triazin-6(5H)-yl)-N-pyrimidin-5-ylacetamid
85 2-(12-Isopropyl-9-oxo-3-thia-1,10,11-triazatricyclo[6.4.0.02,6]dodeca-2(6),4,7,11-tetraen-10-yl)-N-(2-oxo-4-piperidyl)acetamid
86 N-(1-Cyclopropylpyrrolidin-3-yl)-2-(12-isopropyl-9-oxo-3-thia-1,10,11-triazatricyclo[6.4.0.02,6]dodeca-2(6),4,7,11-tetraen-10-yl)acetamid
87 2-(12-Isopropyl-9-oxo-3-thia-1,10,11-triazatricyclo[6.4.0.02,6]dodeca-2(6),4,7,11-tetraen-10-yl)-N-[rac-(3R)-1-tert-butylpyrrolidin-3-yl]acetamid
88 N-[(1S,4R)-2-Azabicyclo[2.2.1]heptan-6-yl]-2-(12-isopropyl-9-oxo-3-thia-1,10,11-triazatricyclo[6.4.0.02,6]dodeca-2(6),4,7,11-tetraen-10-yl)acetamid-hydrochlorid.

11. Medikament, **dadurch gekennzeichnet, dass** es eine Verbindung der Formel (I) nach einem der Ansprüche 1 bis 10 oder ein pharmazeutisch akzeptables Salz davon umfasst.

12. Pharmazeutische Zusammensetzung, **dadurch gekennzeichnet, dass** sie eine Verbindung der Formel (I) nach einem der Ansprüche 1 bis 10 umfasst.

13. Verbindung der Formel (I) nach einem der Ansprüche 1 bis 10 zur Verwendung bei der Behandlung von Parkinson-Krankheit, Multisystematrophie, Alzheimer-Krankheit, frontotemporaler Demenz, multipler Sklerose, amyotropher Lateralsklerose oder Hirnverletzung.

## Revendications

1. Composé de formule (I) 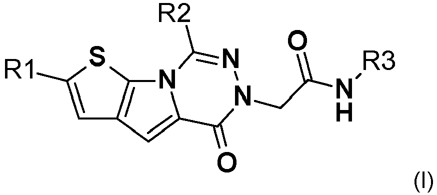 dans laquelle :
R1 représente un atome d'hydrogène, un atome d'halogène, ou un groupe - (C₁-C₂) -alkyle,
R2 représente un groupe -(C₁-C₃)alkyle qui est non substitué ou substitué par 1 à 3 substituants indépendamment choisis parmi un groupe hydroxy, ou un groupe -(C₁-C₃)-alcoxy, ou représente un groupe -(C₃-C₄) cycloalkyle,
R3 est choisi parmi
➢ un groupe - (C₅-C₈) bicycloalkyle qui est non substitué ou substitué par un groupe - (C₁-C₃) alkyle qui est non substitué ou substitué par un ou deux groupes - NH (CO) Me,
➢ un groupe -(C₄-C₇)hétérocycloalkyle comportant un azote en tant qu'hétéroatome, qui est non substitué ou substitué par un ou plusieurs substituants indépendamment choisis parmi
• un atome d'halogène,
• un groupe oxo,
• un groupe -(C₁-C₄)-alkyle, qui est non substitué ou substitué par 1 à 3 substituants indépendamment choisis parmi un groupe hydroxy, un groupe - (C₁-C₂) -alkyle, un atome d'halogène, un groupe - (C₁-C₂) -alcoxy, un groupe nitrile, un groupe -C(O)O(C₁-C₃) , ou un groupe -(C₃-C₈)-cycloalkyle qui est non substitué ou substitué par un ou plusieurs groupes halogène,
• un groupe - (C₃-C₆)-cycloalkyle, qui est non substitué ou substitué par un ou plusieurs groupes (C₁-C₂) alkyle,
• un groupe - (CO) -(C₁-C₂)-alkyle,
• un groupe hétérocycloalkyle, ou
• un groupe hétéroaryle qui est non substitué ou substitué par un ou plusieurs groupes -(C₁-C₄)alkyle,
➢ un groupe -hétéro(C₆-C₉)bicycloalkyle comportant un azote en tant qu'hétéroatome, éventuellement substitué par un ou plusieurs substituants indépendamment choisis parmi
• un groupe -(C₃-C₄)cycloalkyle, ou
• un groupe -(C₁-C₃)alkyle,
ou
➢ un groupe mono, ou bicyclohétéroaryle, qui est non substitué ou substitué par un ou plusieurs substituants indépendamment choisis parmi
➢ un groupe - (C₁-C₄) -alkyle,
➢ un groupe - (C₁-C₄) -alcoxy, ou
➢ un atome d'halogène,
ou sel pharmaceutiquement acceptable correspondant.

2. Composé selon la revendication 1,
R1 représente un atome d'hydrogène, un atome d'halogène choisi parmi un atome de brome et de chlore, ou un groupe méthyle,
R2 représente un groupe - (C₁-C₃) alkyle choisi parmi un groupe méthyle, éthyle, propyle et isopropyle qui est non substitué ou substitué par 1 substituant indépendamment choisi parmi un groupe hydroxy, ou un groupe méthoxy, ou représente un groupe cyclopropyle,
R3 est choisi parmi
➢ un groupe bicyclopentyle qui est substitué par un groupe -(C₁-C₃)alkyle qui est non substitué ou substitué par un groupe -NH(CO)Me,
➢ un groupe -(C₄-C₆) hétérocycloalkyle comportant un azote en tant qu'hétéroatome, qui est non substitué ou substitué par un ou plusieurs substituants indépendamment choisis parmi
• un atome de fluor,
• un groupe oxo,
• un groupe -(C₁-C₄)-alkyle, qui est non substitué ou substitué par 1 à 3 substituants indépendamment choisis parmi un groupe hydroxy, un groupe méthyle, un atome de fluor, un groupe méthoxy, un groupe nitrile, un groupe - C(O)O(C₁-C₂), ou un groupe - (C₃-C₆)-cycloalkyle qui est non substitué ou substitué par un atome de fluor,
• un groupe -(C₃-C₅)-cycloalkyle, qui est non substitué ou substitué par un ou plusieurs groupes méthyle,
• un groupe -(CO)-méthyle,
• un groupe hétérocyclopropyle, ou
• un imidazole, qui est non substitué ou substitué par un groupe méthyle,
➢ un groupe -hétéro(C₆-C₉)bicycloalkyle comportant un azote en tant qu'hétéroatome, éventuellement substitué par un ou plusieurs substituants indépendamment choisis parmi
• un groupe -(C₃-C₄) cycloalkyle, ou
• un groupe méthyle,
ou
➢ un groupe mono, ou bicyclohétéroaryle, qui est non substitué ou substitué par un ou deux substituants indépendamment choisis parmi
➢ un groupe méthyle,
➢ un groupe méthoxy, ou
➢ un atome de fluor,
ou sel pharmaceutiquement acceptable correspondant.

3. Composé selon l'une quelconque des revendications 1 ou 2, dans lequel
R1 représente un atome d'hydrogène, ou un atome d'halogène choisi parmi un atome de brome et un atome de chlore,
R2 représente un groupe -(C₁-C₃) alkyle qui est non substitué ou substitué par 1 à 3 substituants indépendamment choisis parmi un groupe hydroxy, ou représente un groupe -(C₃-C₄)cycloalkyle,
R3 est choisi parmi
➢ un groupe - (C₅-C₈) bicycloalkyle qui est non substitué ou substitué par un groupe - (C₁-C₃) alkyle qui est non substitué ou substitué par un ou deux groupes - NH(CO)Me,
➢ un groupe -(C₄-C₇)hétérocycloalkyle comportant un azote en tant qu'hétéroatome, qui est non substitué ou substitué par un ou plusieurs substituants indépendamment choisis parmi
• un groupe - (C₁-C₄) -alkyle qui est non substitué ou substitué par 1 à 3 substituants indépendamment choisis parmi un groupe hydroxy, un groupe méthyle, un atome de fluor, un groupe méthoxy, un groupe nitrile, un groupe - C(O)O(C₁-C₃), ou un groupe - (C₃-C₅)-cycloalkyle qui est non substitué ou substitué par un ou plusieurs atomes de fluor,
• un groupe -(C₃-C₆)-cycloalkyle, qui est non substitué ou substitué par un ou plusieurs groupes -(C₁-C₂) -alkyle, ou
• un groupe hétérocycloalkyle,
➢ un groupe -hétéro(C₆-C₈) bicycloalkyle comportant un azote en tant qu'hétéroatome, éventuellement substitué par un ou plusieurs groupes -(C₃-C₄)cycloalkyle,
ou
➢ un groupe mono, ou bicyclohétéroaryle, qui est non substitué ou substitué par un atome de fluor,
ou sel pharmaceutiquement acceptable correspondant.

4. Composé selon l'une quelconque des revendications 1 à 3, dans lequel R1 représente un atome d'hydrogène ou sel pharmaceutiquement acceptable correspondant.

5. Composé selon l'une quelconque des revendications 1 à 3, dans lequel R1 représente un atome de brome ou sel pharmaceutiquement acceptable correspondant.

6. Composé selon l'une quelconque des revendications 1 à 3, dans lequel R1 représente un atome de chlore ou sel pharmaceutiquement acceptable correspondant.

7. Composé selon l'une quelconque des revendications 1 à 6, dans lequel R2 représente un groupe -iPr ou sel pharmaceutiquement acceptable correspondant.

8. Composé selon l'une quelconque des revendications 1 à 6, dans lequel R2 représente un groupe -cyclopropyle ou sel pharmaceutiquement acceptable correspondant.

9. Composé selon l'une quelconque des revendications 1 à 8, dans lequel R3 représente un groupe - (C₄-C₇)hétérocycloalkyle comportant un azote en tant qu'hétéroatome, qui est non substitué ou substitué par un ou plusieurs substituants indépendamment choisis parmi
• -(C₁-C₄)-alkyl group, being unsubstituted or substituted with 1 to 3 substituents independently selected from a hydroxy group, -(C₁-C₂)-alkyl group, a fluorine atom, -(C₁-C₂)-alkoxy group, a nitrile group, a -C(O)O(C₁-C₃) group, or a -(C₃-C₅)-cycloalkyl group being unsubstituted or substituted by one or more fluorine atom,
• un groupe -(C₃-C₆)-cycloalkyle group, qui est non substitué ou substitué par un ou plusieurs groupes (C₁-C₂) alkyle, ou
• un groupe hétérocyclopropyle.
ou sel pharmaceutiquement acceptable correspondant.

10. Composés de formule (I) selon la revendication 1 qui sont choisis parmi la liste suivante :
1 (R)-2-(8-isopropyl-5-oxothiéno[3',2':4,5]pyrrolo[1,2-d][1,2,4]triazin-6(5H)-yl)-N-(1-méthylpipéridin-3-yl)acétamide
2 2-(8-isopropyl-5-oxothiéno[3',2':4,5]pyrrolo[1,2-d][1,2,4]triazin-6(5H)-yl)-N-(thiazol-5-yl) acétamide
3 N-((R)-1-((S)-2-hydroxypropyl)pipéridin-3-yl)-2-(8-isopropyl-5-oxothiéno[3',2':4,5]pyrrolo[1,2-d] [1,2,4]triazin-6(5H)-yl)acétamide
4 (R)-N-(1-cyclopropylpipéridin-3-yl)-2-(8-isopropyl-5-oxothiéno[3',2':4,5]pyrrolo[1,2-d] [1,2,4]triazin-6(5H)-yl)acétamide
5 2,2,2-trifluoroacétate de (R)-2-(2-chloro-8-isopropyl-5-oxothiéno[3',2':4,5]pyrrolo[1,2-d] [1,2,4]triazin-6(5H)-yl)-N-(1-méthylpipéridin-3-yl)acétamide
6 2,2,2-trifluoroacétate de (R)-2-(2-chloro-8-isopropyl-5-oxothiéno[3',2':4,5]pyrrolo[1,2-d] [1,2,4]triazin-6(5H)-yl)-N-(1-cyclopropylpipéridin-3-yl)acétamide
7 2-(2-chloro-8-isopropyl-5-oxothiéno[3',2':4,5]pyrrolo[1,2-d][1,2,4]triazin-6(5H)-yl)-N-(thiazol-5-yl)acétamide
8 (R)-2-(2-bromo-8-isopropyl-5-oxothiéno[3',2':4,5]pyrrolo[1,2-d][1,2,4]triazin-6(5H)-yl)-N-(1-méthylpipéridin-3-yl)acétamide
9 2,2,2-trifluoroacétate de (R)-2-(8-isopropyl-2-méthyl-5-oxothiéno[3',2':4,5]pyrrolo[1,2-d] [1,2,4]triazin-6(5H)-yl)-N-(1-méthylpipéridin-3-yl)acétamide
10 formiate de (R)-2-(2-chloro-8-isopropyl-5-oxothiéno[3',2':4,5]pyrrolo[1,2-d][1,2,4]triazin-6(5H)-yl)-N-(1-cyclobutylpipéridin-3-yl)acétamide
11 formiate de (R)-N-(1-cyclobutylpipéridin-3-yl)-2-(8-isopropyl-5-oxothiéno[3',2':4,5]pyrrolo[1,2-d] [1,2,4]triazin-6(5H)-yl)acétamide
12 N-(benzo[d]thiazol-6-yl)-2-(8-isopropyl-5-oxothiéno[3',2':4,5]pyrrolo[1,2-d][1,2,4]triazin-6(5H)-yl)acétamide
13 (R)-2-(2-chloro-8-cyclopropyl-5-oxothiéno[3',2':4,5]pyrrolo[1,2-d][1,2,4]triazin-6(5H)-yl)-N-(1-cyclopropylpipéridin-3-yl)acétamide
14 (R)-N-(1-isobutylpipéridin-3-yl)-2-(8-isopropyl-5-oxothiéno[3',2':4,5]pyrrolo[1,2-d][1,2,4]triazin-6(5H)-yl)acétamide
15 N-(benzo[d]thiazol-5-yl)-2-(8-isopropyl-5-oxothiéno[3',2':4,5]pyrrolo[1,2-d][1,2,4]triazin-6(5H)-yl)acétamide
16 2-(8-isopropyl-5-oxothiéno[3',2':4,5]pyrrolo[1,2-d] [1,2,4]triazin-6(5H)-yl)-N-(6-méthoxypyridin-3-yl)acétamide
17 (R)-N-(1-(cyclopentylméthyl)pipéridin-3-yl)-2-(8-isopropyl-5-oxothiéno[3',2':4,5]pyrrolo[1,2-d] [1,2,4]triazin-6(5H)-yl)acétamide
18 N-((3R)-1-((2,2-difluorocyclopropyl)méthyl)pipéridin-3-yl)-2-(8-isopropyl-5-oxothiéno[3',2':4,5]pyrrolo[1,2-d][1,2,4]triazin-6(5H)-yl)acétamide composé avec le formaldéhyde
19 (R)-N-(1-(2-hydroxyéthyl)pipéridin-3-yl)-2-(8-isopropyl-5-oxothiéno[3',2':4,5]pyrrolo[1,2-d][1,2,4]triazin-6(5H)-yl)acétamide composé avec le formaldéhyde
20 (R)-N-(1-cyclobutylpipéridin-3-yl)-2-(8-cyclopropyl-5-oxothiéno[3',2':4,5]pyrrolo[1,2-d] [1,2,4]triazin-6(5H)-yl)acétamide
21 (R)-2-(8-cyclopropyl-5-oxothiéno[3',2':4,5]pyrrolo[1,2-d][1,2,4]triazin-6(5H)-yl)-N-(1-cyclopropylpipéridin-3-yl)acétamide
22 2-(8-cyclopropyl-5-oxothiéno[3',2':4,5]pyrrolo[1,2-d][1,2,4]triazin-6(5H)-yl)-N-((R)-1- ((S)-2-hydroxypropyl)pipéridin-3-yl)acétamide
23 formiate de (R)-N-(1-(2-hydroxy-2-méthylpropyl)pipéridin-3-yl)-2-(8-isopropyl-5-oxothiéno[3',2':4,5]pyrrolo[1,2-d][1,2,4]triazin-6(5H)-yl)acétamide
24 formiate de (R)-2-(8-cyclopropyl-5-oxothiéno[3',2':4,5]pyrrolo[1,2-d][1,2,4]triazin-6(5H)-yl)-N-(1-méthylpipéridin-3-yl)acétamide
25 formiate de N-((R)-1-cyclobutylpipéridin-3-yl)-2-(8-isopropyl-5-oxothiéno[3',2':4,5]pyrrolo[1,2-d] [1,2,4]triazin-6(5H)-yl)propanamide
26 formiate de N-((R)-1-cyclopropylpipéridin-3-yl)-2-(8-isopropyl-5-oxothiéno[3',2':4,5]pyrrolo[1,2-d] [1,2,4]triazin-6(5H)-yl)propanamide
27 formiate de 2-(8-isopropyl-5-oxothiéno[3',2':4,5]pyrrolo[1,2-d][1,2,4]triazin-6(5H)-yl)-N-(1-méthyl-1H-indazol-6-yl)acétamide
28 formiate de (R)-N-(1-(cyclopropylméthyl)pipéridin-3-yl)-2-(8-isopropyl-5-oxothiéno[3',2' :4,5]pyrrolo[1,2-d] [1,2,4]triazin-6(5H)-yl)acétamide
29 formiate de (R)-2-(2-chloro-8-(méthoxyméthyl)-5-oxothiéno[3',2':4,5]pyrrolo[1,2-d][1,2,4]triazin-6(5H)-yl)-N-(1-cyclobutylpipéridin-3-yl)acétamide
30 formiate de (R)-2-(2-chloro-8-(méthoxyméthyl)-5-oxothiéno[3',2':4,5]pyrrolo[1,2-d][1,2,4]triazin-6(5H)-yl)-N-(1-cyclopropylpipéridin-3-yl)acétamide
31 2,2,2-trifluoroacétate de (R)-2-(2-bromo-8-isopropyl-5-oxothiéno[3',2':4,5]pyrrolo[1,2-d] [1,2,4]triazin-6(5H)-yl)-N-(1-cyclobutylpipéridin-3-yl)acétamide
32 (R)-N-(1-cyclobutylpipéridin-3-yl)-2-(8-(méthoxyméthyl)-5-oxothiéno[3',2':4,5]pyrrolo[1,2-d] [1,2,4]triazin-6(5H)-yl)acétamide
33 2,2,2-trifluoroacétate de (R)-N-(1-cyclopropylpipéridin-3-yl)-2-(8-(méthoxyméthyl)-5-oxothiéno[3',2':4,5]pyrrolo[1,2-d][1,2,4]triazin-6(5H)-yl)acétamide
34 2,2,2-trifluoroacétate de 2-(2-chloro-8-isopropyl-5-oxothiéno[3',2':4,5]pyrrolo[1,2-d][1,2,4]triazin-6 (5H)-yl)-N-(5,5-difluoro-1-méthylpipéridin-3-yl)acétamide
35 N-(5,5-difluoro-1-méthylpipéridin-3-yl)-2-(8-isopropyl-5-oxothiéno[3',2':4,5]pyrrolo[1,2-d] [1,2,4]triazin-6(5H)-yl)acétamide
36 formiate de (R)-N-(1-(2-fluoroéthyl)pipéridin-3-yl)-2-(8-isopropyl-5-oxothiéno[3',2':4,5]pyrrolo[1,2-d] [1,2,4]triazin-6(5H)-yl)acétamide
37 formiate de N-(1-(tert-butyl)pipéridin-3-yl)-2-(8-isopropyl-5-oxothiéno[3',2':4,5]pyrrolo[1,2-d] [1,2,4]triazin-6(5H)-yl)acétamide
38 2,2,2-trifluoroacétate de 2-(8-isopropyl-5-oxothiéno[3',2':4,5]pyrrolo[1,2-d][1,2,4]triazin-6(5H)-yl)-N-(1-(2,2,2-trifluoroéthyl)pipéridin-3-yl)acétamide
39 2-(8-isopropyl-5-oxothiéno[3',2':4,5]pyrrolo[1,2-d] [1,2,4]triazin-6(5H)-yl)-N-(quinuclidin-3-yl)acétamide
40 (R)-2-(8-isopropyl-5-oxothiéno[3',2':4,5]pyrrolo[1,2-d][1,2,4]triazin-6(5H)-yl)-N-(1-(oxétan-3-yl)pipéridin-3-yl)acétamide
41 formiate de (R)-N-(1-(3-fluoropropyl)pipéridin-3-yl)-2-(8-isopropyl-5-oxothiéno[3',2':4,5]pyrrolo[1,2-d] [1,2,4]triazin-6(5H)-yl)acétamide
42 (R)-N-(1-acétylpipéridin-3-yl)-2-(8-isopropyl-5-oxothiéno[3',2':4,5]pyrrolo[1,2-d][1,2,4]triazin-6(5H)-yl)acétamide
43 formiate de (R)-N-(1-((3,3-difluorocyclobutyl)méthyl)pipéridin-3-yl)-2-(8-isopropyl-5-oxothiéno[3',2':4,5]pyrrolo[1,2-d] [1,2,4]triazin-6(5H)-yl)acétamide
44 N-((R)-1-cyclopropylpipéridin-3-yl)-2-(8-(1-hydroxyéthyl)-5-oxothiéno[3',2':4,5]pyrrolo[1,2-d] [1,2,4]triazin-6(5H)-yl)acétamide
45 (R)-2-(8-isopropyl-5-oxothiéno[3',2':4,5]pyrrolo[1,2-d][1,2,4]triazin-6(5H)-yl)-N-(1-(1-méthyl-1H-imidazol-2-yl)pipéridin-3-yl)acétamide
46 2-(8-isopropyl-5-oxothiéno[3',2':4,5]pyrrolo[1,2-d] [1,2,4]triazin-6(5H)-yl)-N-(1-méthyl-2-oxopipéridin-4-yl)acétamide
47 2-(8-isopropyl-5-oxothiéno[3',2':4,5]pyrrolo[1,2-d] [1,2,4]triazin-6(5H)-yl)-N-(1-méthyl-6-oxopipéridin-3-yl)acétamide
48 2-(12-isopropyl-9-oxo-3-thia-1,10,11-triazatricyclo[6.4.0.02,6]dodéca-2(6),4,7,11-tétraén-10-yl)-N-[(3R)-1-isopropyl-3-pipéridyl]acétamide
49 N-[(3R)-1-cyclopentyl-3-pipéridyl]-2-(12-isopropyl-9-oxo-3-thia-1,10,11-triazatricyclo[6.4.0.02,6]dodéca-2(6),4,7,11-tétraén-10-yl)acétamide
50 N-((R)-1-cyclobutylpipéridin-3-yl)-2-(8-(1-hydroxyéthyl)-5-oxothiéno[3',2':4,5]pyrrolo[1,2-d][1,2,4]triazin-6(5H)-yl)acétamide
51 N-((R)-1-cyclopropylpipéridin-3-yl)-2-(8-(1-hydroxyéthyl)-5-oxothiéno[3',2':4,5]pyrrolo[1,2d][1,2,4]triazin-6(5H)-yl)acétamide
52 (R)-N-(1-cyclopropylpipéridin-3-yl)-2-(8-(2-hydroxypropan-2-yl)-5-oxothiéno[3',2':4,5]pyrrolo[1,2-d][1,2,4]triazin-6(5H)-yl)acétamide
53 2-(12-isopropyl-9-oxo-3-thia-1,10,11-triazatricyclo[6.4.0.02,6]dodéca-2(6),4,7,11-tétraén-10-yl)-N-(2-méthylpyrazol-3-yl)acétamide
54 2-(12-isopropyl-9-oxo-3-thia-1,10,11-triazatricyclo[6.4.0.02,6]dodéca-2(6),4,7,11-tétraén-10-yl)-N-(1-méthylpyrazol-3-yl)acétamide
55 N-[(1-tert-butyl-5-oxo-pyrrolidin-3-yl)méthyl]-2-(12-isopropyl-9-oxo-3-thia-1,10,11-triazatricyclo[6.4.0.02,6]dodéca-2(6),4,7,11-tétraén-10-yl)acétamide
56 2-(12-isopropyl-9-oxo-3-thia-1,10,11-triazatricyclo[6.4.0.02,6]dodéca-2(6),4,7,11-tétraén-10-yl)-N-(3-pyridyl)acétamide
57 2-(12-isopropyl-9-oxo-3-thia-1,10,11-triazatricyclo[6.4.0.02,6]dodéca-2(6),4,7,11-tétraén-10-yl)-N-[rac-(3R)-1-(2,2-diméthylcyclobutyl)-3-pipéridyl]acétamide
58 2-(12-isopropyl-9-oxo-3-thia-1,10,11-triazatricyclo[6.4.0.02,6]dodéca-2(6),4,7,11-tétraén-10-yl)-N-(2-pyridyl)acétamide
59 2-(4-bromo-12-isopropyl-9-oxo-3-thia-1,10,11-triazatricyclo[6.4.0.02,6]dodéca-2(6),4,7,11-tétraén-10-yl)-N-(1-tert-butyl-3-pipéridyl)acétamide
60 N-[(3R)-1-cyclopropyl-3-pipéridyl]-2-(12-éthyl-9-oxo-3-thia-1,10,11-triazatricyclo[6.4.0.02,6]dodéca-2(6),4,7,11-tétraén-10-yl)acétamide
61 N-[(3R)-1-cyclopropylazépan-3-yl]-2-(12-isopropyl-9-oxo-3-thia-1,10,11-triazatricyclo[6.4.0.02,6]dodéca-2(6),4,7,11-tétraén-10-yl)acétamide
62 2-(12-isopropyl-9-oxo-3-thia-1,10,11-triazatricyclo[6.4.0.02,6]dodéca-2(6),4,7,11-tétraén-10-yl)-N-[ (3R)-1-méthylazépan-3-yl]acétamide
63 2,2,2-trifluoroacétate de N-(1-cyclopropyl-5,5-difluoropipéridin-3-yl)-2-(8-isopropyl-5-oxothiéno[3',2':4,5]pyrrolo[1,2-d][1,2,4]triazin-6(5H)-yl)acétamide
64 2-(8-isopropyl-5-oxothiéno[3',2':4,5]pyrrolo[1,2-d][1,2,4]triazin-6(5H)-yl)-N-((3R)-1-(1-méthoxypropan-2-yl)pipéridin-3-yl)acétamide
65 2-(8-isopropyl-5-oxothiéno[3',2':4,5]pyrrolo[1,2-d][1,2,4]triazin-6(5H)-yl)-N-(2-méthoxypyridin-4-yl)acétamide
66 2-(12-isopropyl-9-oxo-3-thia-1,10,11-triazatricyclo[6.4.0.02,6]dodéca-2(6),4,7,11-tétraén-10-yl)-N-pyrimidin-4-yl-acétamide
67 2-(12-isopropyl-9-oxo-3-thia-1,10,11-triazatricyclo[6.4.0.02,6]dodéca-2(6),4,7,11-tétraén-10-yl)-N-(4-pyridyl)acétamide
68 2-(12-isopropyl-9-oxo-3-thia-1,10,11-triazatricyclo[6.4.0.02,6]dodéca-2(6),4,7,11-tétraén-10-yl)-N-(6-méthyl-3-pyridyl)acétamide
69 2-(12-éthyl-9-oxo-3-thia-1,10,11-triazatricyclo[6.4.0.02,6]dodéca-2(6),4,7,11-tétraén-10-yl)-N-[(3R)-1-[(2S)-2-hydroxypropyl]-3-pipéridyl]acétamide
70 N-(5-fluoropyrimidin-4-yl)-2-(12-isopropyl-9-oxo-3-thia-1,10,11-triazatricyclo[6.4.0.02,6]dodéca-2(6),4,7,11-tétraén-10-yl)acétamide
71 2-(12-isopropyl-9-oxo-3-thia-1,10,11-triazatricyclo[6.4.0.02,6]dodéca-2(6),4,7,11-tétraén-10-yl)-N-[(3R)-1-(1-méthylcyclopentyl)-3-pipéridyl]acétamide
72 N-[(3R)-1-cyclopropylpipéridin-3-yl]-2-(9-oxo-12-propyl-3-thia-1,10,11-triazatricyclo[6.4.0.02,6]dodéca-2(6),4,7,11-tétraén-10-yl)acétamide
73 2-(12-éthyl-9-oxo-3-thia-1,10,11-triazatricyclo[6.4.0.02,6]dodéca-2(6),4,7,11-tétraén-10-yl)-N-[(3R)-1-méthyl-3-pipéridyl]acétamide
74 N-[3-(acétamidométhyl)bicyclo[1.1.1]pentan-1-yl]-2-(8-isopropyl-5-oxothiéno[3',2':4,5]pyrrolo[1,2-d] [1,2,4]triazin-6(5H)-yl)acétamide
75 N-[(3R)-1-(cyanométhyl)pipéridin-3-yl]-2-(8-isopropyl-5-oxothiéno[3',2':4,5]pyrrolo[1,2-d] [1,2,4]triazin-6(5H)-yl)acétamide
76 N-[(3R)-1-méthylpyrrolidin-3-yl]-2-(8-isopropyl-5-oxothiéno[3',2':4,5]pyrrolo[1,2-d][1,2,4]triazin-6(5H)-yl)acétamide
77 N-[(3R)-1-(2-cyanoéthyl)pipéridin-3-yl]-2-(8-isopropyl-5-oxothiéno[3',2':4,5]pyrrolo[1,2-d] [1,2,4]triazin-6(5H)-yl)acétamide
78 N-[(3R)-1-[(2R)-2-hydroxypropyl]pipéridin-3-yl]-2-(8-isopropyl-5-oxothiéno[3',2':4,5]pyrrolo[1,2-d] [1,2,4]triazin-6(5H)-yl)acétamide
79 N-[(3R)-1-(1-hydroxy-2-méthylpropan-2-yl)pipéridin-3-yl]-2-(8-isopropyl-5-oxothiéno[3',2':4,5]pyrrolo[1,2-d][1,2,4]triazin-6(5H)-yl)acétamide
80 2-méthyl-2-[3-[[2-(8-isopropyl-5-oxothiéno[3',2':4,5]pyrrolo[1,2-d][1,2,4]triazin-6(5H)-yl)acétyl]amino]pipéridin-1-yl]propanoate d'éthyle
81 N-((R)-1-cyclobutylpipéridin-3-yl)-2-(8-(1-hydroxyéthyl)-5-oxothiéno[3',2':4,5]pyrrolo[1,2-d] [1,2,4]triazin-6(5H)-yl)acétamide
82 N-[(1R,4R)-2-méthyl-2-azabicyclo[2.2.1]heptan-6-yl]-2-(8-isopropyl-5-oxothiéno[3',2':4,5]pyrrolo[1,2-d] [1,2,4]triazin-6(5H)-yl)acétamide
83 N-(2-méthyl-2-azaspiro[3.3]heptan-6-yl)-2-(8-isopropyl-5-oxothiéno[3',2':4,5]pyrrolo[1,2-d] [1,2,4]triazin-6(5H)-yl)acétamide
84 2-(8-isopropyl-5-oxothiéno[3',2':4,5]pyrrolo[1,2-d] [1,2,4]triazin-6(5H)-yl)-N-pyrimidin-5-ylacétamide
85 2-(12-isopropyl-9-oxo-3-thia-1,10,11-triazatricyclo[6.4.0.02,6]dodéca-2(6),4,7,11-tétraén-10-yl)-N-(2-oxo-4-pipéridyl)acétamide
86 N-(1-cyclopropyIpyrrolidin-3-yl)-2-(12-isopropyl-9-oxo-3-thia-1,10,11-triazatricyclo[6.4.0.02,6]dodéca-2(6),4,7,11-tétraén-10-yl)acétamide
87 2-(12-isopropyl-9-oxo-3-thia-1,10,11-triazatricyclo[6.4.0.02,6]dodéca-2(6),4,7,11-tétraén-10-yl)-N-[rac-(3R)-1-tert-butylpyrrolidin-3-yl]acétamide
88 chlorhydrate de N-[(1S,4R)-2-azabicyclo[2.2.1]heptan-6-yl]-2-(12-isopropyl-9-oxo-3-thia-1,10,11-triazatricyclo[6.4.0.02,6]dodéca-2(6),4,7,11-tétraén-10-yl)acétamide.

11. Médicament, **caractérisé en ce qu'**il comprend un composé de formule (I) selon l'une quelconque des revendications 1 à 10 ou un sel pharmaceutiquement acceptable de celui-ci.

12. Composition pharmaceutique, **caractérisée en ce qu'**elle comprend un composé de formule (I) selon l'une quelconque des revendications 1 à 10.

13. Composé de formule (I) selon l'une quelconque des revendications 1 à 10, pour une utilisation dans le traitement de la maladie de Parkinson, l'atrophie multisystémique, la maladie d'Alzheimer, la démence frontotemporale, la sclérose en plaques, la sclérose latérale amyotrophique ou une lésion cérébrale.
